# EUROPEAN PATENT SPECIFICATION

(11) **EP 2 968 190 B1**
(45) Date of publication and mention of the grant of the patent: **17.10.2018**
(21) Application number: 14779109.9
(22) Date of filing: 13.03.2014
(51) Int. Cl.: A61K 9/50, A01N 43/04, A61K 8/73, A61Q 19/08, A61L 27/20, A61L 27/50, A61L 31/04, A61K 9/00, A61K 8/02, A61K 9/70, A61L 26/00, A61K 31/715, A61K 31/726, A01N 43/16

(54) **TREATMENT OF DISEASE WITH POLY-N-ACETYLGLUCOSAMINE NANOFIBERS**
KRANKHEITSBEHANDLUNG MIT POLY-N-ACETYL-GLUCOSAMIN-NANOFASERN
TRAITEMENT DE MALADIE PAR DES NANOFIBRES DE POLY-N-ACÉTYLGLUCOSAMINE

(30) Priority: 14.03.2013 US 201361784765 P
(43) Date of publication of application: 20.01.2016
(73) Proprietor: Marine Polymer Technologies, Inc., Burlington, MA 01803 (US)
(72) Inventor: FINKIELSZTEIN, Sergio, Newton, MA 02459 (US); VOURNAKIS, John, N., Charleston, SC 29403 (US)
(74) Representative: Jones Day
(86) International application number: PCT/US2014/025623
(87) International publication number: WO 2014/165302

(56) References cited:
- WO-A1-2011/130646
- WO-A1-2012/142581
- WO-A2-2008/103345
- US-A1- 2004 087 015
- SHIRLEY ET AL.: 'Ehlers-Danlos Syndrome in Orthopaedics' SPORTS HEALTH. vol. 4, no. 5, September 2012, pages 394 - 403, XP008181241
- STEEN ET AL.: 'Improvement in Skin Thickening in Systemic Sclerosis Associated With Improved Survival.' ARTHRITIS & RHEUMATISM. vol. 44, no. 12, 01 December 2001, pages 2828 - 2835, XP055285681 DOI: 10.1002/1529-0131(200112)44:12<2828::AID-AR T470>
- FINE ET AL.: 'Revised clinical and laboratory criteria for subtypes of inherited epidermolysis bullosa: A consensus report by the Subcommittee on Diagnosis and Classification of the National Epidermolysis Bullosa Registry.' JOURNAL OF THE AMERICAN ACADEMY OF DERMATOLOGY. vol. 24, no. 1, 01 January 1991, pages 119 - 135, XP023851691 DOI: 10.1016/0190-9622(91)70021-S

## Description

### 1. FIELD

Described herein are compositions comprising shortened fibers of poly-N-acetylglucosamine and/or a derivative thereof ("sNAG nanofibers") and the use of such compositions in the treatment of various conditions and diseases, in particular, those associated with decreased tensile strength of tissue, decreased elasticity of tissue, increased collagen content or abnormal collagen content in tissue, abnormal alignment of collagen in tissue, and/or increased myofibroblast content in tissue.

### 2. BACKGROUND

A number of conditions and diseases that are either incurable at this time or have suboptimal treatments available, due to only partial effectiveness of such treatments or side effects associated with such treatments. For example, there are a number of incurable or only partially curable conditions and diseases associated with decreased tensile strength of tissue, decreased elasticity of tissue, increased collagen content or abnormal collagen content in tissue, abnormal alignment of collagen in tissue, and/or increased myofibroblast content in tissue. Such conditions and diseases include, among others, Ehlers-Danlos Syndrome, Epidermolysis bullosa, scleroderma, osteoporosis, intervertebral disc disorder, degenerative disc disorder, osteoarthritis, fibrosis, wrinkling of the skin, and scarring associated with wounds. There remains a need for an effective treatment for these conditions and diseases that can be used alone, or in combination with a standard therapy, that is safe and effective.

### 3. SUMMARY

In one aspect, the invention provides a method for treating wrinkles or depressions in the skin's surface in a human subject, comprising administering a composition comprising shortened fibers of poly-N-acetylglucosamine ("sNAG nanofibers") to the human subject, wherein the composition is prepared for topical administration directly or in proximity to, or local injection directly to, the wrinkles or depressions, wherein more than 70% of the monosaccharides of the sNAG nanofibers are N-acetylglucosamine monosaccharides, and wherein the sNAG nanofibers are: (i) from about 1 µm to less than 10 µm in length, or (ii) less than 10 µm in average length, and have the microstructure of non-irradiated poly-N-acetylglucosamine fibers.

In another aspect, the invention provides a composition comprising shortened fibers of poly-N-acetylglucosamine ("sNAG nanofibers") for use in a method for treating wrinkles or depressions in the skin's surface in a human subject, comprising administering the composition to the human subject, wherein the composition is prepared for topical administration directly or in proximity to, or local injection directly to, the wrinkles or depressions, wherein more than 70% of the monosaccharides of the sNAG nanofibers are N-acetylglucosamine monosaccharides, and wherein the sNAG nanofibers are: (i) from about 1 µm to less than 10 µm in length, or (ii) less than 10 µm in average length, and have the microstructure of non-irradiated poly-N-acetylglucosamine fibers.

In one embodiment, more than 80%, more than 90%, more than 95%, or 100% of the monosaccharides of the sNAG nanofibers are N-acetylglucosamine monosaccharides.

In another embodiment, the subject has an increased total collagen content, increased expression of collagen type I, decreased expression of collagen type III, decreased expression of elastin, increased expression of smooth muscle actin, decreased tensile strength of the skin and/or decreased elasticity of the skin.

In another embodiment, the sNAG nanofibers are nonreactive when tested in an intramuscular implantation test; and/or wherein the sNAG nanofibers increase the metabolic rate of serum-starved human umbilical cord vein endothelial cells in a MTT assay and/or do not rescue apoptosis of serum-starved human umbilical cord endothelial cells in a trypan blue exclusion test.

In another embodiment, more than 50% of the sNAG nanofibers are between about 2 to 10 µm in length; wherein more than 50% of the sNAG nanofibers are between about 4 to 7 µm in length; or wherein the sNAG nanofibers have an average length of 4 to 7 µm.

In another embodiment, the sNAG nanofibers are produced by gamma irradiation of poly-N-acetylglucosamine and/or a derivative thereof, and wherein the poly-N-acetylglucosamine and/or a derivative thereof is irradiated in the form of dry fibers or a dry fiber membrane at 500-2,000 kgy, optionally 750-1,250 kgy; or the poly-N-acetylglucosamine and/or a derivative thereof was irradiated in the form of a suspension, a slurry or a wet cake at 100-500 kgy, optionally 150-250 kgy.

In another embodiment, the sNAG nanofibers are produced by irradiation of poly-N-acetylglucosamine or a derivative thereof with a dose of irradiation that reduces the average length of the sNAG nanofibers to less than 10 µm in length.

In another embodiment, the sNAG nanofibers are administered in an amount effective to achieve at least one or more of the following effects: (a) the increase in the tensile strength of a tissue in a subject, (b) the increase in elasticity in a tissue of a subject, (c) the increase in elastin content or production in a tissue of a subject, (d) the decrease in total collagen content in a tissue of a subject, (e) the decrease of collagen I expression or content in a tissue of a subject, (f) the increase in collagen III expression or content in a tissue of a subject, (g) the inducement of more organized collagen alignment in a tissue of a subject, (h) the reduction in smooth muscle actin content or expression in a tissue of a subject, and (i) the reduction in myofibroblast content in a tissue of a subject.

In another embodiment, the infrared spectrum of the sNAG nanofibers is equivalent or substantially equivalent to that of non-irradiated microalgal poly-β-1→4-N-acetylglucosamine fibers.

In another embodiment, the composition is formulated as a cream, a membrane, a film, a liquid solution, a suspension, a powder, a paste, an ointment, an aerosol, a gel, or a spray.

In another embodiment, the composition is formulated for administration to the subject once or twice a day for a duration equal to or greater than 1 week.

In another embodiment, the composition comprises (i) 0.2 to 20 mg/cm² of sNAG nanofibers per dose/application of the composition in a form suitable for topical delivery to the subject, or (ii) 5 to 50 mg/ml of sNAG nanofibers per dose/application of the composition in a form suitable for topical delivery to the subject.

In another embodiment, the composition is formulated for local injection and the wrinkles are from a few micrometers to several millimeters into the skin.

In another embodiment, the sNAG nanofibers are not produced from crustacean poly-N-acetylglucosamine; and/or the sNAG nanofibers are produced from a microalgal poly-N-acetylglucosamine.

In another embodiment, the composition is prepared for topical administration directly to the wrinkles or depressions in the skin's surface of the human subject, wherein greater than 50% of the sNAG nanofibers have an average length of 4 to 7 µm, and wherein the infrared spectrum of the sNAG nanofibers is equivalent or substantially equivalent to that of non-irradiated poly-β-1→4-N-acetylglucosamine fibers.

In the context of the invention, references to methods of treatment are to be understood as:
1) references to the compounds and compositions of the invention for use in said methods; or
2) or non-therapeutic or non-surgical methods.

Disclosed herein are methods of treating various diseases associated with decreased tensile strength of tissue, decreased elasticity of tissue, increased collagen content or abnormal collagen content in tissue, abnormal or disorganized alignment of collagen in tissue, and/or increased myofibroblast content in tissue. Further, disclosed herein are methods of treating various diseases associated with an increase in collagen type I content (e.g., expression) in tissue, a decrease of collagen type III content (e.g., expression) in tissue, a decrease in elastin content (e.g., expression) in tissue, or an increase in alpha smooth muscle actin content in tissue.

Disclosed herein is a method of treating a symptom of Ehler-Danols syndrome in a subject (e.g., a human subject), comprising administering a composition comprising sNAG nanofibers to the subject, wherein more than 50% of the sNAG nanofibers are between about 1 to 15 µm in length. In one instance, the symptom is a skin-related symptom. In a further instance, the skin-related symptom is soft skin, fragile skin, skin that bruises easily, excessive scarring of the skin, or blunted wound healing in the skin. In a specific case, the composition is administered topically to the subject. In yet another case, the composition is administered directly to the skin affected by the skin-related symptom.

Disclosed herein is a method of treating a symptom of scleroderma in a subject (e.g., a human subject), comprising administering a composition comprising sNAG nanofibers to the subject, wherein more that 50% of the sNAG nanofibers are between 1 to 15 µm in length. In one instance, the symptom is a skin-related symptom. In another instance, the skin-related symptom is swollen skin, thickened skin, shiny skin, discoloration of skin, or numbness of skin. In a specific case, the composition is administered topically to the subject. In another case, the composition is administered directly to the skin affected by the skin-related symptom.

Disclosed herein is a method for treating a symptom of Epidermolysis bullosa in a subject (e.g., a human subject), comprising administering a composition comprising sNAG nanofibers to a subject, wherein more than 50% of the sNAG nanofibers are between about 1 to 15 µm in length. In one instance the symptom is a skin-related symptom or a mucosal membrane-related symptom. In another instance, the skin-related symptom or the mucosal membrane-related symptom is a blister. In a specific case, the composition is administered topically to the subject. In yet another case, the composition is administered directly to the skin affected by the skin-related symptom or the mucosal membrane-related symptom.

Disclosed provided herein is a method for using a composition comprising sNAG nanofibers in regenerative medicine, wherein more than 50% of the sNAG nanofibers are between about 1 to 15 µm in length. In specific instances, disclosed herein is a method for regenerating a tissue in a subject (e.g., a human subject), comprising administering a composition comprising sNAG nanofibers to the tissue in the subject, wherein more than 50% of the sNAG nanofibers are between about 1 to 15 µm in length. The tissue can be a soft tissue (e.g., skin or muscle) or a hard tissue (e.g., bone). In some instances, the composition is administered topically to the tissue in need of regeneration. In other instances, the composition is administered to the tissue in need of regeneration by local injection into the tissue. In some instances, the composition is administered intradermally, subcutaneously or intramuscularly. In one instance, the composition is administered subcutaneously. In some instances, the composition is used for regenerating a tissue in the context of a cosmetic procedure or a surgical procedure. In one instance, the composition is used for regenerating a tissue in the context of a plastic surgery procedure. In some of these applications, the sNAG nanofibers are formulated and/or used as a tissue filler. In some of these applications, the sNAG nanofibers are formulated together with and/or used in combination with another tissue filler.

In certain embodiments, provided herein is a composition comprising sNAG nanofibers, wherein more than 50% of the sNAG nanofibers are between about 1 to 15 µm in length, and wherein the composition is formulated as a tissue filler (e.g., for cosmetic and/or surgical applications). In one instance, the composition is formulated as a filler for plastic surgery applications. For example, the composition may be formulated as a soft tissue filler or a hard tissue filler. In a specific example, the composition is formulated as a dermal filler. The tissue filler compositions comprising sNAG nanofibers can be used in any cosmetic or surgical procedure including, without limitation, for reducing wrinkles or depressions in the skin's surface, for adding volume to and/or altering the shape of the lips, for adding volume to any area of the face including cheeks or for restoring youthful fullness to the face, or for adding volume or restoring fullness to any part of the human body, e.g., neck.

In specific embodiments, provided herein is a method for adding volume to a tissue in a subject (e.g., a human subject), comprising administering a composition comprising sNAG nanofibers to the tissue (e.g., by injection into the tissue) in the subject, wherein more than 50% of the sNAG nanofibers are between about 1 to 15 µm in length. For example, such methods can be practiced in the context of a cosmetic procedure or a plastic surgery procedure. In some cases, the composition is administered by local injection into the tissue. In some cases, the composition is administered intradermally, subcutaneously or intramuscularly. In one embodiment, the composition is administered subcutaneously. In other embodiments, the composition is administered topically to the tissue, for example, in the context of surgery (e.g., plastic surgery) wherein the composition is administered topically to an exposed tissue.

In particular instances, the sNAG nanofiber composition is formulated with another agent known in the art to be useful as a tissue filler for cosmetic and/or surgical applications. For example, the composition can be formulated with or used in combination with hyaluronic acid, collagen, calcium hydroxylapatite, fat (e.g., human fat, in particular, autologous fat), polylactic acid, cadaveric dermis (e.g., human), polymethylmethacrylate, and/or polyalkylimide). In a particular instance, the composition is formulated with or used in combination with hyaluronic acid. In another instance, the composition is formulated with or used in combination with collagen. In yet another instance, the composition is formulated with or used in combination with autologous human fat. In one instance, the composition is formulated with or used in combination with botulinum toxin. In yet another instance, the sNAG nanofiber composition is not formulated with or used in combination with another tissue filler.

Disclosed herein is a method for treating or preventing wrinkles or depressions in the skin's surface in a subject (e.g., a human subject), comprising administering a composition comprising sNAG nanofibers to the subject, wherein more than 50% of the sNAG nanofibers are between about 1 to 15 µm in length. In a specific instance, the composition is administered topically to the subject. In other instances, the composition is administered by local injection. In some instances, the composition is administered intradermally, subcutaneously or intramuscularly. In one instance, the composition is administered subcutaneously. In particular instances, the composition is formulated and/or used as a dermal filler for treating or preventing wrinkles or depressions in the skin's surface in a subject (e.g., a human subject).

Disclosed herein is a method for treating wrinkles or depressions in the skin's surface in a human subject, comprising topically administering a composition comprising sNAG nanofibers to a human subject having wrinkles or depressions, wherein more than 50% of the sNAG nanofibers are between about 1 to 15 µm in length. In some instances, the composition is administered directly to the wrinkles or depressions in the skin's surface in a human subject. In another specific instance disclosed herein is a method for treating wrinkles or depressions in the skin's surface in a human subject, wherein the human subject has wrinkles or depressions in an area on the skin's surface, and wherein the composition comprising sNAG nanofibers is administered by local injection into the area under the skin's surface, wherein more than 50% of the sNAG nanofibers are between about 1 to 15 µm in length. In some instances, the composition is injected intradermally, subcutaneously or intramuscularly. In some instances, the composition is injected intradermally, subcutaneously or intramuscularly directly under the area on the skin surface having wrinkles or depressions in a human subject. In some instances, disclosed herein is a method for reducing or eliminating a wrinkle or a depression in an area on the skin's surface in a subject, comprising injecting a composition comprising sNAG nanofibers into the area (i.e., under the skin's surface), wherein more than 50% of the sNAG nanofibers are between about 1 to 15 µm in length. In certain embodiments described herein, sNAG nanofibers are effective in treating wrinkles or depressions in the skin's surface in a human subject. For example, sNAG nanofibers can be effective in reducing or eliminating a wrinkle or a depression in an area on the skin's surface in a subject.

Disclosed herein is a method of reducing scarring associated with wounds in a subject (e.g., a human subject), comprising administering a composition comprising sNAG nanofibers to a wound in the subject, wherein more than 50% of the sNAG nanofibers are between about 1 to 15 µm in length. In one instance, the wound is a surgical wound. In another instance, the wound is a cutaneous wound. In a specific case, the composition is administered topically to the subject. In one instance, the subject has a scar from a wound, and the sNAG nanofibers are administered topically to the area of the scar. In some instances, the composition is administered topically for at least or more than 10, 11, 12, 13, 14, 15, 16, 17, 18, 19, 20, 21, 22, 23, 24, 25, 26, 27, 28, 29 or 30 days (e.g., for at least or more than 21 days). In another instance, the subject has a scar from a wound, and the sNAG nanofibers are administered by local injection into the area of the scar. In certain instances described herein, sNAG nanofibers are effective in reducing scarring associated with wounds in a subject. For example, sNAG nanofibers can be effective in reducing scarring associated with a cutaneous wound in a subject.

Disclosed herein is a method of reducing scarring associated with cutaneous wounds in a human subject, comprising administering a composition comprising sNAG nanofibers to a cutaneous wound in a human subject, wherein more than 50% of the sNAG nanofibers are between about 1 to 15 µm in length. In a specific instance, the composition is administered topically to the subject. In one instance, the subject has a scar from a cutaneous wound, and the sNAG nanofibers are administered topically to the area of the scar. In some instances, the composition is administered topically for at least or more than 10, 11, 12, 13, 14, 15, 16, 17, 18, 19, 20, 21, 22, 23, 24, 25, 26, 27, 28, 29 or 30 days. In a particular case, the composition is administered topically for 21 days. In another instance, the subject has a scar from a cutaneous wound, and the sNAG nanofibers are administered by local injection into the area of the scar.

Disclosed herein is a method for treating or preventing adhesions associated with wounds in a subject (e.g., a human subject), comprising administering a composition comprising sNAG nanofibers to a wound in the subject, wherein more than 50% of the sNAG nanofibers are between about 1 to 15 µm in length. In one instance, the wound is a surgical wound. In another instance, the wound is a cutaneous wound. In a specific case, the composition is administered topically to the subject. In one instance, the subject has an adhesion from a wound, and the sNAG nanofibers are administered topically to the area of adhesion. In some instances, the composition is administered topically for at least or more than 10, 11, 12, 13, 14, 15, 16, 17, 18, 19, 20, 21, 22, 23, 24, 25, 26, 27, 28, 29 or 30 days (e.g., for at least or more than 21 days). In another instance, the subject has an adhesion from a wound, and the sNAG nanofibers are administered by local injection into the area of adhesion.

Disclosed herein is a method for treating or preventing fibrosis associated with wounds in a subject (e.g., a human subject), comprising administering a composition comprising sNAG nanofibers to a wound in the subject, wherein more than 50% of the sNAG nanofibers are between about 1 to 15 µm in length. In one instance, the wound is a surgical wound. In another instance, the wound is a cutaneous wound. In a specific case, the composition is administered topically to the subject. In one instance, the subject has fibrosis associated with a wound, and the sNAG nanofibers are administered topically to the area of fibrosis. In some instances, the composition is administered topically for at least or more than 10, 11, 12, 13, 14, 15, 16, 17, 18, 19, 20, 21, 22, 23, 24, 25, 26, 27, 28, 29 or 30 days (e.g., for at least or more than 21 days). In another instance, the subject has fibrosis associated with a wound, and the sNAG nanofibers are administered by local injection into the area of fibrosis.

In one embodiment, the sNAG nanofibers are not administered to a wound.

Disclosed herein is a method of treating a symptom of osteoporosis in a subject (e.g., a human subject), comprising administering a composition comprising sNAG nanofibers to the subject, wherein more than 50% of the sNAG nanofibers are between about 1 to 15 µm in length. In one instance, the composition is administered to an area of low bone density in a subject (e.g., a human subject). In another instance, the sNAG nanofibers are administered by local injection. In a specific instance, the composition is administered topically to the subject.

Disclosed herein is a method of treating a symptom of intervertebral disc disorder or degenerative disc disorder in a subject (e.g., a human subject), comprising topically administering a composition comprising sNAG nanofibers to the subject, wherein more than 50% of the sNAG nanofibers are between about 1 to 15 µm in length. In one instance, the composition is administered in the disc in the subject (e.g., a human subject) in the area of lower back pain. In another instance the composition is administered by local injection. In a specific case, the composition is administered topically to the subject.

Disclosed herein is a method for treating a symptom of osteoarthritis in a subject (e.g., a human subject), comprising administering a composition comprising sNAG nanofibers to the subject, wherein more than 50% of the sNAG nanofibers are between about 1 to 15 µm in length. In one instance, the sNAG nanofibers are administered topically to the joints of the subject (e.g., a human subject). In a specific instance, the composition is administered topically to the subject. In one instance, the sNAG nanofibers are administered by local injection to the joints of the subject (e.g., a human subject).

Disclosed herein is a method of treating fibrosis or a symptom of fibrosis in a subject (e.g., a human subject), comprising administering a composition comprising sNAG nanofibers to the subject, wherein more than 50% of the sNAG nanofibers are between 1 to 15 µm in length. In some instances, a composition comprising sNAG nanofibers is administered directly to the organ or tissue that is at risk of fibrosis or has fibrosis. In one instance, a composition comprising sNAG nanofibers is administered directly to the fibrotic tissue (e.g., on the skin). In a specific case, the composition is administered topically to the subject.

In some embodiments, the subject (e.g., human) treated in accordance with the methods described herein has an increased content or expression of collagen type I, a decreased content or expression of collagen type III, a decreased content or expression of elastin, and/or an increased content or expression of smooth muscle actin, in a tissue (e.g., skin). In further embodiments, the subject (e.g., human) treated in accordance with the methods described herein has decreased tensile strength of tissue (e.g., skin) and/or decreased elasticity of tissue (e.g., skin). In further embodiments, the subject (e.g., human) treated in accordance with the methods described herein has an increased myofibroblast content in a tissue (e.g., skin).

In certain embodiments, the sNAG nanofibers are non-reactive when tested in an intramuscular implantation test. In other embodiments, the sNAG nanofibers increase the metabolic rate of serum-starved human umbilical cord vein endothelial cells in a MTT assay and/or do not rescue apoptosis of serum-starved human umbilical cord endothelial cells in a trypan blue exclusion test. In further embodiments of the methods, more than 50% of the sNAG nanofibers are between 2 to 10 µm in length. In other embodiments, more than 50% of the sNAG nanofibers are between 4 to 7 µm in length. In other embodiments, 100% of the sNAG nanofibers are between 1 to 15 µm in length.

In specific embodiments of the methods described herein, the sNAG nanofibers were produced by gamma irradiation of poly-N-acetylglucosamine and/or a derivative thereof, and wherein the poly-β-N-acetylglucosamine and/or a derivative thereof was irradiated in the form of dried fibers at 500-2,000 kgy, or the poly-N-acetylglucosamine and/or a derivative thereof was irradiated in the form of wet fibers at 100-500 kgy. In particular embodiments, the sNAG nanofibers were produced from microalgal poly-N-acetylglucosamine. In further embodiments, the sNAG nanofibers comprise N-acetylglucosamine monosaccharides and/or glucosamine monosaccharides, wherein more than 70% of the monosaccharides of the sNAG nanofibers are N-acetylglucosamine monosaccharides. In other embodiments, the sNAG nanofibers comprise N-acetylglucosamine monosaccharides and/or glucosamine monosaccharides, wherein more than 90% of the monosaccharides of the sNAG nanofibers are N-acetylglucosamine monosaccharides. In still other embodiments, the sNAG nanofibers comprise N-acetylglucosamine monosaccharides and/or glucosamine monosaccharides, wherein more than 95% of the monosaccharides of the sNAG nanofibers are N-acetylglucosamine monosaccharides.

Disclosed herein are (the references below to the "item" number(s) refer to the subject matter recited in one or more of the preceding paragraphs identified by such number(s)):
1. A method for treating a symptom of Ehlers-Danlos syndrome in a human subject, comprising topically administering a composition comprising sNAG nanofibers to the human subject, wherein more than 50% of the sNAG nanofibers are between about 1 to 15 µm in length.
2. The method of item 1, wherein the symptom is a skin-related symptom.
3. The method of item 1, wherein the skin-related symptom is soft skin, fragile skin, skin that bruises easily, excessive scarring of the skin, or blunted wound healing in the skin.
4. The method of item 2 or 3, wherein the sNAG nanofibers are administered directly to the skin affected by the skin-related symptom.
5. A method for treating a symptom of scleroderma in a human subject, comprising topically administering a composition comprising sNAG nanofibers to the human subject, wherein more than 50% of the sNAG nanofibers are between about 1 to 15 µm in length.
6. The method of item 5, wherein the symptom is a skin-related symptom.
7. The method of item 6, wherein the skin-related symptom is swollen skin, thickened skin, shiny skin, discoloration of skin, or numbness of skin.
8. The method of item 6 or 7, wherein the sNAG nanofibers are administered directly to the skin affected by the skin-related symptom.
9. A method for treating a symptom of Epidermolysis bullosa in a human subject, comprising topically administering a composition comprising sNAG nanofibers to a human subject, wherein more than 50% of the sNAG nanofibers are between about 1 to 15 µm in length.
10. The method of item 9, wherein the symptom is a skin-related symptom or a mucosal membrane-related symptom.
11. The method of item 10, wherein the skin-related symptom or the mucosal membrane-related symptom is a blister.
12. The method of item 10 or 11, wherein the sNAG nanofibers are administered directly to the skin affected by the skin-related symptom or the mucosal membrane-related symptom.

Particular embodiments disclosed herein include:
13. A method for treating or preventing wrinkles or depressions in the skin's surface in a human subject, comprising topically administering a composition comprising sNAG nanofibers to the human subject, wherein more than 50% of the sNAG nanofibers are between about 1 to 15 µm in length.
14. A method of treating wrinkles or depressions in the skin's surface in a human subject, comprising topically administering a composition comprising sNAG nanofibers to a human subject having wrinkles or depressions, wherein more than 50% of the sNAG nanofibers are between about 1 to 15 µm in length.

Further disclosed herein are:
15. A method for reducing scarring associated with cutaneous wounds in a human subject, comprising topically administering a composition comprising sNAG nanofibers to a cutaneous wound in the human subject, wherein more than 50% of the sNAG nanofibers are between about 1 to 15 µm in length.
16. The method of item 15, wherein the subject has a scar from a cutaneous wound, and wherein the sNAG nanofibers are administered topically to the area of the scar.
17. The method of item 15 or 16, wherein the sNAG nanofibers are administered topically for 21 days.
18. A method for treating a symptom of osteoporosis in a human subject, comprising topically administering a composition comprising sNAG nanofibers to the human subject, wherein more than 50% of the sNAG nanofibers are between about 1 to 15 µm in length.
19. The method of item 18, wherein the sNAG nanofibers are administered to an area of low bone density in a human subject.
20. A method for treating a symptom of intervertebral disc disorder or degenerative disc disorder in a human subject, comprising topically administering a composition comprising sNAG nanofibers to the human subject, wherein more than 50% of the sNAG nanofibers are between about 1 to 15 µm in length.
21. The method of item 20, wherein the sNAG nanofibers are administered into the disc in the human subject in the area of lower back pain.
22. The method of any one of items 18-21, wherein the sNAG nanofibers are administered topically by local injection.
23. A method for treating a symptom of osteoarthritis in a human subject, comprising topically administering a composition comprising sNAG nanofibers to the human subject, wherein more than 50% of the sNAG nanofibers are between about 1 to 15 µm in length.
24. The method of item 23, wherein the sNAG nanofibers are administered topically to the joints of the human subject.

Further disclosed herein are the following items and with respect to items 13 and 14 embodiments:
25. The methods of any one of items 1-24, wherein the subject has increased total collagen content, increased expression of collagen type I, decreased expression of collagen type III, decreased expression of elastin, and/or increased expression of smooth muscle actin.
26. The methods of any one of items 1-25, wherein the subject has decreased tensile strength of the skin and/or decreased elasticity of the skin.
27. The method of any one of items 1-26, wherein the sNAG nanofibers are non-reactive when tested in an intramuscular implantation test.
28. The method of any one of items 1-27, wherein the sNAG nanofibers increase the metabolic rate of serum-starved human umbilical cord vein endothelial cells in a MTT assay and/or do not rescue apoptosis of serum-starved human umbilical cord endothelial cells in a trypan blue exclusion test.
29. The method of any one of items 1-28, wherein more than 50% of the sNAG nanofibers are between about 2 to 10 µm in length.
30. The method of any one of items 1-28, wherein more than 50% of the sNAG nanofibers are between about 4 to 7 µm in length.
31. The method of any one of items 1-28, wherein 100% of the sNAG nanofibers are between about 1 to 15 µm in length.
32. The method of any one of items 1-31, wherein the sNAG nanofibers were produced by gamma irradiation of poly-N-acetylglucosamine and/or a derivative thereof, and wherein the poly-β-N-acetylglucosamine and/or a derivative thereof was irradiated in the form of dried fibers at 500-2,000 kgy, or the poly-N-acetylglucosamine and/or a derivative thereof was irradiated in the form of wet fibers at 100-500 kgy.
33. The method of any one of items 1-32, wherein the sNAG nanofibers were produced from a microalgal poly-N-acetylglucosamine.
34. The method of any one of items 1-33, wherein the sNAG nanofibers comprise N-acetylglucosamine monosaccharides and/or glucosamine monosaccharides, and wherein more than 70% of the monosaccharides of the sNAG nanofibers are N-acetylglucosamine monosaccharides.
35. The method of any one of items 1-34, wherein the sNAG nanofibers comprise N-acetylglucosamine monosaccharides and/or glucosamine monosaccharides, and wherein more than 90% of the monosaccharides of the sNAG nanofibers are N-acetylglucosamine monosaccharides.
36. The method of any one of items 1-35, wherein the sNAG nanofibers comprise N-acetylglucosamine monosaccharides and/or glucosamine monosaccharides, and wherein more than 95% of the monosaccharides of the sNAG nanofibers are N-acetylglucosamine monosaccharides.

Other particular embodiments contemplated herein include, without limitation (the references below to the "item" number(s) refer to the subject matter recited in one or more of the preceding paragraphs identified by such number(s)):
1. A method for treating or preventing wrinkles or depressions in the skin's surface in a human subject, comprising administering a composition comprising sNAG nanofibers to the human subject, wherein more than 50% of the sNAG nanofibers are between about 1 to 15 µm in length.
2. The method of item 1, which is a method for treating wrinkles or depressions in the skin's surface, wherein the human subject has wrinkles or depressions in an area on the skin's surface, and wherein the composition is administered to the area.
3. The method of item 1 or 2, wherein the composition is administered topically.
4. The method of item 2, wherein the composition is administered by local injection into the area under the skin's surface, and wherein the local injection is an intradermal injection, subcutaneous injection or an intramuscular injection.

Further disclosed herein are:
5. A method for regenerating a tissue or adding volume to a tissue in a human subject, comprising administering a composition comprising sNAG nanofibers to the tissue in the human subject, wherein more than 50% of the sNAG nanofibers are between about 1 to 15 µm in length.
6. The method of item 5, wherein the administering is performed by local injection into the tissue, and wherein the local injection is an intradermal injection, subcutaneous injection or an intramuscular injection.
7. A method for reducing scarring, reducing fibrosis or reducing adhesions associated with a wound in a human subject, comprising topically administering a composition comprising sNAG nanofibers to the wound in the human subject, wherein more than 50% of the sNAG nanofibers are between about 1 to 15 µm in length.
8. The method of item 7, wherein the subject has a scar, fibrosis or an adhesion from a wound, and wherein the sNAG nanofibers are administered topically to the area of the scar, fibrosis or adhesion.
9. The method of item 7 or 8, wherein the wound is a cutaneous wound or a surgical wound.
10. The method of any one of items 7-9, wherein the sNAG nanofibers are administered topically for 21 days.
11. The method of any one of items 7-10, which is a method for reducing scarring.
12. A method for treating a symptom of a disease in a human subject, wherein the disease is Ehlers-Danlos syndrome, scleroderma, Epidermolysis bullosa, osteoporosis, intravertebral disc disorder, degenerative disc disorder or osteoarthritis, comprising topically administering a composition comprising sNAG nanofibers to the human subject, wherein more than 50% of the sNAG nanofibers are between about 1 to 15 µm in length.
13. The method of item 12, wherein the disease is Ehlers-Danlos syndrome, scleroderma, or Epidermolysis bullosa, and wherein the symptom is a skin-related symptom.
14. The method of item 13, wherein the disease is Ehlers-Danlos syndrome, and wherein the skin-related symptom is soft skin, fragile skin, skin that bruises easily, excessive scarring of the skin, or blunted wound healing in the skin.
15. The method of item 13, wherein the disease is scleroderma, and the skin-related symptom is swollen skin, thickened skin, shiny skin, discoloration of skin, or numbness of skin.
16. The method of item 12, wherein the disease is Epidermolysis bullosa, and wherein the symptom is a mucosal membrane-related symptom, optionally, a blister.
17. The method of any one of items 12-16, wherein the sNAG nanofibers are administered directly to the area affected by the symptom.
18. The method of item 12, wherein the disease is osteoporosis, and wherein the sNAG nanofibers are administered to an area of low bone density in the human subject.
19. The method of item 12, wherein the disease is an intervertebral disc disorder or degenerative disc disorder, and wherein the sNAG nanofibers are administered into the disc in the human subject in the area of lower back pain.
20. The method of item 18 or 19, wherein the sNAG nanofibers are administered by local injection.
21. The method of item 12, wherein the disease is osteoarthritis, and wherein the sNAG nanofibers are administered topically to the joints of the human subject.

Further disclosed herein are the following items and with respect to items 1 to 4 embodiments:
22. The methods of any one of items 1-21, wherein the subject has an increased total collagen content, increased expression of collagen type I, decreased expression of collagen type III, decreased expression of elastin, increased expression of smooth muscle actin, decreased tensile strength of the skin and/or decreased elasticity of the skin.
23. The method of any one of items 1-22, wherein the sNAG nanofibers are non-reactive when tested in an intramuscular implantation test.
24. The method of any one of items 1-23, wherein the sNAG nanofibers increase the metabolic rate of serum-starved human umbilical cord vein endothelial cells in a MTT assay and/or do not rescue apoptosis of serum-starved human umbilical cord endothelial cells in a trypan blue exclusion test.
25. The method of any one of items 1-24, wherein more than 50% of the sNAG nanofibers are between about 2 to 10 µm in length or between about 4 to 7 µm in length, or wherein 100% of the sNAG nanofibers are between about 1 to 15 µm in length.
26. The method of any one of items 1-25, wherein the sNAG nanofibers were produced by gamma irradiation of poly-N-acetylglucosamine and/or a derivative thereof, and wherein the poly-β-N-acetylglucosamine and/or a derivative thereof was irradiated in the form of dried fibers at 500-2,000 kgy, or the poly-N-acetylglucosamine and/or a derivative thereof was irradiated in the form of wet fibers at 100-500 kgy.
27. The method of any one of items 1-26, wherein the sNAG nanofibers were produced from a microalgal poly-N-acetylglucosamine.
28. The method of any one of items 1-27, wherein the sNAG nanofibers comprise N-acetylglucosamine monosaccharides and/or glucosamine monosaccharides, and wherein more than 70%, more than 90% or more than 95%, of the monosaccharides of the sNAG nanofibers are N-acetylglucosamine monosaccharides.

### 3.1 Terminology

As used herein, the terms "sNAG nanofiber," "sNAG," "Taliderm," or "Talymed" (formerly known as "Taliderm") are used interchangeably to refer to shortened fibers of poly-N-acetylglucosamine and/or derivatives thereof. In a preferred embodiment, sNAG nanofibers consist entirely of shortened fibers of poly-N-acetylglucosamine and/or derivatives thereof. Taliderm or Talymed are examples of sNAG nanofibers which are membranes consisting entirely of shortened fibers of poly-N-acetylglucosamine and/or derivatives thereof.

As used herein, the term "about" means a range around a given value wherein the resulting value is the same or substantially the same (e.g., within 10%, 5% or 1%) as the expressly recited value. In one embodiment, "about" means within 10% of a given value or range. In another embodiment, the term "about" means within 5% of a given value or range. In another embodiment, the term "about" means within 1% of a given value or range.

As used herein, the terms "disease" and "disorder" are used interchangeably to refer to a condition in a subject. Exemplary diseases/disorders that can be treated or prevented in accordance with the methods described herein include, without limitation, Ehlers-Danlos Syndrome, Epidermolysis bullosa, scleroderma, osteoporosis, intervertebral disc disorder, degenerative disc disorder, osteoarthritis, fibrosis, wrinkling of the skin, and scarring associated with wounds.

As used herein, the term "subject" and "patient" are used interchangeably to refer to an animal (e.g., cow, horse, sheep, pig, chicken, turkey, quail, cat, dog, mouse, rat, rabbit, guinea pig, etc.). In some instances, the subject is a mammal such as a non-primate and a primate (e.g., monkey and human). In specific embodiments, the subject is a human.

As used herein, the term "effective amount" in the context of administering a sNAG nanofiber or composition thereof to a subject refers to the amount of a sNAG nanofiber or composition thereof that results in a beneficial or therapeutic effect. In specific embodiments, an "effective amount" of a sNAG nanofiber or composition thereof refers to an amount of a sNAG nanofiber or composition thereof which is sufficient to achieve at least one, two, three, four or more of the following effects: (i) reduction or amelioration of the severity of a disease in the subject or population of subjects or a symptom associated therewith; (ii) reduction of the duration of a symptom associated with a disease; (iii) prevention of the progression of a disease in the subject or population of subjects or a symptom associated therewith; (iv) regression of a symptom associated with a disease; (v) prevention of the development or onset of a symptom associated with a disease; (vi) prevention of the recurrence of a symptom associated with a disease; (vii) reduction of the incidence of hospitalization of the subject or population of subjects; (viii) reduction of the hospitalization length of the subject or population of subjects; (ix) an increase the survival of the subject or population of subjects; (x) elimination of a condition in the subject or population of subjects; (xi) enhancement or improvement of the prophylactic or therapeutic effect(s) of another therapy in the subject or population of subjects; (xii) reduction of the number of symptoms of a disease in the subject or population of subjects; (xiiii) the increase in the tensile strength of a tissue in a subject; (xiv) the increase in elasticity in a tissue of a subject; (xv) the increase in elastin content or production in a tissue of a subject; (xvi) the reduction in scar size in a tissue of a subject; (xvii) the decrease in total collagen content in a tissue of a subject; (xviii) the decrease of collagen I expression or content in a tissue of a subject; (xix) the increase in collagen III expression or content in a tissue of a subject; (xx) the inducement of more organized collagen alignment in a tissue of a subject; (xxi) the reduction in smooth muscle actin content or expression, or the reduction in myofibroblast content in a tissue of a subject; (xxii) the prevention of the onset, development or recurrence of a condition caused by or associated with one or more of: decreased tensile strength of tissue, decreased elasticity of tissue, increased collagen content or abnormal collagen content in tissue, increased collagen I expression in tissue, decreased collagen III expression in tissue, abnormal alignment of collagen in tissue, increased smooth muscle actin expression in tissue, and increased myofibroblast content in tissue; and/or (xxiii) improvement in quality of life as assessed by methods well known in the art, *e.g.,* a questionnaire. In specific embodiments, an "effective amount" of a sNAG nanofiber refers to an amount of a sNAG nanofiber composition specified herein, *e.g.,* in Section 5.6, *infra.*

As used herein, the term "premature human infant" refers to a human infant born at less than 37 weeks of gestational age.

As used herein, the term "human infant" refers to a newborn to 1 year old human.

As used herein, the term "premature human infant" refers to a newborn to 1 year old year human who was born of less than 37 weeks gestational age (e.g., before 37 weeks, 36 weeks, 35 weeks, 34 weeks, 33 weeks, 32 weeks, 31 weeks, 30 weeks, 29 weeks, 28 weeks, or less than 28 weeks of pregnancy).

As used herein, the term "human toddler" refers to a human that is 1 years to 3 years old.

As used herein, the term "human child" refers to a human that is 1 year to 18 years old.

As used herein, the term "human adult" refers to a human that is 18 years or older.

As used herein, the term "elderly human" refers to a human 65 years or older.

As used herein the "normal" expression of one or more gene products is: (i) the average expression level known to be found in subjects not displaying symptoms or not diagnosed with the condition and disease to be treated; (ii) the average expression level detected in three, five, ten, twenty, twenty-five, fifty or more subjects not displaying symptoms or not diagnosed with the condition and disease to be treated; and/or (iii) the level of expression detected in a patient to be administered a composition described herein before the onset of the condition and disease.

As used herein, the term "low expression," or "low level of expression" in the context of expression of a gene (e.g., based on the level of protein, peptide and/or mRNA produced by the gene) refers to an expression that is less than the "normal" expression of the gene. In a specific embodiment, "low expression" refers to expression of a gene that is less than 99%, less than 95%, less than 90%, less than 85%, less than 75%, less than 70%, less than 65%, less than 60%, less than 55%, less than 50%, less than 45%, less than 40%, less than 35%, less than 30%, less than 25%, or less than 20% of the "normal" expression of the gene. In another specific embodiment, "low expression" refers to expression of a gene that is about 20-fold, about 15-fold, about 10-fold, about 5-fold, about 4-fold, about 3-fold, about 2-fold, or about 1.5 fold less than the "normal" expression of the gene. In further embodiments, "low expression" refers to expression of a gene that is more than about 1.25 fold, 1.5 fold, 2 fold, 2.5 fold, 3 fold, 3.5 fold, 4 fold, 4.5 fold, 5 fold, 6 fold, 7 fold, 8 fold, 9 fold, 10 fold lower than "normal" expression of a gene.

As used herein, the term "high expression", or "high level of expression" in the context of expression of a gene (e.g., based on the level of protein, peptide and/or mRNA produced by the gene) refers to an expression that is more than the "normal" expression of the gene. In a specific embodiment, "high expression" refers to expression of a gene that is more than 99%, more than 95%, more than 90%, more than 85%, more than 75%, more than 70%, more than 65%, more than 60%, more than 55%, more than 50%, more than 45%, more than 40%, more than 35%, more than 30%, more than 25%, or more than 20% of the "normal" expression of the gene. In another specific embodiment, "high expression" refers to expression of a gene that is about 20-fold, about 15-fold, about 10-fold, about 5-fold, about 4-fold, about 3-fold, about 2-fold, or about 1.5 fold more than the "normal" expression of the gene. In further embodiments, "high expression" refers to expression of a gene that is more than about 1.25 fold, 1.5 fold, 2 fold, 2.5 fold, 3 fold, 3.5 fold, 4 fold, 4.5 fold, 5 fold, 6 fold, 7 fold, 8 fold, 9 fold, 10 fold higher than "normal" expression of a gene.

As used herein, the term "altered expression" or "altered level of expression" of a gene product is a level that differs (e.g., by more than 20%, 25%, 30%, 50%, 75%, 100%, 150%, 200%, 250%, 300%) from the normal level of expression of the gene.

As used herein, the term "majority" refers to greater than 50%, including, *e.g.,* 50.5%, 51%, 55%, 60%, 65%, 70%, 75%, 80%, 85%, 90%, 95%, 98%, 99%, etc.

As used herein, the terms "therapies" and "therapy" can refer to any protocol(s), method(s), compositions, formulations, and/or agent(s) that can be used in the prevention and/or treatment of any disease or disorder associated with decreased tensile strength or elasticity of tissue, increased total collagen content in tissue, increased collagen type I content (e.g., expression) in tissue, decreased collagen type III content in tissue, abnormal (e.g., disorganized) collagen alignment in tissue, decreased elastin content (e.g., expression) in tissue, increased myofibroblast content in tissue, and/or increased alpha smooth muscle actin content (e.g., expression) in tissue. Examples of diseases or disorders include, without limitation, Ehlers-Danlos Syndrome, Epidermolysis bullosa, scleroderma, osteoporosis, intervertebral disc disorder, degenerative disc disorder, osteoarthritis, fibrosis, wrinkling of the skin, and scarring associated with wounds. In certain embodiments, the terms "therapies" and "therapy" refer to drug therapy, adjuvant therapy, radiation, surgery, biological therapy, supportive therapy, and/or other therapies useful in treatment and/or prevention of the diseases or disorders listed herein. In certain embodiments, the term "therapy" refers to a therapy other than a sNAG nanofiber or a composition thereof. In specific embodiments, an "additional therapy" and "additional therapies" refer to a therapy other than a treatment using a sNAG nanofiber or a composition thereof. In a specific embodiment, a therapy includes the use of a sNAG nanofiber as an adjuvant therapy. For example, using a sNAG nanofiber in conjunction with a drug therapy, biological therapy, surgery, and/or supportive therapy.

### 4. BRIEF DESCRIPTION OF FIGURES

**Figure 1.** sNAG nanofibers increase tensile strength (relative stress) and elasticity of tissue. On day 21 post-wounding, wounds, both treated and untreated and unwounded control skin were harvested and subjected to tensile strength and elasticity testing using an Instron 5942 strain gauge extensometer and Bluehill 3 Testing Software. Tensile strength of the skin was determined by measuring the relative stress the skin could bear before breaking 20% and elasticity was measured in the mm extension. (A) Tensile strength measurement. (B) Elasticity measurement.
**Figure 2.** sNAG nanofibers increase elastin production in tissue. On day 10 post-wounding, tissue sections from wounded animals treated with sNAG and control (untreated) were stained for elastin fibers using Van Geison staining procedures.
**Figure 3.** sNAG nanofibers reduce scar size in tissue. On day 21 post-wounding, scars of wounded animals treated with sNAG and control (untreated) were measured using a caliper.
**Figure 4.** sNAG nanofibers increase amount of collagen and induce an organized alignment of collagen. (A) Masson's Trichrome stain of tissue section from wounds treated with sNAG and control (untreated), 10 days post-wounding. (b) Hydroxyproline assay quantitatively analyzing the amount of collagen deposition in wounds treated with sNAG and control (untreated), 10 days post-wounding.
**Figure 5.** sNAG nanofibers decrease collagen I expression and increase collagen III expression. RNA isolated from wounds sNAG treated and untreated (control) at day 5 post wounding were tested for expression of collagen type I and collagen type III by RT-PCR.
**Figure 6.** sNAG nanofibers decrease α- smooth muscle actin. (A) Wound sections treated with sNAG or untreated were labeled with an antibody directed against α- smooth muscle actin. (B) Quantification of the expression of α- smooth muscle actin in wound sections treated with sNAG or untreated.
**Figure 7.** sNAG treatment results in decreased scar size and more organized collagen alignment. (A) Quantification of scar size from excisional wounds of Wild Type male mice (n=3) that were either treated with sNAG membrane or left untreated. Scars were measured after 21 days of healing. (B) Masson Trichrome staining of paraffin embedded sections of cutaneous wounds harvested on day 10 post wounding from both WT untreated (n=3) and sNAG treated (n=3) mice. The 4x magnification illustrates the entire skin section while the 20x magnification is focused on the regenerating tissue directly under the wounded area. Arrows illustrate collagen fiber alignment. (C) Quantitative analysis of collagen content in sNAG treated wounds compared to untreated wounds of WT mice using a hydroxyproline assay (p<0.05).
**Figure 8.** sNAG treatment results in decreased alpha smooth muscle expression. (A) Paraffin embedded sections of cutaneous wounds harvested on day 10 post wounding from both sNAG treated (n=3) and untreated (n=3) WT mice. Immunofluorescence (20X) was performed using antibodies directed against α-SMA, and TOPRO. White arrows indicate vasculature that is also positively stained with α-SMA antibodies. (B) Quantitation of α-SMA expression from paraffin embedded sections was performed using NIH ImageJ software. (*p<.05)
**Figure 9.** sNAG treatment causes increased tensile strength and elasticity of wounded skin. (A) Quantitation of the relative stress wounded skin could withstand from sNAG treated (n=3) and untreated (n=3) WT mice. Tissue was harvested 21 days post wounding and subjected to mechanical testing. Measurements are relative to control (CTRL) skin which was derived from unwounded tissue of WT animals (n=3). (B) Quantitation of skin elasticity from sNAG treated (n=3) and untreated (n=3) wounds harvested 21 days post wounding from WT animals. Control skin was derived from unwounded tissue of WT animals (n=3). (C) Van Gieson staining of paraffin embedded tissue sections derived from unwounded skin (control), sNAG treated, and untreated wounds of WT animals 10 days post wounding. Arrows indicate darkly stained elastin fibers. (*p<.05, **p<.01)
**Figure 10.** sNAG treatment results in increased fibroblast alignment. (A) Representative images of Hematoxylin and Eosin stained sections of paraffin embedded fibrin gels. Fibroblasts were serum starved and either left untreated or stimulated with sNAG (50 µg/mL) overnight prior to embedding in fibrin gels. Black circles indicate where minutien pins are located in the fibrin gels to serve as tension points along which the gel contracts. (B) Immunofluorescent staining of paraffin embedded fibrin gels. Sections are labeled with phalloidin and DAPI.
**Figure 11.** The sNAG dependent alignment of fibroblasts requires Akt1. (A) Quantification of contraction of fibrin gels that were embedded with serum starved fibroblasts, sNAG treated fibroblasts, or fibroblasts that were transduced with a lentivirus directed against Aktl prior to sNAG stimulation (50µg/mL). (B) Representative images from Hematoxylin and Eosin stained sections of paraffin embedded fibrin gels. Fibroblasts were serum starved and transduced with either Scrambled control or Aktl lentivirus prior to sNAG stimulation (50µg/mL). (C) Immunofluorescence of both serum starved (untreated) or sNAG treated fibrin embedded fibroblasts using an antibody directed against phospho-Akt and DAPI. (D) Quantitation of the relative stress wounded skin could withstand from sNAG treated (n=3) and untreated (n=3) Aktl null animals. Tissue was harvested 21 days post wounded and subjected to mechanical testing. Measurements are relative to control (CTRL) skin which was derived from unwounded tissue of Aktl-/- animals (n=3). (*p<.05)

### 5. DETAILED DESCRIPTION

The inventors of the present invention have found that sNAG nanofibers can increase tensile strength of tissue, increase elasticity of tissue, decrease total collagen content or abnormal collagen content in tissue, decrease collagen type I expression in tissue, increase collagen type III expression in tissue, induce organized alignment of collagen in tissue, increase elastin production in tissue, decrease smooth muscle actin expression in tissue, and/or decrease myofibroblast content in tissue. In particular, as demonstrated in the examples presented in Section 6, *infra,* the inventors of the present invention have found that sNAG nanofibers can increase tensile strength, increase elasticity, increase elastin production, decrease total collagen content, decrease collagen type I expression, increase collagen type III expression, induce organized alignment of collagen, and decrease alpha smooth muscle actin during cutaneous wound healing.

Thus, without being bound by any mechanism of action, sNAG nanofibers may act in the treatment of any conditions and diseases that are associated with decreased tensile strength of tissue, decreased elasticity of tissue, decreased elastin content (e.g., expression) in tissue, increased total collagen content or abnormal collagen content in tissue, increased collagen type I expression in tissue, decreased collagen type III expression in tissue, abnormal alignment of collagen in tissue, increased smooth muscle actin expression in tissue, and/or increased myofibroblast content in tissue. sNAG nanofibers may act in the treatment of any conditions, disorders and diseases that are associated with decreased tensile strength of the skin, decreased elasticity of the skin, decreased elastin content (e.g., expression) in the skin, increased total collagen content or abnormal collagen content in the skin, increased collagen type I content (e.g., expression) in the skin, decreased collagen type III content (e.g., expression) in the skin, abnormal (e.g., disorganized) alignment of collagen in the skin, increased smooth muscle actin expression in the skin, and/or increased myofibroblast content in the skin. In some embodiments, sNAG nanofibers may act to increase tensile strength, mediate organized alignment of collagen in the cells, increase elasticity and/or increase elastin production in the skin. sNAG nanofibers may act in the treatment of any conditions, disorders and diseases that are associated with cutaneous wound healing. sNAG nanofibers may act to decrease scarring, increase tensile strength and/or mediate organized alignment of cells or collagen in the cells during cutaneous wound healing.

Accordingly, described herein is the use of sNAG nanofibers in methods for preventing and/or treating of any condition and disease associated with decreased tensile strength of tissue, decreased elasticity of tissue, decreased elastin content (e.g., expression) in tissue, increased total collagen content or abnormal collagen content in tissue, increased collagen type I content (e.g., expression) in tissue, decreased collagen type III content (e.g., expression) in tissue, abnormal (e.g., disorganized) alignment of collagen in tissue, increased smooth muscle actin content (e.g., expression) in tissue (such as, increased alpha smooth muscle actin content in tissue), and/or increased myofibroblast content in tissue. In particular, described herein are topical uses of sNAG nanofibers in methods for preventing and/or treating of any condition, disorder or disease associated with decreased tensile strength of tissue, decreased elasticity of tissue, decreased elastin content (e.g., expression) in tissue, increased total collagen content or abnormal collagen content in tissue, increased collagen type I content (e.g., expression) in tissue, decreased collagen type III content (e.g., expression) in tissue, abnormal (e.g., disorganized) alignment of collagen in tissue, increased smooth muscle actin (e.g., alpha smooth muscle actin) content (e.g., expression) in tissue, and/or increased myofibroblast content in tissue. Also described herein is the use of the sNAG nanofibers in the methods for decreasing scarring, increasing elasticity, or increasing tensile strength of the skin. Also described herein is the use of the sNAG nanofibers in the methods for regenerating a tissue or adding volume to a tissue in a human subject (e.g., use of the sNAG nanofibers as a tissue filler).

In a particular embodiment, described herein is the use of the sNAG nanofibers in the methods for preventing or treating wrinkles or scars in the skin of a patient. In other instances, described herein is the use of sNAG nanofibers in the methods for decreasing scarring associated with cutaneous wounds using sNAG nanofibers. In some embodiments, described herein is the use of the sNAG nanofibers in methods for treatment of wrinkles, scars or cutaneous wounds in a patient, wherein the patient has decreased tensile strength of tissue, decreased elasticity of tissue, decreased elastin content in tissue, increased total collagen content or abnormal collagen content in tissue, increased collagen type I expression in tissue, decreased collagen type III expression in tissue, abnormal alignment of collagen in tissue, increased alpha smooth muscle actin, or increased myofibroblast content. In other instances, described herein is the use of sNAG nanofibers in the methods for treatment of Ehlers-Danlos Syndrome, Epidermolysis bullosa, scleroderma, osteoporosis, intervertebral disc disorder, degenerative disc disorder, osteoarthritis, or fibrosis. For example, the sNAG nanofibers may be used to reduce one or more symptoms of the above-listed disorders or diseases.

### 5.1 sNAG Nanofibers

Described herein are sNAG nanofiber compositions. The sNAG nanofibers comprise fibers of poly-N-acetylglucosamine and/or a derivative(s) thereof, the majority of which are less than 30 microns in length and at least 1 micron in length as measured by any method known to one skilled in the art, for example, by scanning electron microscopy ("SEM"). Such sNAG nanofibers may be obtained, for example, as described herein.

In certain embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are less than about 30, 25, 20, 15, 12, 10, 9, 8, 7, 6, 5, 4, or 3 microns in length, and at least 1 micron in length as measured by any method known to one skilled in the art, for example, by SEM. In specific embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are less than about 15 microns or less than about 12 microns in length, and at least 1 micron in length as measured by any method known to one skilled in the art, for example, by SEM. In specific embodiments, all (100%) of the sNAG nanofibers are less than about 15 microns or less than about 10 microns in length, and at least 1 micron in length as measured by any method known to one skilled in the art, for example, by SEM. In certain embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are equal to or less than 14, 13, 12, 11, 10, 9, 8 or 7 microns in length, and at least 1 micron in length as measured by any method known to one skilled in the art, for example, by SEM. In some embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are between 1 to 15, 2 to 15, 2 to 14, 1 to 12, 2 to 12, 1 to 10, 2 to 10, 3 to 12, 3 to 10, 4 to 12, 4 to 10, 5 to 12, 5 to 10, 1 to 9, 2 to 9, 3 to 9, 1 to 8, 2 to 8, 3 to 8, 4 to 8, 1 to 7, 2 to 7, 3 to 7, 4 to 7, 1 to 6, 1 to 5, 1 to 4, or 1 to 3 microns in length as measured by any method known to one skilled in the art, for example, by SEM.

In a specific embodiment, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are about 8, 7, 6, 5, 4, 3 or 2 microns in length as measured by any method known to one skilled in the art, for example, by SEM. In another specific embodiment, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are between about 2 to about 10 microns, about 3 to about 8 microns, about 4 to about 7 microns, about 4 to about 10 microns, or about 5 to about 10 microns in length as measured by any method known to one skilled in the art, for example, by SEM. In another specific embodiment, all (100%) of the sNAG nanofibers are between about 2 to about 10 microns, about 3 to about 8 microns, about 4 to about 7 microns, about 4 to about 10 microns, or about 5 to about 10 microns in length as measured by any method known to one skilled in the art, for example, by SEM.

In certain embodiments, the sNAG nanofibers fibers are in a range between 0.005 to 5 microns in thickness and/or diameter as determined by electron microscopy. In specific embodiments, the sNAG nanofibers are about 0.01, 0.02, 0.03, 0.04, 0.05, 0.06, 0.07, 0.08, 0.09, 0.1, 0.2, 0.25, 0.3, 0.35, 0.4, 0.45, 0.5, 0.55, 0.6, 0.65, 0.7, 0.75, 0.8, 0.85, 0.9, 1, 1.1, 1.2, 1.3, 1.4, 1.5, 1.6, 1.7, 1.8, 1.9, 2, 2.2, 2.4, 2.6, 2.8, 3 or 4 microns in thickness and/or diameter on average, or any range in between (e.g., 0.02 to 2 microns, 0.02 to 1 microns, 0.02 to 0.75 microns, 0.02 to 0.5 microns, 0.02 to 0.5 microns, 0.05 to 1 microns, 0.05 to 0.75 microns, 0.05 to 0.5 microns, 0.1 to 1 microns, 0.1 to 0.75 microns, 0.1 to 0.5 microns, etc.). In specific embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers have a thickness or diameter of about 0.02 to 1 microns. In other specific embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers have a thickness or diameter of about 0.05 to 0.5 microns. In specific embodiments, all (100%) of the sNAG nanofibers have a thickness or diameter of about 0.02 to 1 microns or about 0.05 to 0.5 microns. In certain embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers have a thickness or diameter of about 0.02 to 2 microns, 0.02 to 1 microns, 0.02 to 0.75 microns, 0.02 to 0.5 microns, 0.02 to 0.5 microns, 0.05 to 1 microns, 0.05 to 0.75 microns, 0.05 to 0.5 microns, 0.1 to 1 microns, 0.1 to 0.75 microns, or 0.1 to 0.5 microns.

In certain embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers are between 1 and 15 microns, or between (or in the range of) 1 to 10 microns, 2 to 10 microns, 3 to 10 microns, 4 to 10 microns, 4 to 7 microns, 5 to 10 microns, or 5 to 15 microns in length and have a thickness or diameter of about 0.02 to 1 microns.

In certain embodiments, the molecular weight of the sNAG nanofibers is less than 100 kDa, 90 kDa, 80 kDa, 75 kDa, 70 kDa, 65 kDa, 60 kDa, 55 kDa, 50 kDa, 45 kDA, 40 kDa, 35 kDa, 30 kDa, or 25 kDa. In certain embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers have a molecular weight of less than 100kDa, 90kDa, 80kDa, 75kDa, 70 kDa, 65 kDa, 60kDa, 55kDa, 50kDa, 45 kDA, 40 kDa, 35 kDa, 30 kDa, or 25 kDa. In other embodiments, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers have a molecular weight between about 5kDa to 100kDa, about 10kDa to 100kDa, about 20kDa to 100kDa, about 10kDa to 80kDa, about 20kDa to 80kDa, 20kDa to 75kDa, about 25kDa to about 75kDa, about 30kDa to about 80kDa, about 30kDa to about 75kDa, about 40kda to about 80kDa, about 40kDa to about 75kDa, about 40kDa to about 70kDa, about 50kDa to about 70kDa, or about 55kDa to about 65kDa. In one embodiment, the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the sNAG nanofibers have a molecular weight of about 60kDa.

In certain instances, 1% to 5%, 5% to 10%, 5% to 15%, 20% to 30% or 25% to 30% of the sNAG nanofibers are deacetylated. In some instances, 1%, 5%, 10%, 15%, 20%, 25%, or 30% of the sNAG nanofibers are deacetylated. In other instances, less than 30%, 25%, 20%, 15%, 10%, 5%, 4%, 3%, 2% or 1% of the sNAG nanofibers are deacetylated. In some instances, equal to or more than 1%, 5%, 10%, 15%, 20%, 25%, 30%, 35%, 40%, 45%, 50%, 55%, 60%, 65%, 70%, 75%, 80%, 85%, 90%, 95% or 99%, or all (100%), of the sNAG nanofibers are deacetylated. In other instances, less than 1%, 5%, 10%, 15%, 20%, 25%, 30%, 35%, 40%, 45%, 50%, 55%, 60%, 65%, 70%, 75%, 80%, 85%, 90%, 95%, 99%, or 100% of the sNAG nanofibers are deacetylated.

In certain instances, 70% to 80%, 75% to 80%, 75% to 85%, 85% to 95%, 90% to 95%, 90% to 99% or 95% to 100% of the sNAG nanofibers are acetylated. In some instances, 70%, 75%, 80%, 85%, 90%, 95%, 98%, 99% or 100% of the sNAG nanofibers are acetylated. In other instances, more than 70%, 75%, 80%, 85%, 90%, 95%, 97%, 98%, 99%, 99.5% or 99.9% of the sNAG nanofibers are acetylated. In some instances, equal to or more than 1%, 5%, 10%, 15%, 20%, 25%, 30%, 35%, 40%, 45%, 50%, 55%, 60%, 65%, 70%, 75%, 80%, 85%, 90%, 95%, 97%, 98% or 99%, or all (100%), of the sNAG nanofibers are acetylated. In other instances, less than 1%, 5%, 10%, 15%, 20%, 25%, 30%, 35%, 40%, 45%, 50%, 55%, 60%, 65%, 70%, 75%, 80%, 85%, 90%, 95%, 97%, 98%, 99%, or 100% of the sNAG nanofibers are acetylated.

In some instances, the majority (and in certain embodiments, at least 60%, 70%, 80%, 90%, 95%, 98%, 99%, 99.5%, 99.9%, or 100%) of the sNAG nanofibers are between (or in the range of) 2 to 12 microns, 2 to 10 microns, 4 to 15 microns, 4 to 10 microns, 5 to 15 microns, or 5 to 10 microns, and such sNAG nanofibers are at least 70%, 75%, 80%, 85%, 90%, 95%, 97%, 98%, 99% or 100% acetylated.

In some instances, the sNAG nanofibers comprise at least one glucosamine monosaccharide, and may further comprise at least 10%, 20%, 30%, 40%, 50%, 60%, 70%, 80%, 90%, 95% or 99% of the N-acetylglucosamine monosaccharides. In other instances, the sNAG nanofibers comprise at least one N-acetylglucosamine monosaccharide, and may further comprise at least 10%, 20%, 30%, 40%, 50%, 60%, 70%, 80%, 90%, 95% or 99% of glucosamine monosaccharides.

In one embodiment, the sNAG nanofibers increase the metabolic rate of serum-starved human umbilical cord vein endothelial cells ("EC") in a MTT assay. A MTT assay is a laboratory test and a standard colorimetric assay (an assay which measures changes in color) for measuring cellular proliferation (cell growth). Briefly, yellow MTT (3-(4,5-Dimethylthiazol-2-yl)-2,5-diphenyltetrazolium bromide, a tetrazole) is reduced to purple formazan in the mitochondria of living cells. This reduction takes place only when mitochondrial reductase enzymes are active, and therefore conversion can be directly related to the number of viable (living) cells. The MTT assay is described in WO2011/130646 and WO2012/142581. The metabolic rate of cells may also be determined by other techniques commonly known to the skilled artisan.

In another embodiment, the sNAG nanofibers do not rescue apoptosis of serum-starved EC in a trypan blue exclusion test. A trypan blue exclusion test is a dye exclusion test used to determine the number of viable cells present in a cell suspension. It is based on the principle that live cells possess intact cell membranes that exclude certain dyes, such as trypan blue, Eosin, or propidium, whereas dead cells do not. The trypan blue assay is described in WO2011/130646 and WO2012/142581. The viability of cells may also be determined by other techniques commonly known to the skilled artisan.

In certain embodiments, compositions comprising the sNAG nanofibers are described, wherein the sNAG nanofibers increase the metabolic rate of serum-starved human umbilical cord vein endothelial cells in a MTT assay and/or do not rescue apoptosis of serum-starved human umbilical cord vein endothelial cells in a trypan blue exclusion test. In some embodiments, the sNAG nanofibers increase the metabolic rate of serum-starved human umbilical cord vein endothelial cells in a MTT assay and do not rescue apoptosis of serum-starved human umbilical cord vein endothelial cells in a trypan blue exclusion test.

In a specific embodiment, the sNAG nanofibers are biocompatible. Biocompatibility may be determined by a variety of techniques, including, but not limited to such procedures as the elution test, intramuscular implantation, or intracutaneous or systemic injection into animal subjects. Such tests are described in U.S. Patent No. 6,686,342 (see, e.g., Example 10). Some of the biocompatibility tests are also described in WO2011/130646 and WO2012/142581.

In certain embodiments, the sNAG nanofibers used in the methods described herein are non-reactive in a biocompatibility test or tests. For example, the sNAG nanofibers used in the methods described herein may be non-reactive when tested in an elution test, an intramuscular implantation test, an intracutaneous test, and/or a systemic test. In other embodiments, the sNAG nanofibers used in the methods described herein have Grade 0 or Grade 1 test score when tested in an elution test, an intramuscular implantation test, an intracutaneous test, or a systemic test. In yet another embodiment, the sNAG nanofibers used in the methods described herein are at most mildly reactive when tested in an elution test, an intramuscular implantation test, an intracutaneous test, and/or a systemic test. In certain embodiments, the compositions described herein do not cause an allergenic reaction or an irritation. In other embodiments, the compositions described herein cause at most a mild allergenic reaction or a mild irritation, e.g., at the site of application. The relevant tests and evaluation of test results are described in, e.g., U.S. Patent No. 6,686,342, WO2011/130646 and WO2012/142581.

In a specific embodiment, the sNAG nanofibers are non-reactive when tested in an intramuscular implantation test. In one aspect, an intramuscular implantation test is an intramuscular implantation test - ISO 4 week implantation, as described in Section 6.8.3, infra. In certain embodiments, the sNAG nanofibers display no biological reactivity as determined by an elution test (Elution Test Grade = 0). In some embodiments, the sNAG nanofibers have a test score equal to "0" and/or are at most a negligible irritant as determined by intracutaneous injection test. In some embodiments, the sNAG nanofibers elicit no intradermal reaction (i.e., Grade I reaction) in Kligman test and/or have a weak allergenic potential as determined by Kligman test. WO2011/130646 and WO2012/142581 show that sNAG nanofibers are non-reactive in an intramuscular implantation test, an intracutaneous injection test, and Kligman test.

In certain aspects, the sNAG nanofibers are immunoneutral (i.e., they do not elicit an immune response).

In some embodiments, the sNAG nanofibers are biodegradable. The sNAG nanofibers preferably degrade within about 1 day, 2 days, 3 days, 5 days, 7 days (1 week), 8 days, 10 days, 12 days, 14 days (2 weeks), 17 days, 21 days (3 weeks), 25 days, 28 days (4 weeks), 30 days, 1 month, 35 days, 40 days, 45 days, 50 days, 55 days, 60 days, 2 months, 65 days, 70 days, 75 days, 80 days, 85 days, 90 days, 3 months, 95 days, 100 days or 4 months after administration or implantation into a patient.

In certain embodiments, the sNAG nanofibers do not cause a detectable foreign body reaction. A foreign body reaction, which may occur during wound healing, includes accumulation of exudate at the site of injury, infiltration of inflammatory cells to debride the area, and the formation of granulation tissue. The persistent presence of a foreign body can inhibit full healing. Rather than the resorption and reconstruction that occurs in wound healing, the foreign body reaction is characterized by the formation of foreign body giant cells, encapsulation of the foreign object, and chronic inflammation. Encapsulation refers to the firm, generally avascular collagen shell deposited around a foreign body, effectively isolating it from the host tissues. In one embodiment, treatment of a site (e.g., a wound or a site of a bacterial infection in a wound) with the sNAG nanofibers does not elicit a detectable foreign body reaction in 1 day, 3 days, 5 days, 7 days, 10 days or 14 days after treatment. In one such embodiment, treatment of a site (e.g., a wound) with the sNAG nanofibers does not elicit a foreign body encapsulations in 1 day, 3 days, 5 days, 7 days, 10 days or 14 days after treatment.

In some embodiments, the sNAG nanofibers (i) comprise fibers, wherein majority of the fibers are between about 1 and 15 microns in length, and (ii) (a) increase the metabolic rate of serum-starved EC in a MTT assay and/or do not rescue apoptosis of serum-starved EC in a trypan blue exclusion test, and (b) are non-reactive when tested in an intramuscular implantation test. In certain embodiments, the sNAG nanofibers (i) comprise fibers, wherein majority of the fibers are between about 1 and 12 microns in length, and (ii) (a) increase the metabolic rate of serum-starved EC in a MTT assay and/or do not rescue apoptosis of serum-starved EC in a trypan blue exclusion test, and (b) are non-reactive when tested in an intramuscular implantation test. In some embodiments, the sNAG nanofibers (i) comprise fibers, wherein majority of the fibers are between (or in the range of) 1 to 10 microns, 2 to 10 microns, 4 to 10 microns, 5 to 10 microns, or 5 to 15 microns in length, and (ii) (a) increase the metabolic rate of serum-starved EC in a MTT assay and/or do not rescue apoptosis of serum-starved EC in a trypan blue exclusion test, and (b) are non-reactive when tested in an intramuscular implantation test. In some embodiments, the sNAG nanofibers (i) comprise fibers, wherein majority of the fibers are between about 4 and 10 microns in length, and (ii) (a) increase the metabolic rate of serum-starved EC in a MTT assay and/or do not rescue apoptosis of serum-starved EC in a trypan blue exclusion test, and (b) are non-reactive when tested in an intramuscular implantation test. In certain embodiments, the sNAG nanofibers (i) comprise fibers, wherein majority of the fibers are between about 4 and 7 microns in length, and (ii) (a) increase the metabolic rate of serum-starved EC in a MTT assay and/or do not rescue apoptosis of serum-starved EC in a trypan blue exclusion test, and (b) are non-reactive when tested in an intramuscular implantation test.

In certain embodiments, the sNAG nanofibers do not have a direct effect on the growth or survival of bacteria, such as *S. aureus,* as determined by one skilled in the art. In other embodiments, sNAG nanofibers do not have a direct effect on the growth or survival of bacteria, such as S. aureus, as determined by the methods set forth in WO2011/130646. In some embodiments, the sNAG nanofibers do not have a direct effect in vitro on bacterial growth or survival. In one embodiment, the sNAG nanofibers do not have a direct effect (e.g., in vitro) on growth or survival of gram-negative bacteria. In another embodiment, the sNAG nanofibers do not have a direct effect (e.g., in vitro) on growth or survival of gram-positive bacteria. In yet another embodiment, the sNAG nanofibers do not have a direct effect (e.g., in vitro) on growth or survival of either gram-positive or gram-negative bacteria.

In some embodiments, the sNAG nanofibers (i) comprise fibers, wherein majority of the fibers are between (or in the range of) about 1 and 15 microns, 1 and 12 microns, 1 and 10 microns, 4 and 10 microns, 4 and 15 microns, 5 and 10 microns, 5 and 15 microns, or 4 and 7 microns in length, (ii) do not have an effect on bacterial growth or survival of Staphylococcus aureus bacterial cultures in vitro, and (iii) are non-reactive when tested in a biocompatibility test (e.g., an intramuscular implantation test).

In certain embodiments, the sNAG nanofibers induce a certain pattern of gene expression (RNA or protein expression as determined by, e.g., RT-PCR, microarray or ELISA) in a cell, tissue or organ treated with or exposed to a sNAG nanofiber composition.

In certain embodiments, the sNAG nanofibers or a composition comprising the sNAG nanofibers reduces expression of collagen type I. In certain embodiments, the sNAG nanofibers or a composition comprising the sNAG nanofibers increases expression of collagen type III. In certain embodiments, the sNAG nanofibers or a composition comprising the sNAG nanofibers reduces total expression of collagen proteins.

In certain embodiments, the sNAG nanofibers or a composition comprising the sNAG nanofibers increases expression of elastin protein.

In certain embodiments, the sNAG nanofibers or a composition comprising the sNAG nanofibers reduces expression of one or more actin proteins. In certain embodiments, the sNAG nanofibers or a composition comprising the sNAG nanofibers reduces expression of one or more actin proteins in smooth muscle cells (e.g., alpha smooth muscle actin protein).

In some embodiments, the sNAG nanofibers or a composition comprising the sNAG nanofibers reduce expression of one or more of the above-listed proteins in the amount equal to or more than about 0.25 fold, 0.5 fold, 1 fold, 1.5 fold, 2 fold, 2.5 fold, 3 fold, 3.5 fold, 4 fold, 4.5 fold, 5 fold, 6 fold, 7 fold, 8 fold, 9 fold, 10 fold, 12 fold, 15 fold or 20 fold as compared to the level of expression of the one or more of the above-listed proteins in a cell, tissue or organ of a subject before treatment with the sNAG nanofibers (e.g., a known average level of expression of the one or more of the above-listed proteins). In some embodiments, the sNAG nanofibers or a composition comprising the sNAG nanofibers reduce expression of one or more of the above-listed proteins in the amount equal to or more than about 10%, 25%, 50%, 75% or 100%, 125%, 150%, 175%, 200%, 225%, 250%, 275%, 300%, 350%, 400%, 450%, 500%, 550%, 600%, 650%, 700%, 750%, 800%, 900% or 1000% the level of expression of the one or more of the above-listed proteins in a cell, tissue or organ of a subject before treatment with the sNAG nanofibers (e.g., a known average level of expression of the one or more of the above-listed proteins).

In some embodiments, the sNAG nanofibers or a composition comprising the sNAG nanofibers induce expression of one or more defensin proteins, one or more defensin-like proteins, and/or one or more Toll-like receptors.

In certain embodiments, the sNAG nanofibers or a composition comprising the sNAG nanofibers induces/increases expression of one or more α-defensins (e.g., DEFA1 (i.e., α-defensin 1), DEFA1B, DEFA3, DEFA4, DEFA5, DEFA6), one or more β-defensins (e.g., DEFB1 (i.e., β-defensin 1), DEFB2, DEFB4, DEFB103A, DEFB104A, DEFB105B, DEFB107B, DEFB108B, DEFB110, DEFB112, DEFB114, DEFB118, DEFB119, DEFB123, DEFB124, DEFB125, DEFB126, DEFB127, DEFB128, DEFB129, DEFB131, DEFB136), and/or one or more θ-defensins (e.g., DEFT1P). In some embodiments, the sNAG nanofibers or a composition comprising the sNAG nanofibers induce/increase expression of one or more of DEFA1, DEFA3, DEFA4, DEFA5, DEFB1, DEFB3, DEFB103A, DEFB104A, DEFB108B, DEFB112, DEFB114, DEFB118, DEFB119, DEFB123, DEFB124, DEFB125, DEFB126, DEFB128, DEFB129 and DEFB131. In certain embodiments, the sNAG nanofibers or a composition comprising the sNAG nanofibers induces/increases expression of one or more Toll receptors (e.g., TLR1, TLR2, TLR3, TLR4, TLR5, TLR6, TLR7, TLR8, TLR9, TLR10, TLR11, and/or TLR12). In other embodiments, the sNAG nanofibers or a composition comprising the sNAG nanofibers induces/increases expression of one or more of IL-1, CEACAM3, SPAG11, SIGIRR (IL1-like receptor), IRAK1, IRAK2, IRAK4, TBK1, TRAF6 and IKKi. In some embodiments, the sNAG nanofibers or a composition comprising the sNAG nanofibers induces/increases expression of one or more of IRAK2, SIGIRR, TLR1, TLR2, TLR4, TLR7, TLR8, TLR10 and TRAF6. In one embodiment, the sNAG nanofibers or a composition comprising the sNAG nanofibers induces/increases expression of at least one of the above-listed gene products.

In some embodiments, the sNAG nanofibers or a composition comprising the sNAG nanofibers induces/increases expression of one or more of the above-listed gene products in the amount equal to or more than about 0.25 fold, 0.5 fold, 1 fold, 1.5 fold, 2 fold, 2.5 fold, 3 fold, 3.5 fold, 4 fold, 4.5 fold, 5 fold, 6 fold, 7 fold, 8 fold, 9 fold, 10 fold, 12 fold, 15 fold or 20 fold as compared to the level of expression of the one or more of the above-listed gene products in a cell, tissue or organ of a subject before treatment with the sNAG nanofibers (e.g., a known average level of expression of the one or more of the above-listed gene products). In some embodiments, the sNAG nanofibers or a composition comprising the sNAG nanofibers induces/increases expression of one or more of the above-listed gene products in the amount equal to or more than about 10%, 25%, 50%, 75%, 100%, 125%, 150%, 175%, 200%, 225%, 250%, 275%, 300%, 350%, 400%, 450%, 500%, 550%, 600%, 650%, 700%, 750%, 800%, 900% or 1000% the level of expression of the one or more of the above-listed gene products in a cell, tissue or organ of a subject before treatment with the sNAG nanofibers (e.g., a known average level of expression of the one or more of the above-listed gene products).

In some embodiments, the sNAG nanofibers but not long poly-N-acetylglucosamine, chitin and/or chitosan induce/increase expression of the one or more gene products listed above, as determined by a method known to one skilled in the art, or described herein. In some of these embodiments, long poly-N-acetylglucosamine, chitin and/or chitosan do not induce/increase expression of the one or more gene products listed above or induce lower level (e.g., more than 1.25 fold, 1.5 fold, 2 fold, 2.5 fold, 3 fold, 3.5 fold, 4 fold, 4.5 fold, 5 fold, 6 fold, 7 fold, 8 fold, 9 fold, or 10 fold lower) of expression of the one or more gene products listed above as compared to the level of expression of the one or more gene products listed above induced by the sNAG nanofibers, as determined by a method known to one skilled in the art, or described herein.

In certain embodiments, the sNAG nanofibers but not long poly-N-acetylglucosamine, chitin and/or chitosan reduce/decrease expression of the one or more gene products listed above, as determined by a method known to one skilled in the art, or described herein. In some of these embodiments, long poly-N-acetylglucosamine, chitin and/or chitosan do not reduce/decrease expression of the one or more gene products listed above or induce a lower level (e.g., more than 1.25 fold, 1.5 fold, 2 fold, 2.5 fold, 3 fold, 3.5 fold, 4 fold, 4.5 fold, 5 fold, 6 fold, 7 fold, 8 fold, 9 fold, or 10 fold lower) of expression of the one or more gene products listed above as compared to the level of expression of the one or more gene products listed above reduced by the sNAG nanofibers, as determined by a method known to one skilled in the art, or described herein.

In certain embodiments, the sNAG nanofibers or a composition comprising the sNAG nanofibers induce a gene expression profile that is consistent with, similar to, about the same as, or equivalent to one or more gene expression profiles demonstrated in WO 2011/130646 and WO 2012/142581 (see Tables I, II, III, V, VIII and IX, Sections 6.2-6.5).

In certain embodiments, the sNAG nanofibers or a composition comprising the sNAG nanofibers induce a gene expression profile that differs from the profile induced by long poly-N-acetylglucosamine polymers or fibers. In specific embodiments, a gene expression profile induced by the sNAG nanofibers is consistent with, similar to, about the same as, or equivalent to that shown in WO 2011/130646 and WO 2012/142581 (see Tables I, II, III, V, VIII and IX, Sections 6.2-6.5), whereas gene expression profile induced by long poly-N-acetylglucosamine polymers or fibers is consistent with, similar to, about the same with, or equivalent to that shown in Table VIII and/or IX, Section 6.5 of WO 2011/130646 and WO 2012/142581. In other embodiments, the sNAG nanofibers or a composition comprising the sNAG nanofibers induce a gene expression profile that differs from the gene expression profile induced by chitin or chitosan.

The sNAG nanofibers may be obtained by irradiating poly-N-acetylglucosamine and/or a derivative thereof. See Section 5.1.1, infra, regarding poly-N-acetylglucosamine and derivatives thereof and Section 5.2, infra, regarding methods for producing the sNAG nanofibers using irradiation. Irradiation may be used to reduce the length of poly-N-acetylglucosamine fibers and/or poly-N-acetylglucosamine derivative fibers to form shortened poly-β-1→4-N-acetylglucosamine fibers and/or shortened poly-N-acetylglucosamine derivative fibers, i.e. sNAG nanofibers. Specifically, irradiation may be used to reduce the length and molecular weight of poly-N-acetylglucosamine or a derivative thereof without disturbing its microstructure. The infrared spectrum (IR) of sNAG nanofibers is similar to, about the same as, or equivalent to that of the non-irradiated poly-β-1→4-N-acetylglucosamine or a derivative thereof.

In one embodiment, the sNAG nanofibers are not derived from chitin or chitosan. Whereas in another embodiment, the compositions described herein may be derived from chitin or chitosan, or the sNAG nanofibers may be derived from chitin or chitosan.

### 5.1.1 Poly-N-Acetylglucosamine and Derivatives Thereof

U.S. Patent Nos. 5,622,834; 5,623,064; 5,624,679; 5,686,115; 5,858,350; 6,599,720; 6,686,342; 7,115,588 and U.S. Patent Pub. 2009/0117175 describe the poly-N-acetylglucosamine and derivatives thereof, and methods of producing the same. In some embodiments, the poly-N-acetylglucosamine has a β-1→4 configuration. In other embodiments, the poly-N-acetylglucosamine has a α-1→4 configuration. The poly-N-acetylglucosamine and derivatives thereof may be in the form of a polymer or in the form of a fiber.

Poly-N-acetylglucosamine can, for example, be produced by, and may be purified from, microalgae, preferably diatoms. The diatoms which may be used as starting sources for the production of the poly-N-acetylglucosamine include, but are not limited to members of the Coscinodiscus genus, the Cyclotella genus, and the Thalassiosira genus. Poly-N-acetylglucosamine may be obtained from diatom cultures via a number of different methods, including the mechanical force method and chemical/biological method known in the art (see, e.g., U.S. Patent Nos. 5,622,834; 5,623,064; 5,624,679; 5,686,115; 5,858,350; 6,599,720; 6,686,342; and 7,115,588). In certain embodiments, the poly-N-acetylglucosamine is not derived from one or more of the following: a shell fish, a crustacean, an insect, a fungi or yeasts.

In one embodiment, poly-β-1→4-N-acetylglucosamine is derived from a process comprising a) treating a microalgae comprising a cell body and a poly-β-1→4-N-acetylglucosamine polymer fiber with a biological agent (such as hydrofluoric) capable of separating the N-acetylglucosamine polymer fiber from the cell body for a sufficient time so that the poly-β-1→4-N-acetylglucosamine polymer fiber is released from the cell body; b) segregating the poly-β-1→4-N-acetylglucosamine polymer fiber from the cell body; and c) removing contaminants from the segregated poly-β-1→4-N-acetylglucosamine polymer fiber, so that the poly-β-1→4-N-acetylglucosamine polymer is isolated and purified.

In other embodiments, the poly-β-1→4-N-acetylglucosamine may be derived from one or more of the following: a shell fish, a crustacean, an insect, a fungi or yeasts. In certain embodiments, the compositions described herein do not comprise chitin or chitosan.

One or more of the monosaccharide units of the poly-N-acetylglucosamine may be deacetylated. In certain embodiments, 1% to 5%, 5% to 10%, 5% to 15%, 20% to 30% or 25% to 30% of the poly-N-acetylglucosamine is deacetylated. In some embodiments, 1%, 5%, 10%,15%, 20%, 25%, or 30% of the poly-N-acetylglucosamine is deacetylated. In other embodiments, less than 30%, 25%, 20%, 15%, 10%, 5%, 4%, 3%, 2% or 1% of the poly-N-acetylglucosamine is deacetylated. In some instances, equal to or more than 1%, 5%, 10%, 15%, 20%, 25%, 30%, 35%, 40%, 45%, 50%, 55%, 60%, 65%, 70%, 75%, 80%, 85%, 90%, 95% or 99%, or all (100%), of the poly-N-acetylglucosamine is deacetylated. In other instances, less than 1%, 5%, 10%, 15%, 20%, 25%, 30%, 35%, 40%, 45%, 50%, 55%, 60%, 65%, 70%, 75%, 80%, 85%, 90%, 95%, 99%, or 100% of the poly-N-acetylglucosamine is deacetylated.

In certain embodiments, a poly-N-acetylglucosamine composition comprises 70% to 80%, 75% to 80%, 75% to 85%, 85% to 95%, 90% to 95%, 90% to 99% or 95% to 100% of acetylated glucosamine (i.e., N-acetylglucosamine) monosaccharides. In some embodiments, a poly-N-acetylglucosamine composition comprises 70%, 75%, 80%, 85%, 90%, 95%, 97%, 98%, 99% or 100% of acetylated glucosamine (i.e., N-acetylglucosamine) monosaccharides. In other embodiments, a poly-N-acetylglucosamine composition comprises more than 70%, 75%, 80%, 85%, 90%, 95%, 97%, 98%, 99%, 99.5% or 99.9% of the acetylated glucosamine. In some instances, a poly-N-acetylglucosamine composition comprises equal to or more than 1%, 5%, 10%, 15%, 20%, 25%, 30%, 35%, 40%, 45%, 50%, 55%, 60%, 65%, 70%, 75%, 80%, 85%, 90%, 95%, 97%, 98%, or 99%, or all (100%), of the acetylated glucosamine. In other instances, a poly-N-acetylglucosamine composition comprises less than 1%, 5%, 10%, 15%, 20%, 25%, 30%, 35%, 40%, 45%, 50%, 55%, 60%, 65%, 70%, 75%, 80%, 85%, 90%, 95%, 99%, or 100% of the acetylated glucosamine.

In some instances, a poly-N-acetylglucosamine composition comprises at least one glucosamine monosaccharide, and may further comprise at least 10%, 20%, 30%, 40%, 50%, 60%, 70%, 80%, 90%, 95% or 99% of N-acetylglucosamine monosaccharides. In some instances, a poly-N-acetylglucosamine composition comprises at least one N-acetylglucosamine monosaccharide, and may further comprise at least 10%, 20%, 30%, 40%, 50%, 60%, 70%, 80%, 90%, 95% or 99% of glucosamine monosaccharides.

Derivatives of poly-N-acetylglucosamine may also be used in a composition described herein. Derivatives of poly-N-acetylglucosamine and methods of making such derivatives are described in U.S. Patent No. 5,623,064 (see, e.g., Section 5.4). Derivatives of poly-N-acetylglucosamine may include, but are not limited to, partially or completely deacetylated poly-N-acetylglucosamine, or its deacetylated derivatives. Further, poly-N-acetylglucosamine may be derivatized by being sulfated, phosphorylated and/or nitrated. Poly-N-acetylglucosamine derivatives include, e.g., sulfated poly-N-acetylglucosamine derivatives, phosphorylated poly-N-acetylglucosamine derivatives, or nitrated poly-N-acetylglucosamine derivatives. Additionally, one or more of the monosaccharide units of the poly-N-acetylglucosamine may contain one or more sulfonyl groups one or more O-acyl groups. In addition, one or more of the monosaccharides of the deacetylated poly-N-acetylglucosamine may contain an N-acyl group. One or more of the monosaccharides of the poly-N-acetylglucosamine or of its deacetylated derivative, may contain an O-alkyl group. One or more of the monosaccharide units of the poly -N-acetylglucosamine may be an alkali derivative. One or more of the monosaccharide units of the deacetylated derivative of poly-N-acetylglucosamine may contain an N-alkyl group. One or more of the monosaccharide units of the deacetylated derivative of poly-N-acetylglucosamine may contain at least one deoxyhalogen derivative. One or more of the monosaccharide units of the deacetylated derivative of poly- N-acetylglucosamine may form a salt. One or more of the monosaccharide units of the deacetylated derivative of poly-N-acetylglucosamine may form a metal chelate. In a specific embodiment, the metal is zinc. One or more of the monosaccharide units of the deacetylated derivative of poly-N-acetylglucosamine may contain an N-alkylidene or an N-arylidene group. In one embodiment, the derivative is an acetate derivative. In another embodiment, the derivative is not an acetate derivative. In one embodiment the poly-N-acetylglucosamine or deacetylated poly-N-acetylglucosamine is derivatized with lactic acid. Wherein, in another embodiment, the derivative is not derivatized with lactic acid.

### 5.2 Methods of Producing sNAG Nanofibers

The poly-N-acetylglucosamine polymers or fibers, and any derivatives of poly-N-acetylglucosamine polymers or fibers described above, can be irradiated as dry polymers or fibers or polymer or fiber membranes. Alternatively, poly-N-acetylglucosamine polymers or fibers, and any derivatives of poly-N-acetylglucosamine polymers or fibers described above, can be irradiated when wet. The methods of making sNAG nanofibers by irradiation and the sNAG nanofibers so produced have been described in U.S. Patent Pub. No. 2009/0117175.

In certain embodiments, the poly-N-acetylglucosamine polymers or fibers are formulated into a suspension/slurry or wet cake for irradiation. Irradiation can be performed prior to, concurrently with or following the formulation of the polymers or fibers into its final formulation, such as a dressing. Generally, the polymer or fiber content of suspensions/slurries and wet cakes can vary, for example from about 0.5 mg to about 50 mg of polymer or fiber per 1 ml of distilled water are used for slurries and from about 50 mg to about 1000 mg of polymer or fiber per 1 ml of distilled water are use for wet cake formulations. The polymer or fiber may first be lyophilized, frozen in liquid nitrogen, and pulverized, to make it more susceptible to forming a suspension/slurry or wet cake. Also, the suspensions/slurries can be filtered to remove water such that a wet cake is formed. In certain aspects, the polymer or fiber is irradiated as a suspension comprising about 0.5 mg, 1 mg, 2 mg, 3 mg, 4 mg, 5 mg, 6 mg, 7 mg, 8 mg, 9 mg, 10 mg, 12 mg, 15 mg, 18 mg, 20 mg, 25 mg or 50 mg of polymer or fiber per ml of distilled water, or any range in between the foregoing embodiments (*e.g*., 1-10 mg/ml, 5-15 mg/ml, 2-8 mg/ml, 20-50 mg/ml, *etc*.). In other aspects, the polymer or fiber is irradiated as a wet cake, comprising about 50-1,000 mg polymer or fiber per 1 ml of distilled water. In specific embodiments, the wet cake comprises about 50, 100, 200, 300, 400, 500, 600, 700, 800, 900 or 1000 mg of polymer or fiber per 1 ml distilled water, or any range in between (*e.g.,* 100-500 mg/ml, 300-600 mg/ml, 50-1000 mg/ml, *etc*.).

The irradiation is preferably in the form of gamma radiation, e-beam radiation, or x-rays. Two sources of irradiation are preferred: radioactive nuclides and electricity. In specific embodiment, the radioactive nuclides are cobalt-60 and cesium-137. Both of these nuclides emit gamma rays, which are photons containing no mass. The gamma rays have energies from 0.66 to 1.3 MeV. Using electricity, electrons are generated and accelerated to energies up to 10 MeV or higher. When irradiating polymers or fibers to reduce their size, a consideration to take into account is that the depth of penetration of materials with densities similar to water by 10 MeV electrons is limited to about 3.7 cm with one-sided exposure or about 8.6 cm with two-sided exposure. Depth of penetration decreases at lower electron energies. Electron energy can be converted to x-rays by placing a metal (usually tungsten or tantalum) target in the electron beam path. Conversion to x-rays is limited to electrons with energies up to 5 MeV. X-rays are photons with no mass and can penetrate polymers or fibers similar to gamma rays. There is only about 8% efficiency in the conversion of electron energy to x-ray energy. High powered electron beam machines are needed in x-ray production facilities to account for the low conversion efficiency.

In a specific embodiment, the irradiation is gamma irradiation.

The absorbed dose of radiation is the energy absorbed per unit weight of product, measured in gray (gy) or kilogray (kgy). For dried polymers or fibers, the preferred absorbed dose is about 500-2,000 kgy of radiation, most preferably about 750-1,250 kgy or about 900-1,100 kgy of radiation. For wet polymers or fibers, the preferred absorbed dose is about 100-500 kgy of radiation, most preferably about 150-250 kgy or about 200-250 kgy of radiation.

The dose of radiation can be described in terms of its effect on the length of the polymers or fibers. In specific embodiments, the dose of radiation used preferably reduces the length of the polymer or fiber by anywhere from about 10% to 90% of the starting length of the polymer or fiber, respectively. In specific embodiments, the average length is reduced by about 10%, by about 20%, by about 30%, by about 40%, by about 50%, by about 60%, by about 70%, by about 80%, or by about 90%, or any range in between (e.g., 20-40%, 30-70%, and so on and so forth). Alternatively, the dose of radiation used preferably reduces the length of the polymer or fiber to anywhere from 1 to 100 microns. In specific embodiments, and depending on the starting fiber length, the average length of the polymer or fiber is reduced to less than about 15 microns, less than about 14 microns, less than about 13 microns, less than about 12 microns, less than about 11 microns, less than about 10 microns, less than about 8 microns, less than about 7 microns, less than about 5 microns, less than about 4 microns, less than about 3 microns, less than 2 microns, or less than 1 microns. In certain embodiments, the length of the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the polymers or fibers is reduced to no greater than about 20 microns, no greater than about 15 microns, no greater than about 12 microns, no greater than about 10 microns, no greater than about 8 microns, no greater than about 7 microns, or no greater than about 5 microns. In certain embodiments, irradiation of the polymers or fibers reduces the length of the majority (and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the fibers to anywhere between about 1 to 20 microns, between about 1 to 15 microns, between about 2 to 15 microns, between about 1 to 12 microns, between about 2 to 12 microns, between about 1 to 10 microns, between about 2 to 10 microns, between about 1 to 8 microns, between about 2 to 8 microns, between about 1 to 7 microns, between about 2 to 7 microns, between about 3 to 8 microns, between about 4 to 10 microns, between about 4 to 7 microns, between about 5 to 10 microns, between about 1 to 5 microns, between about 2 to 5 microns, between about 3 to 5 microns, between about 4 to 10 microns, or any ranges between the foregoing lengths, which are also encompassed.

The dose of radiation can also be described in terms of its effect on the molecular weight of the polymer or fiber. In specific embodiments, the dose of radiation used preferably reduces the molecular weight of the polymer or fiber by anywhere from about 10% to 90% of the starting weight of the polymer or fiber. In specific embodiments, the average molecular weight is reduced by about 10%, by about 20%, by about 30%, by about 40%, by about 50%, by about 60%, by about 70%, by about 80%, or by about 90%, or any range in between (e.g., 20-40%, 30-70%, and so on and so forth). Alternatively, the dose of radiation used preferably reduces the molecular weight of the polymer or fiber to anywhere from 1,000 to 1,000,000 daltons. In specific embodiments, and depending on the starting molecular weight, the average molecular weight of the polymer or fiber is reduced to less than 1,000,000 daltons, less than 750,000 daltons, less than 500,000 daltons, less than 300,000 daltons, less than 200,000 daltons, less than 100,000 daltons, less than 90, 000 daltons, less than 80,000 daltons, less than 70,000 daltons, less than 60,000 daltons, less than 50,000 daltons, less than 25,000 daltons, less than 10,000 daltons, or less than 5,000 daltons. In certain embodiments, the average molecular weight is reduced to no less than 500 daltons, no less than 1,000 daltons, no less than 2,000 daltons, no less 3,500 daltons, no less than 5,000 daltons, no less than 7,500 daltons, no less than 10,000 daltons, no less than 25,000 daltons, no less than 50,000 daltons, no less than 60, 000 daltons or no less than 100,000 daltons. Any ranges between the foregoing average molecular weights are also encompassed; for example, in certain embodiments, irradiation of the polymer or fiber reduces the average molecular weight to anywhere between 10,000 to 100,000 daltons, between 1,000 and 25,000 daltons, between 50,000 and 500,000 daltons, between 25,000 and 100,000 daltons, between 30,000 and 90,000 daltons, between about 40,000 and 80,000 daltons, between about 25,000 and 75,000 daltons, between about 50,000 and 70,000 daltons, or between about 55,000 and 65,000 daltons and so on and so forth. In certain embodiments, irradiation of the polymers or fibers reduces the molecular weight of the majority and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the fibers to anywhere between about 20,000 and 100,000 daltons, about 25,000 and 75,000 daltons, about 30,000 and 90,000 daltons, about 40,000 and 80,000 daltons, about 50,000 and 70,000 daltons, or about 55,000 and 65,000 daltons. In certain embodiments, irradiation of the polymers or fibers reduces the molecular weight of the majority and in certain embodiments, at least 60%, 70%, 80%, 90%, 95%, 98%, 99%, 99.5%, 99.8%, 99.9%, or 100%, or between 55% to 65%, 55% to 75%, 65% to 75%, 75% to 85%, 75% to 90%, 80% to 95%, 90% to 95%, or 95% to 99%) of the fibers to about 60,000 daltons.

Following irradiation, slurries can be filtered and dried, and wet cakes can be dried, to form compositions (e.g., dressings and other compositions described herein) that are useful in the practice of the invention.

### 5.3 Compositions Comprising sNAG Nanofibers

The sNAG nanofibers may be formulated in a variety of compositions by any route of administration (e.g., topical, oral, intramuscular, intravenous, rectal, subcutaneous, systemic or local) as described herein.

A composition comprising the sNAG nanofibers may be formulated as a cream, a membrane, a film, a liquid solution, a suspension (e.g., a thick suspension), a powder, a paste, an ointment, a suppository, a gelatinious composition, an aerosol, a gel, or a spray. In one embodiment, a composition comprising the sNAG nanofibers is formulated as an ultra-thin membrane. In some embodiments, a composition comprising the sNAG nanofibers is formulated as a dressing, a mat or a bandage. In some embodiments, a composition comprising the sNAG nanofibers is formulated as a mask or a peel. In particular embodiments, compositions comprising sNAG nanofibers are not solid or barrier-forming. In another embodiment, compositions comprising sNAG nanofibers are solid or barrier-forming. Solid formulations suitable for solution in, or suspension in, liquids prior to administration are also contemplated. It is also possible that such compositions are incorporated in or coated on implantable devices, such as orthopedic implants (for hip, knee, shoulder; pins, screws, etc.), cardiovascular implants (stents, catheters, etc.) and the like where the antibacterial activity would be of benefit.

In certain instances, a composition comprising sNAG nanofibers is formulated for systemic administration (e.g., parenteral administration). In some instances, a composition comprising sNAG nanofibers is formulated for oral, intramuscular, intravenous, rectal, or subcutaneous administration. In other embodiments, a composition comprising sNAG nanofibers is formulated for local (not systemic) administration. In some embodiments, a composition comprising sNAG nanofibers is formulated for intradermal, subcutaneous or intramuscular administration. In one embodiment, a composition comprising sNAG nanofibers is formulated for intradermal administration (e.g., as a suspension for intradermal injection). In one embodiment, a composition comprising sNAG nanofibers is formulated for subcutaneous administration (e.g., as a suspension for subcutaneous injection). In one embodiment, a composition comprising sNAG nanofibers is formulated for intramuscular administration (e.g., as a suspension for intramuscular injection). In specific embodiments, a composition comprising sNAG nanofibers is formulated for topical administration. In particular embodiments, a composition comprising sNAG nanofibers is formulated for topical administration as a gel, spray, cream, suspension, ointment or paste. In particular embodiments, a composition comprising sNAG nanofibers is formulated for topical administration as a mask or a peel for cosmetic applications.

A composition comprising the sNAG nanofibers may include one or more of pharmaceutically acceptable excipients. Suitable excipients may include water, saline, salt solution, dextrose, glycerol, ethanol and the like, or combinations thereof. Suitable excipients also include starch, glucose, lactose, sucrose, gelatin, malt, rice, flour, chalk, silica gel, sodium stearate, glycerol monostearate, oil (including those of petroleum, animal, vegetable or synthetic origin, such as peanut oil, soybean oil, mineral oil, sesame oil and the like), talc, sodium chloride, dried skim milk, propylene, glycol and the like. In addition, a composition comprising the sNAG nanofibers may include one or more of wetting agents, emulsifying agents, pH buffering agents, and other agents. The sNAG nanofiber compositions may also be incorporated in a physiologically acceptable carrier, for example in a physiologically acceptable carrier suitable for topical application. The term "pharmaceutically acceptable" means approved by a regulatory agency of the Federal or a state government or listed in the U.S. Pharmacopeia or other generally recognized pharmacopeia for use in animals, and more particularly in humans. Examples of suitable pharmaceutical carriers are described in "Remington's Pharmaceutical Sciences" by E.W. Martin.

The final amount of the sNAG nanofibers in a composition may vary. For example, the amount of the sNAG nanofibers in a composition (e.g., prepared for administration to a patient) may be greater than or equal to about 50%, about 60%, about 70%, about 75%, about 80%, about 85%, about 90%, about 95%, about 98%, or about 99% weight by volume. In one embodiment, the amount of the sNAG nanofibers in a composition is about 95%, about 98%, about 99, or about 100%. Also, the amount of the sNAG nanofibers in a composition (e.g., prepared for administration to a patient) may be about 50%-100%, about 60%-100%, about 70%-100%, about 75%-100%, about 80%-100%, about 90%-100%, about 95%-100%, about 70%-95%, about 75%-95%, about 80%-95%, about 90%-95%, about 70%-90%, about 75%-90%, or about 80%-90% weight/volume. A composition may comprise more than 30%, 40%, 50%, 60%, 70%, 75%, 80%, 90%, 95% or 99% solution of the sNAG nanofibers.

A sNAG nanofiber composition may be formulated to contain from 0.1 mg/ml to 50 mg/ml of the sNAG nanofibers. In some embodiments, a sNAG nanofiber composition may be formulated to contain from 0.5 mg/ml to 30 mg/ml, 5 mg/ml to 30 mg/ml, 5 mg/ml to 25 mg/ml, 7.5 mg/ml to 20 mg/ml, or 10 mg/ml to 20 mg/ml of the sNAG nanofibers. In specific embodiments, a sNAG nanofiber composition may be formulated to contain about 1 mg/ml, 5 mg/ml, 10 mg/ml, 15 mg/ml, 20 mg/ml, 25 mg/ml or 30 mg/ml of the sNAG nanofibers. A sNAG nanofiber composition may be formulated for administration by local injection (e.g., intradermal, intracutaneous or intramuscular injection, for example, in the form of a suspension) to contain from 5 mg/ml to 30 mg/ml of the sNAG nanofibers (e.g., 5 mg/ml, 6 mg/ml, 7 mg/ml, 7.5 mg/ml, 8 mg/ml, 9 mg/ml, 10 mg/ml, 11 mg/ml, 12 mg/ml, 12.5 mg/ml, 13, mg/ml, 14 mg/ml, 15 mg/ml, 16 mg/ml, 17 mg/ml, 17.5 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, or 30 mg/ml). In some embodiments, a sNAG nanofiber composition may be formulated for administration by local injection (e.g., intradermal, intracutaneous or intramuscular injection, for example, in the form of a suspension) to contain 7.5 mg/ml to 25 mg/ml of the sNAG nanofibers. In some embodiments, a sNAG nanofiber composition may be formulated for administration by local injection (e.g., intradermal, intracutaneous or intramuscular injection, for example, in the form of a suspension) to contain 10 mg/ml to 20 mg/ml of the sNAG nanofibers. In one embodiment, a sNAG nanofiber composition may be formulated for administration by local injection (e.g., intradermal, intracutaneous or intramuscular injection, for example, in the form of a suspension) to contain about 12.5 mg/ml to about 17.5 mg/ml, or about 15 mg/ml of the sNAG nanofibers. A sNAG nanofiber composition may be formulated for topical administration (e.g., administration to the skin, to a mucosal surface or to a wound, for example in the form of a suspension or a spray) to contain from 0.1 mg/ml to 50 mg/ml of the sNAG nanofibers (e.g., 0.1 mg/ml, 0.5 mg/ml, 1 mg/ml, 2 mg/ml, 3 mg/ml, 4 mg/ml, 5 mg/ml, 6 mg/ml, 7 mg/ml, 7.5 mg/ml, 8 mg/ml, 9 mg/ml, 10 mg/ml, 11 mg/ml, 12 mg/ml, 12.5 mg/ml, 13, mg/ml, 14 mg/ml, 15 mg/ml, 16 mg/ml, 17 mg/ml, 17.5 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, or 50 mg/ml). In some embodiments, a sNAG nanofiber composition may be formulated for topical administration to contain 0.5 mg/ml to 50 mg/ml, 0.5 mg/ml to 30 mg/ml, 1 mg/ml to 50 mg/ml, 1 mg/ml to 40 mg/ml, 1 mg/ml to 30 mg/ml, 5 mg/ml to 30 mgl, 5 mg/ml to 20 mg/ml of the sNAG nanofibers. In some embodiments, a sNAG nanofiber composition may be formulated for topical administration to contain 1 mg/ml to 30 mg/ml of the sNAG nanofibers. In one embodiment, a sNAG nanofiber composition may be formulated for topical administration to contain about 5 mg/ml to about 25 mg/ml, about 10 mg/ml to about 20 mg/ml, or about 15 mg/ml of the sNAG nanofibers. A sNAG nanofiber composition may be formulated for topical administration (e.g., administration to the skin, to a mucosal surface or to a wound, for example, in the form of a gel, a cream, or a paste) to contain from 0.1 mg/cm² to 10 mg/cm² of the sNAG nanofibers (e.g., 0.1 mg/cm², 0.25 mg/cm², 0.5 mg/cm², 0.75 mg/cm², 1 mg/cm², 2 mg/cm², 2.5 mg/cm², 3 mg/cm², 4 mg/cm², 5 mg/cm², 6 mg/cm², 7 mg/cm², 7.5 mg/cm², 8 mg/cm², 9 mg/cm², or 10 mg/cm²). In some embodiments, a sNAG nanofiber composition may be formulated for topical administration to contain 0.5 mg/cm² to 10 mg/cm², 0.75 mg/cm² to 7.5 mg/cm², 1 mg/cm² to 7.5 mg/cm², or 1 mg/cm² to 5 mg/cm² of the sNAG nanofibers. In particular embodiments, a sNAG nanofiber composition may be formulated for topical administration to contain about 1 mg/cm² to about 5 mg/cm², or about 1 mg/cm², about 2.5 mg/cm², or about 5 mg/cm² of the sNAG nanofibers.

A sNAG nanofiber composition may be formulated into a wound dressing. In certain instances, a sNAG nanofiber composition is formulated as a wound dressing in the form of a barrier, a membrane, or a film. Alternatively, a sNAG nanofiber composition may be added to dressing backings, such as barriers, membranes, or films. A barrier, membrane, or film can be supplied in a variety of standard sizes, which can be further cut and sized to the area being treated. The backing can be a conventional dressing material, such as a bandage or gauze to which a polymer or fiber is added or coated on, prior to application to the patient. Alternatively, the sNAG nanofibers can be formulated as a barrier, membrane, or film made out of strings, microbeads, microspheres, or microfibrils, or the composition can be formulated as a barrier-forming mat. In certain instances, at least 75%, at least 85%, at least 90%, or at least 95% of a dressing is composed of the sNAG nanofibers. In certain instances, a dressing does not contain a conventional dressing material such as a gauze or bandage. In such instances, the sNAG nanofiber itself is formulated as a wound dressing.

In a specific embodiment, the sNAG nanofiber composition is not formulated into a wound dressing.

A sNAG nanofiber composition may be formulated into a cream, a membrane, a film, a liquid solution, a suspension (e.g., a thick suspension), a powder, a paste, an ointment, a suppository, a gelatinious composition, an aerosol, a gel, or a spray. In certain embodiments, at least 75%, at least 85%, at least 90%, or at least 95% of the formulation is composed of the sNAG nanofibers.

A composition comprising the sNAG nanofibers may further comprise any suitable natural or synthetic polymers or fibers. Examples of suitable polymers or fibers include cellulose polymers, xanthan, polyaramides, polyamides, polyimides, polyamide/imides, polyamidehydrazides, polyhydrazides, polyimidazoles, polybenzoxazoles, polyester/amide, polyester/imide, polycarbonate/amides, polycarbonate/imides, polysulfone/amides, polysulfone imides, and the like, copolymers and blends thereof. Other suitable classes of polymers or fibers include polyvinyledene fluorides and polyacrylonitriles. Examples of these polymers or fibers include those described in U.S. Patent Nos. RE 30,351; 4,705,540, 4,717,393; 4,717,394; 4,912,197; 4,838,900; 4,935,490; 4,851,505; 4,880,442; 4,863,496; 4,961,539; and European Patent Application 0 219 878. The polymers or fibers can include at least one of either of cellulose polymers, polyamides, polyaramides, polyamide/imides or polyimides. In certain embodiments, the polymers or fibers include polyaramides, polyester, urethan and polytetrafluoroethylene. In one embodiment, the compositions described herein comprise more than one type of polymer (*e.g.,* the sNAG nanofiber and cellulose).

In certain aspects, the sNAG nanofiber is the only active ingredient in a composition. In one embodiment, a composition comprising the sNAG nanofibers does not comprise another anti-bacterial agent, for example, does not comprise an antibiotic. In one embodiment, a composition comprising the sNAG nanofibers does not comprise zinc.

In other embodiments, a composition comprises one or more additional active ingredients, e.g., an anti-viral agent, an anti-fungal agent, an anti-yeast agent, a chemotherapeutic agent or any other agent. In some embodiments, the additional active ingredient is one or more of an anti-viral agent, an anti-fungal agent, an anti-yeast agent, a defensin peptide, a defensin-like peptide, or a Toll-receptor-like peptide), or a growth factor. In specific embodiments, the additional active ingredient is a growth factor such as one or more of PDGF-AA, PDGF-AB, PDGF-BB, PDGF-CC, PDGF-DD, FGF-1, FGF-2, FGF-5, FGF-7, FGF-10, EGF, TGF-α, (HB-EGF), amphiregulin, epiregulin, betacellulin, neuregulins, epigen, VEGF-A, VEGF-B, VEGF-C, VEGF-D, VEGF-E, placenta growth factor (PLGF), angiopoietin-1, angiopoietin-2, IGF-I, IGF-II, hepatocyte growth factor (HGF), and macrophage-stimulating protein (MSP). In other embodiments, the additional active ingredient is an agent that boosts the immune system, a pain relief agent, or a fever relief agent. Any one or more additional active ingredients that are known in the art can be used in combination with the sNAG nanofibers. For example, any of the additional active ingredients described in WO 2011/130646 and WO 2012/142581 can be used in combination with the sNAG nanofibers.

In certain embodiments, the additional active ingredient is an anti-viral agent. Any anti-viral agents well-known to one of skill in the art (e.g., described in WO 2011/130646 or WO 2012/142581) may be used in a sNAG nanofiber composition.

In some embodiments, the additional active ingredient is an anti-inflammatory agent. Non-limiting examples of anti-inflammatory agents include non-steroidal anti-inflammatory drugs (NSAIDs) (e.g., celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOXX™), naproxen (ALEVE™, NAPROSYN™), ketoprofen (ACTRON™) and nabumetone (RELAFEN™)), steroidal anti-inflammatory drugs (*e.g.*, glucocorticoids, dexamethasone (DECADRON™), corticosteroids (*e.g*., methylprednisolone (MEDROL™)), cortisone, hydrocortisone, prednisone (PREDNISONE™ and DELTASONE™), and prednisolone (PRELONE™ and PEDIAPRED™)), anticholinergics (*e.g*., atropine sulfate, atropine methylnitrate, and ipratropium bromide (ATROVENT™)), beta2-agonists (*e.g*., abuterol (VENTOLIN™ and PROVENTIL™), bitolterol (TORNALATE™), levalbuterol (XOPONEX™), metaproterenol (ALUPENT™), pirbuterol (MAXAIR™), terbutlaine (BRETHAIRE™ and BRETHINE™), albuterol (PROVENTIL™, REPETABS™, and VOLMAX™), formoterol (FORADIL AEROLIZER™), and salmeterol (SEREVENT™ and SEREVENT DISKUS™)), and methylxanthines (*e.g*., theophylline (UNIPHYL™, THEO-DUR™, SLO-BID™, AND TEHO-42™)).

A sNAG nanofiber composition may contain an additional tissue filler agent such as, for example, hyaluronic acid, collagen, calcium hydroxylapatite, fat (*e.g.*, human fat, in particular, autologous fat), polylactic acid, cadaveric dermis (*e.g.*, human), polymethylmethacrylate, and/or polyalkylimide. In other aspects, a sNAG nanofiber composition does not contain an additional tissue filler agent. A sNAG nanofiber composition may contain botulinum toxin. In other aspects, a sNAG nanofiber composition does not contain botulinum toxin.

A sNAG nanofiber composition may contain collagen, although in certain aspects a sNAG nanofiber composition does not contain collagen. In another embodiment, a sNAG nanofiber composition may contain hyaluronic acid, although in certain aspects a sNAG nanofiber composition does not contain hyaluronic acid. In another embodiment, a sNAG nanofiber composition may contain human fat, although in certain aspects a sNAG nanofiber composition does not contain human fat.

In certain embodiments, a sNAG nanofiber composition does not comprise any additional therapy. In certain embodiments, a sNAG nanofiber composition does not comprise any additional anti-viral agent, anti-cancer agent, anti-fungal agent, anti-yeast agent, anti-inflammatory agent, chemotherapeutic agent, anti-angiogenic agent, a defensin peptide, a defensin-like peptide, a Toll-receptor-like peptide, or a growth factor. In certain embodiments, a sNAG nanofiber composition does not comprise and/or is not administered in combination with zinc.

In some embodiments, the additional active ingredient is not an anti-bacterial agent (e.g., an antibiotic, a defensin peptide, a defensin-like peptide, or a Toll-receptor-like peptide), or a growth factor. In specific embodiments, the additional active ingredient is not a growth factor, such as PDGF-AA, PDGF-AB, PDGF-BB, PDGF-CC, PDGF-DD, FGF-1, FGF-2, FGF-5, FGF-7, FGF-10, EGF, TGF-α, (HB-EGF), amphiregulin, epiregulin, betacellulin, neuregulins, epigen, VEGF-A, VEGF-B, VEGF-C, VEGF-D, VEGF-E, placenta growth factor (PLGF), angiopoietin-1, angiopoietin-2, IGF-I, IGF-II, hepatocyte growth factor (HGF), and macrophage-stimulating protein (MSP). In certain embodiments, the additional active ingredient is not an agents that boost the immune system, a pain relief agent, or a fever relief agent.

In certain embodiments, the additional active ingredient is not an antibiotic.

In other aspects, a sNAG nanofiber composition does not comprise a significant amount of protein material. In specific embodiments, the protein content of a sNAG nanofiber composition is no greater than 0.1%, 0.5% or 1% by weight. In other embodiments, the protein content of the composition is undetectable by Coomassie staining.

In one embodiment, zinc is also included in a sNAG nanofiber composition. In addition to its antimicrobial properties, zinc also plays a role in wound healing (see Andrews et al., 1999, Adv Wound Care 12:137-8). The zinc is preferably added in the form of a salt, such as zinc oxide, zinc sulphate, zinc acetate or zinc gluconate.

### 5.4 Prophylactic and Therapeutic Uses

The sNAG nanofibers or compositions thereof can be used to prevent and/or treat diseases, disorders or syndromes with symptoms that would benefit from an increase in tensile strength and/or elasticity, a reduction in collagen, organization of collagen, and/or a reduction in smooth muscle actin expression or a reduction in myofibroblast content. Such diseases may be associated with or have symptoms comprising decreased tensile strength of tissue, decreased elasticity of tissue, decreased elastin content in tissue, increased total collagen content or abnormal collagen content in tissue, increased collagen type I expression in tissue, decreased collagen type III expression in tissue, abnormal alignment of collagen in tissue, increased smooth muscle actin expression in tissue, and/or increased myofibroblast content in tissue.

### Increase in tensile strength

The inventors have surprisingly discovered that sNAG nanofibers can increase the tensile strength and elasticity of tissue. Accordingly, the sNAG nanofibers described herein can be used to treat and/or prevent diseases and/or syndromes associated with a decrease in the tensile strength of tissue or a decrease in tissue elasticity.

Disclosed herein are methods for treating and/or preventing a symptom of Ehlers-Danlos syndrome in a subject, comprising administering to the subject sNAG nanofibers described herein or a composition thereof. Ehlers-Danlos syndrome is a group of heritable collagen or connective tissue disorders caused by faulty collagen. There are six major types of Ehlers-Danlos syndrome, and each type is classified based on its own unique signs and symptoms. In particular, the six major types of Ehlers-Danlos syndrome are Hypermobility type 3, Classical types 1 and 2, Vascular type 4, Kyphoscoliosis, Arthrochalasis, and Dermatosparaxis. Although the signs of the syndrome may vary depending on which type of Ehlers-Danlos syndrome the patient has, major signs and symptoms of the syndrome include: musculoskeletal symptoms (such as, *e.g.,* unstable joints that are prone to sprain, dislocation, subluxation and hyperextension, early onset of advanced osteoarthritis, chronic degenerative joint disease, swan neck deformity of the fingers, muscle fatigue that increase with use, weak muscle tone in infancy, osteopenia, stretchy ligaments and tendons, and tearing of tendons or muscles), skin symptoms (such as, *e.g.,* stretchy skin with a velvety texture, fragile skin that tears easily, easy bruising, abnormal wound healing and scar formation, leading to widened atrophic scars, redundant skin folds, molluscoid tumors, subcutaneous spheroids, fatty growths on forearms or skins, and angioplasia), and cardiovascular symptoms (such as, *e.g.,* fragile blood vessels, carotoid-cavernous fistula, valvular heart disease, postural orthostatic tachycardia syndrome, orthostatic intolerance, dilation and/or rupture of ascending aorta, cystic medial necrosis, varicose veins, and vascular skin conditions (*e.g.*, Raynaud's phenomenon or Livedo reticularis). In specific instances, the sNAG nanofibers or a composition thereof is used to treat and/or prevent one, two, three or more symptoms of Ehlers-Danlos syndrome. In some instances, the sNAG fibers or a composition thereof is used to treat and/or prevent one, two, three or more symptoms of one, two, three, four, five or all major types of Ehlers-Danlos syndrome. In a specific instance, the sNAG nanofibers or a composition thereof are used to treat and/or prevent one, two or more of the common skin-related symptoms of Ehlers-Danlos syndrome, including, *e.g.,* soft skin, fragile skin, skin that bruises easily, excessive scarring, blunted wound healing and the development of fleshy tumors over tender points in the body's tissues. In other instances, the sNAG nanofibers or a composition thereof are used to treat and/or prevent one, two or more of the joint-related symptoms of Ehlers-Danlos, including, *e.g.,* loose or unstable joints, frequent joint dislocations, joint pain and early onset of degenerative joint disease.

Disclosed herein are methods for treating and/or preventing a symptom of Epidermolysis bullosa (EB), an inherited connective tissue disease characterized by extreme fragility of the skin, in a subject, comprising administering to the subject sNAG nanofibers described herein or a composition thereof. Epidermolysis bullosa has an incidence of 1/50,000 and its severity ranges from mild to lethal. Epidermolysis bullosa can be divided into three types: Epidermolysis bullosa simplex, Junctional epidermolysis bullosa, and Dystrophic epidermolysis bullosa. In certain instances, presented herein are methods for treating and/or preventing a symptom of one, two or all types of Epidermolysis bullosa in a subject, comprising administering to the subject sNAG nanofibers described herein or a composition thereof.

In subjects with healthy skin, there are protein anchors between the layers that prevent them from moving independently from one another (*e.g*., shearing). In those born with Epiderolysis bullosa, however, those top skin layers lack the protein anchors that hold them together, and any action that creates friction between them (like rubbing or pressure) will separate the layers of the skin and cause blisters and painful sores in the skin and mucosal membranes. Thus, in one instance, the sNAG nanofibers or a composition thereof are used to prevent and/or treat symptoms of Epidermolysis bullosa, such as blisters, both on the skin and on the surface of mucosal membranes.

In another specific embodiment, presented herein are methods for treating and/or preventing wrinkles or depressions in a subject skin, comprising administering to the subject sNAG nanofibers described herein or a composition thereof. The wrinkles or depressions may be coarse or fine depending on their depth and may extend from a few micrometers to several millimeters into the skin. Specifically, coarse wrinkles, often referred to as expression lines, appear on the forehead, outer corners of the eyes (*e.g*., crow's feet) and as vertical lines on either side of the mouth (*e.g.*, laugh lines) while fine wrinkles comprise a shallower network of lines or indentations that appear on the skin, especially in areas of facial movement (*e.g*., eye, mouth, upper lip, etc.). Wrinkles or depressions in the skin occur as a result of one or more of a reduction in muscle mass and skin thickness, cross-linking of collagen and elastin in the dermis, and dehydration of the Stratum Corneum (SC). These structural changes in the skin cause a loss of mechanical strength and elasticity and culminate in visible wrinkles apparent on the surface of the skin. Thus, the sNAG nanofibers described herein or a composition thereof can be applied topically to treat and/or prevent loss of mechanical strength and elasticity in the skin and, accordingly, the wrinkles or depressions associated with those structural changes.

### Reduction in collagen

The inventors have also discovered that sNAG nanofibers can reduce collagen levels. Accordingly, the sNAG nanofibers described herein can be used to treat and/or prevent diseases and/or syndromes having symptoms associated with increased collagen formation.

Disclosed herein are methods for treating and/or preventing a symptom of scleroderma, a connective tissue disorder, in a subject, comprising administering to the subject sNAG nanofibers described herein or a composition thereof. Without being bound by any mechanism of action, it is believed that the fibroblasts (the most common cells in connective tissue) of a subject with scleroderma generate excessive amounts of collagen. This excessive collagen can form a thick band of connective tissue that accumulates within the skin and internal organs, which can impair organ functioning. Moreover, excessive collagen production can affect blood vessels and joints and may be symptomatic of diseases such as Raynaud's phenomenon and/or numbness, pain and discoloration in the fingers or toes, gastroesophageal reflux disease or GERD, and skin changes such as swollen fingers and hands, shiny skin and thickened patches of skin. Accordingly, sNAG nanofibers or a composition thereof can be used to prevent and/or treat the collagen-associated symptoms of scleroderma and/or associated diseases or syndromes. In a specific instance, the sNAG nanofibers or a composition thereof are applied topically to the skin of the affected area or locally injected to reduce collagen levels and relieve the associated symptoms.

Some types of scleroderma affect only the skin, whereas other types of scleroderma affect the entire body. Generally, localized scleroderma affects only the skin on the hands and face. In contrast, systemic scleroderma (sclerosis) may affect large areas of skin and organs, such as, *e.g.,* the heart, lungs, or kidneys. There are two types of systemic scleroderma: limited disease (CREST syndrome) and diffuse disease. In certain instances, the sNAG nanofibers or a composition thereof are used to prevent and/or treat one, two or more symptoms of systemic scleroderma. In other instances, the sNAG nanofibers or a composition thereof are used to prevent and/or treat one, two or more symptoms of localized scleroderma.

Symptoms of scleroderma include skin symptoms (*e.g*., fingers or toes that turn blue or white in response to hot and cold temperatures (*see* Raynaud's phenomenon); hair loss; skin hardness; skin that is abnormally dark or light; skin thickening, stiffness, and tightness of fingers, hands, and forearm; small white lumps beneath the skin; sores (ulcers) on the fingertips or toes; and tight and mask-like skin on the face), bone and muscle symptoms (*e.g*., joint pain; numbness and pain in the feet; pain, stiffness and swelling of fingers and joints; and wrist pain), breathing problems (e.g., dry cough, shortness of breath, and wheezing), and digestive tract problems (*e.g*., bloating after meals, constipation, diarrhea, difficulty swallowing, and esophageal reflux or heartburn). In specific instances, the sNAG nanofibers or a composition thereof are used to treat and/or prevent one, two, three or more of these symptoms of scleroderma. In certain instances, the sNAG nanofibers or a composition thereof are used to treat and/or prevent one, two, three or more of the skin symptoms of scleroderma. In some instances, the sNAG nanofibers or a composition thereof are used to treat and/prevent one, two or more of the bone and muscle symptoms of scleroderma.

### Organized collagen phenotype

The inventors have further discovered that sNAG nanofibers can increase collagen fiber organization and/or orientation. Accordingly, the sNAG nanofibers described herein can be used to treat or prevent diseases and/or syndromes having symptoms associated with poor organization of collagen fibers.

The sNAG nanofibers described herein or a composition thereof are used to treat low bone density in a subject. Increased skeletal fragility and bone brittleness is attributed to reduced strength of the bone matrix. This reduced strength in bone matrix is in turn thought to result, in part, from poor organization of collagen fiber. Thus, the sNAG nanofibers described herein or a composition thereof can be used to treat diseases and/or disorders characterized by low bone density (*e.g*., osteoporosis). The sNAG nanofibers or a composition thereof can be injected into areas of low bone density to increase or correct collagen fiber organization or orientation.

Disclosed herein are methods for treating and/or preventing a symptom of osteoporosis in a subject, comprising administering to the subject sNAG nanofibers described herein or a composition thereof. In certain instances, the sNAG nanofibers or a composition thereof are injected into an area of low bone density. The sNAG nanofibers may be administered in the form of, *e.g.,* a gel or a suspension.

Disclosed herein are methods for treating and/or preventing a symptom of intervertebral disc disorder and/or degenerative disc disorder in a subject, comprising administering to the subject sNAG nanofibers described herein or a composition thereof. The morphology of the intervertebral disc is dependent on the type of components present and the manner in which they are assembled. This, in turn, will determine how the tissue carries out its primary physiological functions of load bearing and allowing movement in all directions of the otherwise rigid spine. Although the components of the disc start out ordered, with the outer annulus fibrosus consisting of a series of regular concentric bundles of collagen fibers around the central gelatinous nucleus pulposus, upon advancing age, there is increased complexity of lamellae, with more bifurcations, interdigitations and irregularity in number and size of lamellar bands. The change in the collagen organization of the intervertebral disc can lead to altered load bearing, and may establish a self-perpetuating cycle of disruption to disc morphology, which, once started, could be irreversible. There are also alterations to cell organization the intervertebral disc with disease and degeneration. Accordingly, the sNAG nanofibers or a composition thereof can be used to treat and/or prevent a symptom of intervertebral disc disorder of degenerative disc disorder by topically administering the sNAG nanofibers, by, *e.g.*, injecting the sNAG nanofiber composition into, around or near the diseased disc. In particular, the sNAG nanofibers or a composition thereof can be injected into the disc between the L4 and L5 vetebrae.

Disclosed herein are methods for treating and/or preventing a symptom of osteoarthritis in a subject, comprising administering to the subject sNAG nanofibers described herein or a composition thereof. Break down of the joint cartilage is primarily responsible for osteoarthritis, and collagen makes up 95% of this joint cartilage. The rapid loss of proteoglycan content relative to collagen during the progression of osteoarthritis leads to a severe perturbation of collagen organization in the joint, and the pain and stiffness that characterizes osteoarthritis. Thus, the sNAG nanofibers described herein or a composition thereof can be used to treat and/or prevent osteoarthritis and restore this collagen structure/organization. The sNAG nanofibers described herein or a composition thereof can be applied topically by injecting the sNAG nanofibers into the affected osteoarthritic joint(s) of an individual.

### Reduction in smooth muscle actin expression/reduction in myofibroblast content

The inventors have also discovered that sNAG nanofibers can reduce smooth muscle actin expression and/or reduce myofibroblast content. Accordingly, the sNAG nanofibers described herein can be used to treat and/or prevent diseases and/or syndromes having symptoms associated with increased smooth muscle actin expression or myofibroblast content.

Disclosed herein are methods for treating and/or preventing fibrosis and/or scarring in a subject, comprising administering to the subject sNAG nanofibers described herein or a composition thereof. Fibrosis is the formation of excess fibrous connective tissue in an organ or tissue in a reparative or reactive process. Scarring is confluent fibrosis that obliterates the architecture of the underlying organ or tissue. Tissue destruction by organ fibrosis contributes to the lethal outcomes associated with heart, lung, liver, kidney, and skin diseases. The cell responsible for the detrimental fibrotic tissue contractures is the myofibroblast, which has a phenotype characterized by excessive production of collagenous extracellular matrix (ECM) and tensile force. Myofibroblasts play a pivotal role in the establishment of fibrotic conditions in the tissue and, further, depend on the expression of α-smooth muscle actin to form fibrotic stress fibers in tissue. Thus, the sNAG nanofibers described herein or a composition thereof can be used to treat the fibrosis that characterizes a number of diseases and conditions. The sNAG nanofibers or a composition thereof can be administered topically on the skin or by injecting them into, around or near the affected tissue and/or organ. In one embodiment, the sNAG nanofibers described herein or a composition thereof is not used to treat fibrosis associated with inflammatory bowel disease, and/or is not used to treat inflammatory bowel disease.

Disclosed herein are methods for treating and/or preventing scarring associated with wounds (such as cutaneous wounds) in a subject, comprising administering to the subject sNAG nanofibers described herein or a composition thereof. For example, the subject sNAG nanofibers described herein or a composition thereof can be administered to a fresh wound or to a wound 1 h, 6 h, 12 h, 24 h, 5 days, 1 week, 2 weeks, 1 month, 2 months, 3 months, 6 months, or later after the infliction of the wound, to treat scarring associated with the wound in a subject. In some instances, sNAG nanofibers described herein or a composition thereof are administered to a partially healed or a fully healed wound to treat scarring associated with the wound in the subject. In specific instances, sNAG nanofibers described herein or a composition thereof are administered directly to and/or in the proximity to the wound or to the scar left by the wound. The wounds contemplated herein can be any wound such as a surgical wound, a gunshot wound, a laceration, an incision, a penetration, an abrasion, or a burn. The wound can be an open wound. The wound can be a wound that is at an increased risk of causing scarring.

Disclosed herein is a method of reducing scarring associated with wounds in a subject (e.g., human), comprising administering a composition comprising sNAG nanofibers to a wound in the subject, wherein more than 50% of the sNAG nanofibers are between about 1 to 15 µm in length. In one instance, the wound is a surgical wound. In another instance, the wound is a cutaneous wound. In a specific instance, the composition is administered topically to the subject. In one instance, the subject has a scar from a wound, and the sNAG nanofibers are administered topically to the area of the scar. In a particular instance, the composition is administered topically for at least 21 days. In another instance, the subject has a scar from a wound, and the sNAG nanofibers are administered by local injection into the area of the scar.

Disclosed herein is a method for treating or preventing fibrosis or adhesions associated with wounds in a subject (e.g., human), comprising administering a composition comprising sNAG nanofibers to a wound in the subject, wherein more than 50% of the sNAG nanofibers are between about 1 to 15 µm in length. In one instance, the wound is a surgical wound. In another instance, the wound is a cutaneous wound. In a specific instance, the composition is administered topically to the subject. In one instance, the subject has an adhesion or fibrosis from a wound, and the sNAG nanofibers are administered topically to the area of adhesion or fibrosis. In a particular instance, the composition is administered topically for at least 21 days. In another instance, the subject has an adhesion or fibrosis from a wound, and the sNAG nanofibers are administered by local injection into the area of adhesion or fibrosis.

### Regenerative applications

Because the inventors found that sNAG nanofibers can increase collagen fiber organization and/or orientation, increase tensile strengths, and increase tissue elasticity and elastin content, the sNAG nanofibers described herein can be used to regenerate and/or fill tissues in a subject.

Disclosed herein is a method for using a composition comprising sNAG nanofibers to regenerate or add volume to a tissue, wherein more than 50% of the sNAG nanofibers are between about 1 to 15 µm in length. In specific instances, provided herein is a method for regenerating a tissue in a subject (e.g., human), comprising administering a composition comprising sNAG nanofibers to the tissue, wherein more than 50% of the sNAG nanofibers are between about 1 to 15 µm in length. The tissue can be a soft tissue (e.g., skin or muscle) or a hard tissue (e.g., bone). In some instances, the composition is used for regenerating a tissue in the context of a cosmetic or surgical procedure. In one instance, the composition is used for regenerating a tissue in the context of a plastic surgery procedure. In some of these applications, the sNAG nanofibers are formulated and/or used as a tissue filler. In particular instances, the tissue is a soft tissue of the face of a human subject (e.g., the composition can be used to augment, lift or add volume to lips, cheeks, lids, chin, or facial wrinkles or depressions). In some of these applications, the sNAG nanofibers are formulated together with and/or used in combination with another tissue filler. In a specific instance, the composition is administered topically to the tissue in the subject. In other instances, the composition is administered by local injection into the tissue. In some instances, the composition is administered intradermally, subcutaneously or intramuscularly.

The tissue filler compositions comprising sNAG nanofibers can be used in any cosmetic or surgical procedure including, without limitation, for reducing wrinkles or depressions in the skin's surface, and for adding volume to and/or altering the shape of tissue under the skin's surface. The tissue filler compositions comprising sNAG nanofibers can also be used to restore or regenerate a tissue affected by or lost due to a disease or surgery.

In a specific embodiment, provided herein is a method for treating or preventing wrinkles or depressions in the skin's surface in a subject, comprising administering a composition comprising sNAG nanofibers to the subject, wherein more than 50% of the sNAG nanofibers are between about 1 to 15 µm in length. In particular embodiments, the composition is formulated and/or used as a dermal filler for treating or preventing wrinkles or depressions in the skin's surface in a subject. In some embodiments, provided herein is a method for reducing a wrinkle or a depression in an area on the skin's surface during a cosmetic or plastic surgery procedure in a subject, comprising locally injecting a composition comprising sNAG nanofibers into the area under the skin's surface, wherein more than 50% of the sNAG nanofibers are between about 1 to 15 µm in length.

Disclosed herein is a method for adding volume to a tissue during a cosmetic or plastic surgery procedure in a subject, comprising administering a composition comprising sNAG nanofibers to the tissue, wherein more than 50% of the sNAG nanofibers are between about 1 to 15 µm in length. In some embodiments, provided herein is a method for adding volume to a tissue during a cosmetic or plastic surgery procedure in a subject, comprising locally injecting a composition comprising sNAG nanofibers into the tissue, wherein more than 50% of the sNAG nanofibers are between about 1 to 15 µm in length.

In particular instances, the sNAG nanofiber composition is formulated with another agent known in the art to be useful as a tissue filler (e.g., dermal filler) for cosmetic and/or surgical applications. For example, the composition can be formulated with or used in combination with hyaluronic acid, collagen, calcium hydroxylapatite, fat (e.g., human fat, in particular, autologous fat), polylactic acid, cadaveric dermis (e.g., human), polymethylmethacrylate, and/or polyalkylimide. In yet another embodiment, the sNAG nanofiber composition is not formulated with or used in combination with another tissue filler (e.g., dermal filler). In one instance, the sNAG nanofiber composition is formulated with or used in combination with botulinum toxin. In one instance, the sNAG nanofiber composition is not formulated with botulinum toxin. In one instance, the sNAG nanofiber composition is not used in combination with botulinum toxin.

### Defensins

The sNAG nanofibers described herein or a composition thereof are used to treat and/or prevent a disease which is associated with no or low level of expression of one or more defensin peptides; or a mutation/deletion/low gene copy number ("GCN") in a gene or genes encoding one or more of defensin peptides. Exemplary defensin genes that may be mutated/deleted/have low GCN/not expressed or whose expression may be low or altered include any of the known α-defensins. In some instances, the sNAG nanofibers described herein or a composition thereof are used to treat and/or prevent a disease which is associated with no, low, or altered level of expression of or a mutation/deletion/low GCN of one or more Toll receptors (e.g., TLR1, TLR2, TLR3, TLR4, TLR5, TLR6, TLR7, TLR8, TLR9, TLR10, TLR11, and/or TLR12). In yet other instances, the compositions described herein are used to treat and/or prevent a disease which is associated with no, low, or altered level of expression of or a mutation/deletion/low GCN of one or more of IL-1, CEACAM3, SPAG11, SIGIRR (IL1-like receptor), IRAK1, IRAK2, IRAK4, TBK1, TRAF6 and IKKi.

### 5.5 Patient Populations

A composition described herein may be administered to a naive subject, *i.e.,* a subject that does not have a disease or disorder. In one embodiment, a composition described herein is administered to a naive subject that is at risk of acquiring a disease or disorder.

An sNAG nanofiber composition described herein may be administered to a patient who has been diagnosed with a disease or disorder. In one instance, a composition described herein may be administered to a patient who displays one or more symptoms of a disease or disorder. In certain instances, a patient is diagnosed with a disease or disorder prior to administration of a composition described herein

In other specific instances, the compositions described herein may be administered to a patient diagnosed with or displaying one or more symptoms of a disease described herein, *e.g.,* Ehlers-Danlos syndrome, Epidermolysis bullosa, scleroderma, osteoporosis, intervertebral disc disorder, degenerative disc disorder, osteoarthritis, or fibrosis. In other specific embodiments, the compositions described herein may be administered to a patient that has a condition associated with decreased tensile strength and/or elasticity of the skin, such as wrinkles or depressions in the skin's surface. In one instance, the compositions described herein may be administered to a patient that has a scar. In another instance, the composition described herein is administered to a patient that does not have a wound. In particular instances, the compositions described herein may be administered to a patient that has a wound (e.g., a cutaneous wound), for example, to a wound that is at risk of causing scarring upon healing. In specific instances, the compositions described herein may be administered to a patient that is at an increased risk of scarring. In certain instances, a patient is diagnosed with a condition and a disease (e.g., one of the diseases listed above) or displays one or more symptoms of a condition and a disease prior to administration of a composition described herein. A disease may be diagnosed by any method known to a skilled artisan, including evaluation of the patient's symptoms and/or detection of a pathogen in a biological sample of the patient (e.g., as described above). In one example, the compositions described herein may be administered to a patient diagnosed with a disease or condition by a treating physician or another medical professional. In another example, a patient may use the compositions described herein upon detection of one or more symptoms of a disease or condition.

In certain embodiments, a subject to be administered a composition described herein is a subject with high level of expression of (or, for other reasons, high cellular or tissue content of) collagen type I and smooth muscle actin (e.g., alpha smooth muscle actin). In some embodiments, a subject to be administered a composition described herein is a subject with high level of expression of (or, for other reasons, high cellular or tissue content of) collagen proteins (as measured by the total collagen content). In certain embodiments, a subject to be administered a composition described herein is a subject with no or low level of expression of or a mutation/deletion in (or, for other reasons, low cellular or tissue content of) elastin protein or collagen type III.

In certain embodiments, a subject to be administered a composition described herein is a subject with no or low level of expression of one or more defensin peptides or a mutation/deletion in a gene or genes encoding one or more defensin peptides. In some embodiments, a subject to be administered a composition described herein is a subject with no or low or altered level of expression of one or more α-defensins (e.g., DEFA1, DEFA1B, DEFA3, DEFA4, DEFA5, DEFA6), one or more β-defensins (e.g., DEFB1, DEFB2, DEFB4, DEFB103A, DEFB104A, DEFB105B, DEFB107B, DEFB108B, DEFB110, DEFB112, DEFB114, DEFB118, DEFB119, DEFB123, DEFB124, DEFB125, DEFB126, DEFB127, DEFB128, DEFB129, DEFB131, DEFB136), and/or one or more θ-defensins (e.g., DEFT1P). In some embodiment, a subject to be administered a composition described herein is a subject with no or low or altered level of expression of one or more of DEFA1, DEFA3, DEFA4, DEFA5, DEFB1, DEFB3, DEFB103A, DEFB104A, DEFB108B, DEFB112, DEFB114, DEFB118, DEFB119, DEFB123, DEFB124, DEFB125, DEFB126, DEFB128, DEFB129 and DEFB131. In certain embodiments, a subject to be administered a composition described herein is a subject with no or low or altered level of expression of one or more Toll receptors (e.g., TLR1, TLR2, TLR3, TLR4, TLR5, TLR6, TLR7, TLR8, TLR9, TLR10, TLR11, and/or TLR12). In yet other embodiments, a subject to be administered a composition described herein is a subject with no or low or altered level of expression of one or more of IL-1, CEACAM3, SPAG11, SIGIRR (IL1-like receptor), IRAK1, IRAK2, IRAK4, TBK1, TRAF6 and IKKi. In some embodiments, a subject to be administered a composition described herein is a subject with no or low or altered level of expression of one or more of IRAK2, SIGIRR, TLR1, TLR2, TLR4, TLR7, TLR8, TLR10 and TRAF6.

In certain embodiments, a subject to be administered a composition described herein is a subject with low, normal or high level of expression of AKT1. In particular embodiments, a subject to be administered a composition described herein is a subject with normal or high level of expression of AKT1. In certain embodiments, a subject to be administered a composition described herein is a subject that has a detectable level of Aktl protein. In certain embodiments, a subject to be administered a composition described herein is a subject who is not undergoing therapy with an agent that is known to decrease the expression of AKT1 or level of Akt1 protein.

In certain embodiments, a subject to be administered a composition described herein is a subject has a decreased tensile strength of tissue (e.g., skin), a decreased elasticity of tissue (e.g., skin), an abnormal (e.g., disorganized) alignment of collagen in tissue (e.g., skin), and/or an increased myofibroblast content in tissue (e.g., skin). In one embodiment, the subject has a decreased tensile strength of tissue (e.g., skin). In one embodiment, the subject has a decreased elasticity of tissue (e.g., skin). In one embodiment, the subject has an abnormal (e.g., disorganized) alignment of collagen in tissue (e.g., skin). In one embodiment, the subject has an increased myofibroblast content in tissue (e.g., skin). An increase or a decrease in a characteristic or property of a tissue is a difference that is more than 1.25 fold, 1.5 fold, 2 fold, 2.5 fold, 3 fold, 3.5 fold, 4 fold, 4.5 fold, 5 fold, 6 fold, 7 fold, 8 fold, 9 fold, or 10 fold than the normal characteristic or property of the tissue. Wherein the "normal" characteristic or property of the tissue is: (i) the average characteristic or property of the tissue known to be found in subjects not displaying symptoms or not diagnosed with the condition and disease to be treated; (ii) the average characteristic or property of the tissue detected in three, five, ten, twenty, twenty-five, fifty or more subjects not displaying symptoms or not diagnosed with the condition and disease to be treated; and/or (iii) the characteristic or property of the tissue detected in a patient to be administered a composition described herein before the onset of the condition and disease.

In certain instances, a composition described herein is administered to a patient who has (*e.g.,* has been diagnosed with) Ehlers-Danlos syndrome or displays one, two or more symptoms of Ehlers-Danlos syndrome.

In certain instances, a composition described herein is administered to a patient who has *(e.g.,* has been diagnosed with) Epidermolysis bullosa or displays one, two or more symptoms of Epidermolysis bullosa.

In certain embodiments, a composition described herein is administered to a patient who has wrinkles and/or skin depressions.

In certain instances, a composition described herein is administered to a patient who is undergoing a cosmetic procedure or plastic surgery. In certain instances, a composition described herein is administered to a patient in need of a tissue filler, e.g., a soft tissue filler or a hard tissue filler. In certain instances, a composition described herein is administered to a patient in need of a dermal filler. In certain instances, a composition described herein is administered to a patient in need of tissue regeneration (for example, in need of restoring a tissue depleted or lost due to surgery or disease).

In certain instances, a composition described herein is administered to a patient in need of tissue regeneration (e.g., regeneration of a soft tissue or regeneration of a hard tissue). In one instance, a composition described herein is administered to a patient in need of regeneration of a soft tissue (e.g., skin, connective tissue, or muscle). In one instance, a composition described herein is administered to a patient in need of regeneration of a hard tissue (e.g., bone).

In certain instances, a composition described herein is administered to a patient who has a scar (e.g., a scar caused by a wound such a cutaneous wound). In some instances, a composition described herein is administered to a patient who has a wound such a cutaneous wound (e.g., a wound that is at risk of causing scarring upon healing).

In other instances, a composition described herein is not administered to a patient who has a wound (e.g., a cutaneous wound).

In certain instances, a composition described herein is administered to a patient who has (*e.g.,* has been diagnosed with) scleroderma or displays one, two or more symptoms of scleroderma.

In certain instances, a composition described herein is administered to a patient who has (*e.g.,* has been diagnosed with) osteoporosis or displays one or more symptoms of osteoporosis.

In certain instances, a composition described herein is administered to a patient who has (*e.g.,* has been diagnosed with) intervertebral disc disorder or displays one or more symptoms of intervertebral disc disorder.

In certain instances, a composition described herein is administered to a patient who has (e.g., has been diagnosed with) degenerative disc disorder or displays one or more symptoms of degenerative disc disorder.

In certain instances, a composition described herein is administered to a patient who has (e.g., has been diagnosed with) osteoarthitis or displays one or more symptoms of osteoarthritis.

In certain instances, a composition described herein is administered to a patient who has (e.g., has been diagnosed with) fibrosis or displays one or more symptoms of fibrosis. In some instances, the treated patient does not have an inflammatory bowel disease, or fibrosis associated with inflammatory bowel disease.

In certain instances, a composition described herein is administered to a patient who has (e.g., has been diagnosed with) an adhesion associated with a wound or is at risk of an adhesion associated with a wound. The wound may be a cutaneous wound or a surgical wound.

In certain instances, a composition described herein is administered to a patient who has (e.g., has been diagnosed with) fibrosis associated with a wound or is at risk of fibrosis associated with a wound. The wound may be a cutaneous wound or a surgical wound.

In some instances, a composition described herein is administered to an immunosuppressed patient, and/or a patient susceptible to acute or chronic disease or infection (e.g., an HIV positive patient, or a patient immunosuppressed as a result of cancer treatment or a transplantation procedure). In one instance, a composition described herein is administered to a patient diagnosed with cystic fibrosis.

In some instances, a composition described herein is administered to a patient with a condition and disease before symptoms of the condition and disease manifest or before symptoms of the condition and disease become severe (e.g., before the patient requires treatment or hospitalization). In some instances, a composition described herein is administered to a patient with a disease after symptoms of the disease, disorder or condition manifest or after symptoms of the condition and disease become severe (e.g., after the patient requires treatment or hospitalization).

In some instances, a subject to be administered a composition described herein is an animal. In certain instances, the animal is a bird. In certain instances, the animal is a canine. In certain instances, the animal is a feline. In certain instances, the animal is a horse. In certain instances, the animal is a cow. In certain instances, the animal is a mammal, *e.g*., a horse, swine, mouse, or primate, preferably a human.

In certain embodiments, a subject to be administered a composition described herein is a human adult. In certain embodiments, a subject to be administered a composition described herein is a human adult more than 50 years old. In certain embodiments, a subject to be administered a composition described herein is an elderly human subject.

In certain embodiments, a subject to be administered a composition described herein is a human toddler. In certain embodiments, a subject to be administered a composition described herein is a human child. In certain embodiments, a subject to be administered a composition described herein is a human infant. In certain embodiments, a subject to be administered a composition described herein is a premature human infant.

In a specific embodiment, a composition described herein is not administered to a subject to treat a wound (e.g., an open wound such as an incision, a laceration, a penetration, an abrasion, or a burn).

### 5.6 Modes of Administration

In certain embodiments, methods are described herein for treating and/or preventing a condition and disease or a symptom thereof, wherein a composition comprising the sNAG nanofibers is topically administered to a patient in need of such treatment.

In other embodiments, methods are described herein for treating and/or preventing a condition and disease or a symptom thereof, wherein a composition comprising the sNAG nanofibers is injected locally (i.e., non-systemically) into an organ or tissue of a patient in need of such treatment. The composition can be injection locally into a soft tissue or into a hard tissue. For example, a composition comprising the sNAG nanofibers can be injected into a bone (e.g., into the area of low bone density), or injected into an intervertebral disc (e.g., between L4 and L5). In other examples, a composition comprising the sNAG nanofibers can be administered to the surface of a bone, or administered to the surface of an intervertebral disc. In some embodiments, methods are described herein for regenerating a tissue, adding volume to a tissue, or treating or preventing a wrinkle or a depression in the skin's surface, wherein a composition comprising the sNAG nanofibers is injected locally (i.e., non-systemically) into an organ or tissue of a patient in need of such treatment.

In some instances, methods are described herein for treating and/or preventing a condition and disease or a symptom thereof, wherein a composition comprising the sNAG nanofibers is topically administered to a surface tissue (e.g., skin surface or mucosal surface) in a patient in need of such treatment. In other instances, methods are described herein for treating or preventing a condition and disease or a symptom thereof, wherein a composition comprising the sNAG nanofibers is topically administered to an internal organ or tissue (e.g., post-operatively) in a patient in need of such treatment.

In some embodiments, an effective amount of the sNAG nanofibers and/or a sNAG nanofiber composition is administered to a subject.

In some embodiments, a composition comprising the sNAG nanofibers is administered topically to the skin, for example, to the site of symptom of a condition and disease. In yet other embodiments, a composition comprising the sNAG nanofibers is administered topically to the site and around the site of a condition and disease (e.g., the site of symptom of a condition and disease). In yet other embodiments, a composition comprising sNAG nanofibers is applied in proximity to the site of a condition and disease (e.g., the site of symptom of a disease or disorder). In yet another instance, a composition comprising the sNAG nanofibers is administered topically to the site at high risk of a condition and disease (e.g., the site of symptom of such condition and disease).

The sNAG nanofiber compositions described herein may be administered by any of the many suitable means of topical administration which are well known to those skilled in the art, including but not limited to topically to the skin, topically to any other surface of the body (e.g., mucosal surface), by inhalation, intranasally, vaginally, rectally, buccally, or sublingually. The mode of topical administration may vary depending upon the condition and disease to be treated or prevented. The sNAG nanofiber compositions can be formulated for the various types of topical administration.

In a specific embodiment, the compositions disclosed herein are applied topically, for example to the skin of a patient in need of such treatment or to another tissue of a patient in need of such treatment. In some embodiments, the compositions may be applied directly to the site of a condition and disease (or a symptom thereof) and/or in the proximity to the site of a condition and disease (or a symptom thereof). In some instances, the compositions may be applied directly to a site where a condition and disease might potentially develop (e.g., to an open wound).

In certain instances, methods are described herein for treating and/or preventing a condition and disease or a symptom thereof, wherein a composition comprising the sNAG nanofibers is systemically (e.g., parenterally) administered to a patient in need of such treatment.

In certain instances, methods are described herein for treating and/or preventing a condition and disease or a symptom thereof, wherein a composition comprising the sNAG nanofibers is orally administered to a patient in need of such treatment.

In certain embodiments, methods are described herein for treating and/or preventing a condition and disease or a symptom thereof, wherein a composition comprising the sNAG nanofibers is intramuscularly administered to a patient in need of such treatment. In specific embodiments, methods are described herein for regenerating a tissue, adding volume to a tissue or treating or preventing a wrinkle or a depression in the skin's surface, wherein a composition comprising the sNAG nanofibers is intramuscularly administered to a patient in need of such treatment.

In certain instances, methods are described herein for treating and/or preventing a condition and disease or a symptom thereof, wherein a composition comprising the sNAG nanofibers is intraveneously administered to a patient in need of such treatment.

In certain instances, methods are described herein for treating and/or preventing a condition and disease or a symptom thereof, wherein a composition comprising the sNAG nanofibers is rectally administered to a patient in need of such treatment.

In certain embodiments, methods are described herein for treating and/or preventing a condition and disease or a symptom thereof, wherein a composition comprising the sNAG nanofibers is subcutaneously administered to a patient in need of such treatment. In specific embodiments, methods are described herein for regenerating a tissue, adding volume to a tissue or treating or preventing a wrinkle or a depression in the skin's surface, wherein a composition comprising the sNAG nanofibers is subcutaneously administered to a patient in need of such treatment.

In certain embodiments, methods are described herein for treating and/or preventing a condition and disease or a symptom thereof, wherein a composition comprising the sNAG nanofibers is intradermally administered to a patient in need of such treatment. In specific embodiments, methods are described herein for regenerating a tissue, adding volume to a tissue or treating or preventing a wrinkle or a depression in the skin's surface, wherein a composition comprising the sNAG nanofibers is intradermally administered to a patient in need of such treatment.

In other embodiments, methods are described herein for treating and/or preventing a condition and disease or a symptom thereof, wherein a composition comprising the sNAG nanofibers is not systemically (e.g., parenterally) administered to a patient in need of such treatment.

In other embodiments, methods are described herein for treating and/or preventing a condition and disease or a symptom thereof, wherein a composition comprising the sNAG nanofibers is locally injected into a tissue of a patient in need of such treatment. In specific embodiments, methods are described herein for regenerating a tissue or adding volume to a tissue, wherein a composition comprising the sNAG nanofibers is administered by local injection into the tissue of a patient in need of such treatment. In other embodiments, methods are described herein for treating or preventing a wrinkle or a depression in the skin's surface, wherein a composition comprising the sNAG nanofibers is administered by local injection directly into the wrinkle or depression under the skin's surface of a patient in need of such treatment.

In one embodiment, a composition comprising sNAG nanofibers is applied to the skin of a patient. For example, such a composition may be applied topically to the skin of a patient for treating or preventing a condition and disease of the skin. In another embodiment, the composition can be applied by injection to an area directly under the skin of a patient, for example, for regenerating or adding volume to the area directly under the skin.

In another instance, a composition described herein may be applied topically to a mucosal surface of a patient. For example, such a composition may be applied topically to the oral mucosa for treating or preventing condition and disease of the mouth or gums.

The above-listed methods for topical administration may include administration of a sNAG nanofiber in the form of a suspension (e.g., a thick suspension), a cream, an ointment, a gel, a liquid solution, a membrane, a spray, a paste, a powder or any other formulation described herein or known in the art. A sNAG nanofiber may also be applied in a dressing or a bandage, for example to treat localized diseases or infections on the skin of a patient. In particular embodiments, compositions comprising sNAG nanofibers are not solid or barrier-forming.

In some embodiments, a composition described herein may be applied as a spray.

In some embodiments, a composition described herein may be applied topically with a syringe or another type of applicator (e.g., a spatula, a cotton swab, a tube such as a squeeze tube) suitable for topical delivery of the composition to the patient. For example, a composition described herein formulated as a suspension (e.g., thick suspension), a liquid solution, a cream, an ointment, or a gel can be administered topically to the skin, mucous membrane or other surface tissue of a patient via an applicator (e.g., syringe).

A composition described herein may be applied at the site of a surgical procedure. For example, such composition may be sprayed, applied as a cream, suspension (e.g., a thick suspension), liquid solution, ointment, gel, membrane, or powder, or coated on the surface of the tissue or organ to be subjected to a surgical procedure or that has been subjected to the surgical procedure. In one instance, a composition described herein is applied at the site of the surgical incision, at the site of the excised tissue, or at the site of surgical stitches or sutures. Such administration of a composition described herein may, e.g., treat or prevent tissue fibrosis. For example, a composition described herein may be used during or after a surgical procedure which is known to pose high risk of fibrosis. A composition described herein may be applied at the site of any of the above-listed or other surgical procedures.

In some embodiments, a composition described herein may be applied by injection with a syringe.

In yet other instances, a composition described herein may be coated on a device, for example an oral hygiene product, a catheter, a surgical instrument or another product, to be used in or inserted into a patient, in order to treat or prevent a condition and disease in a patient.

In some instances, methods contemplated herein include a step that includes detection/diagnosis of a disease in a patient. In some instances, detection/diagnosis involves a test or assay for the disease in a biological sample of the patient. In other instances, diagnosis involves assessing whether the patient has one or more symptoms of a disease.

The compositions described herein may exhibit sustained release properties and/or may be administered in a formulation resulting in a sustained release of such compositions. In some embodiments, the sNAG nanofibers biodegrade over time as described in Section 5.1, *supra,* and these properties of sNAG nanofibers may lead to or contribute to sustained release of the compositions described herein. In yet other embodiments, the compositions described herein are formulated to display sustained release capabilities using any methods known in the art. The compositions described herein may exhibit sustained release over a time period equal to or more than about 6 hours, 12 hours, 18 hours, 24 hours (1 day), 2 days, 3 days, 5 days, 7 days (1 week), 10 days, 14 days (2 weeks), 3 weeks or 4 weeks after administration of the composition to the patient.

Contemplated treatment regimes include a single dose or a single application of a sNAG nanofiber composition; two doses or two applications of a sNAG nanofiber composition; or a regiment of multiple doses or multiple applications of a sNAG nanofiber composition. A dose or an application may be administered hourly, daily, weekly or monthly. For example, a dose of a sNAG nanofiber composition may be administered once a day, twice a day, three times a day, four times a day, once a week, 2 times a week, 3 times a week, every other day, once in 2 weeks, once in 3 weeks, once in 4 weeks, once a month, or once in two months.

A sNAG nanofiber composition may be administered for a duration equal to or greater than 2 days, 3 days, 4 days, 5 days, 1 week, 2 weeks, 3 weeks, 1 month, 2 months, 3 months, 4 months, 5 months, 6 months, 9 months, 1 year, 1.5 years, 2 years, 2.5 years, 3 years, 4 years, 5 years, 7 years, 10 years or more. In some embodiments, a sNAG fiber composition is administered to a patient once or twice a day for a duration equal to or greater than 2 days, 3 days, 4 days, 5 days, 1 week, 2 weeks, 3 weeks, 1 month, 2 months, 3 months, 4 months, 5 months, 6 months, 9 months, or 1 year. In one such embodiment, a sNAG nanofiber composition does not cause any side effects or causes only mild side effects during the duration of the treatment. In another embodiment, a sNAG nanofiber composition does not cause irritation (e.g., moderate or severe irritation) or allergy (e.g., moderate or severe allergy).

The concentration of sNAG nanofibers in a composition may vary. In general, an effective amount of sNAG nanofibers are used in the compositions described herein to treat the conditions or diseases described herein. An effective amount may be an amount sufficient to achieve one or more of the effects described herein, for example an amount effective to treat a disease or reduce or eradicate one or more symptoms of a condition and disease. For example, a composition may comprise about 0.2 to 20 mg/cm² of sNAG nanofibers per dose/application of the composition in a form suitable for topical delivery to a patient. In certain embodiments, a composition described herein comprises about 0.25 to 20 mg/cm², about 0.5 to 20 mg/cm², about 1 to 20 mg/cm², about 1 to 15 mg/cm², about 1 to 12 mg/cm², about 1 to 10 mg/cm², about 1 to 8 mg/cm², about 1 to 5 mg/cm², about 2 to 8 mg/cm², or about 2 to 6 mg/cm² of sNAG nanofibers per dose/application of the composition in a form suitable for topical delivery to a patient. In some embodiments, compositions described herein can comprise about 5 to 50 mg/ml of sNAG nanofibers per dose/application of the composition in a form suitable for topical delivery to a patient. In certain embodiments, a composition described herein comprises about 5 to 40 mg/ml, about 5 to 35 mg/ml, about 10 to 50 mg/ml, about 10 to 40 mg/ml, about 10 to 35 mg/ml, about 10 to 30 mg/ml, about 15 to 40 mg/ml, about 15 to 35 mg/ml, about 15 to 30 mg/ml, or about 20 to 30 mg/ml of sNAG nanofibers per dose/application of the composition in a form suitable for topical delivery to a patient. In specific embodiments, a composition described herein comprises about 10 mg/ml, 12 mg/ml, 15 mg/ml, 20 mg/ml, 25 mg/ml or 30 mg/ml of sNAG nanofibers per dose/application of the composition in a form suitable for topical delivery to a patient. In certain embodiments, compositions described herein can comprise an amount of total solution or suspension (comprising sNAG nanofibers) in the range of about 50 to 100 µl, 50 to 200 µl, 50 to 250 µl, 50 to 300 µl, 50 to 350 µl, 50 to 400 µl, 50 to 450 µl, 50 to 500 µl, 100 to 200 µl, 100 to 300 µl, 100 to 400 µl, 100 to 500 µl per 0.5 cm² or 1 cm² of the surface to be treated in a patient (e.g., skin, mucosal surface or other tissue surface). The total solution or suspension can comprise saline, buffer, solution (e.g., Hank buffer solution), or any other physiologically compatible solution.

In other embodiments, a sNAG nanofiber composition can comprise about 5 mg/ml to 30 mg/ml of sNAG nanofibers per dose/application in a form suitable for administration by local injection (e.g., intradermal, intracutaneous or intramuscular injection) into a patient. In particular, a sNAG nanofiber composition can comprise about 5 mg/ml, 6 mg/ml, 7 mg/ml, 7.5 mg/ml, 8 mg/ml, 9 mg/ml, 10 mg/ml, 11 mg/ml, 12 mg/ml, 12.5 mg/ml, 13, mg/ml, 14 mg/ml, 15 mg/ml, 16 mg/ml, 17 mg/ml, 17.5 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, or 30 mg/ml of sNAG nanofibers per dose/application in a form suitable for administration by local injection. In some embodiments, a sNAG nanofiber composition can comprise about 7.5 mg/ml to 25 mg/ml of sNAG nanofibers per dose/application in a form suitable for administration by local injection into a patient. In some embodiments, a sNAG nanofiber composition can comprise about 10 mg/ml to 20 mg/ml of sNAG nanofibers per dose/application in a form suitable for administration by local injection into a patient. In one embodiment, a sNAG nanofiber composition can comprise about 10 mg/ml, about 15 mg/ml, or about 20 mg/ml of sNAG nanofibers per dose/application in a form suitable for administration by local injection into a patient.

In other embodiments, a sNAG nanofiber composition can comprise about 0.1 mg/ml to 50 mg/ml of sNAG nanofibers per dose/application in a form suitable for topical delivery to a patient. In particular, a sNAG nanofiber composition can comprise about 0.1 mg/ml, 0.5 mg/ml, 1 mg/ml, 2 mg/ml, 3 mg/ml, 4 mg/ml, 5 mg/ml, 6 mg/ml, 7 mg/ml, 7.5 mg/ml, 8 mg/ml, 9 mg/ml, 10 mg/ml, 11 mg/ml, 12 mg/ml, 12.5 mg/ml, 13, mg/ml, 14 mg/ml, 15 mg/ml, 16 mg/ml, 17 mg/ml, 17.5 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, or 50 mg/ml of sNAG nanofibers per dose/application in a form suitable for topical delivery to a patient. In some embodiments, a sNAG nanofiber composition can comprise about 0.5 mg/ml to 50 mg/ml, 0.5 mg/ml to 30 mg/ml, 1 mg/ml to 50 mg/ml, 1 mg/ml to 40 mg/ml, 1 mg/ml to 30 mg/ml, 5 mg/ml to 30 mgl, 5 mg/ml to 20 mg/ml, 5 mg/ml to 25 mg/ml, or 10 mg/ml to 20 mg/ml of sNAG nanofibers per dose/application in a form suitable for topical delivery to a patient.

In other embodiments, a sNAG nanofiber composition can comprise about 0.1 mg/cm² to 10 mg/cm² of sNAG nanofibers per dose/application in a form suitable for topical delivery to a patient. In particular, a sNAG nanofiber composition can comprise about .1 mg/cm², 0.25 mg/cm², 0.5 mg/cm², 0.75 mg/cm², 1 mg/cm², 2 mg/cm², 2.5 mg/cm², 3 mg/cm², 4 mg/cm², 5 mg/cm², 6 mg/cm², 7 mg/cm², 7.5 mg/cm², 8 mg/cm², 9 mg/cm², or 10 mg/cm² of sNAG nanofibers per dose/application in a form suitable for topical delivery to a patient. In some embodiments, a sNAG nanofiber composition can comprise about 0.5 mg/cm² to 10 mg/cm², 0.75 mg/cm² to 7.5 mg/cm², 1 mg/cm² to 7.5 mg/cm², or 1 mg/cm² to 5 mg/cm² of sNAG nanofibers per dose/application in a form suitable for topical delivery to a patient.

### 5.7 Combination Therapy

In various embodiments, the sNAG nanofibers described herein or compositions thereof may be administered to a subject in combination with one or more other therapies. The one or more other therapies may be beneficial in the treatment or prevention of a disease or may ameliorate a symptom or associated with a condition and disease. In certain embodiments, the therapies are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In specific embodiments, two or more therapies are administered within the same patent visit.

In certain embodiments, the one or more therapies is surgery. In a specific embodiment, surgery is performed on an organ or tissue in a patient, and a composition described herein is administered to the operated site (e.g., the wound site) before, during, and/or after the surgery.

In a particular embodiment, surgery is performed to remove all or part of a solid tumor or skin cancer, and a composition described herein is administered to the site of the tumor before, during, and/or after the surgery. In certain embodiments, the one or more therapies is radiation therapy.

In certain embodiments, the one or more therapies is an anti-viral or anti-bacterial agent. Any anti-viral agents well-known to one of skill in the art may used in combination with the sNAG nanofibers described herein or compositions thereof. The anti-viral and anti-bacterial agents that can be used in combination with the sNAG nanofibers are described in WO 2011/130646 and WO 2012/142581.

In other embodiments, the compositions described herein do not comprise any additional anti-viral and/or anti-bacterial agents. In one embodiment, the compositions described herein do not comprise an antibiotic. In some embodiments, the sNAG nanofibers are not administered to a subject in combination with one or more anti-viral and/or anti-bacterial agents. In one embodiment, the sNAG nanofibers are not administered to a subject in combination with one or more antibiotics. In one embodiment, the compositions described herein do not comprise and/or are not administered to a subject in combination with zinc.

In some embodiments, the therapy(ies) used in combination with the sNAG nanofibers described herein or compositions thereof is an anti-inflammatory agent. Non-limiting examples of anti-inflammatory agents include non-steroidal anti-inflammatory drugs (NSAIDs) (e.g., celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOXX™), naproxen (ALEVE™, NAPROSYN™), ketoprofen (ACTRON™) and nabumetone (RELAFEN™)), steroidal anti-inflammatory drugs (e.g., glucocorticoids, dexamethasone (DECADRON™), corticosteroids (*e.g.*, methylprednisolone (MEDROL™)), cortisone, hydrocortisone, prednisone (PREDNISONE™ and DELTASONE™), and prednisolone (PRELONE™ and PEDIAPRED™)), anticholinergics (*e.g.*, atropine sulfate, atropine methylnitrate, and ipratropium bromide (ATROVENT™)), beta2-agonists (*e.g*., abuterol (VENTOLIN™ and PROVENTIL™), bitolterol (TORNALATE™), levalbuterol (XOPONEX™), metaproterenol (ALUPENT™), pirbuterol (MAXAIR™), terbutlaine (BRETHAIRE™ and BRETHINE™), albuterol (PROVENTIL™, REPETABS™, and VOLMAX™), formoterol (FORADIL AEROLIZER™), and salmeterol (SEREVENT™ and SEREVENT DISKUS™)), and methylxanthines (*e.g*., theophylline (UNIPHYL™, THEO-DUR™, SLO-BID™, AND TEHO-42™)).

In some embodiments, the therapy(ies) or an additional agent used in combination with the sNAG nanofibers described herein or compositions thereof is a tissue filler (e.g., a dermal filler). The additional tissue filler can be hyaluronic acid, collagen, polylactic acid, calcium hydroxylapatite, polymethylmethacrylate (PMMA), or polyalkylimide. A tissue filler can also be human fat (e.g., autologous human fat). In some of these embodiments, an additional tissue filler can be formulated together with the sNAG nanofibers into one composition (i.e., a composition comprising the sNAG nanofibers and an additional tissue filler). In one embodiment, collagen is formulated together with the sNAG nanofibers. In one embodiment, hyaluronic acid is formulated together with the sNAG nanofibers. In one embodiment, human fat is formulated together with the sNAG nanofibers (in such embodiments the combined formulation may be prepared by a physician prior to its administration to a patient). In yet another embodiment, the sNAG nanofibers can be administered in combination with another tissue filler (e.g., before, simultaneously or after another tissue filler), without being formulated into one composition with another tissue filler.

In some embodiments, the therapy(ies) or an additional agent used in combination with the sNAG nanofibers described herein or compositions thereof is botulinum toxin (e.g., BOTOX™ or DYSPORT™). In some embodiments, the sNAG nanofibers can be administered in combination with botulinum toxin (e.g., before, simultaneously or after botulinum toxin), without being formulated into one composition with botulinum toxin.

In some embodiments, the therapy(ies) used in combination with the sNAG nanofibers described herein or compositions thereof is a cosmetic procedure. For example, a cosmetic procedure can be a chemical peel or a laser treatment. In one embodiment, the sNAG nanofiber composition may be applied after a chemical peel or laser treatment (e.g., laser skin resurfacing). In another embodiment, the sNAG nanofiber composition may be applied at the same time or during a chemical peel or laser treatment (e.g., laser skin resurfacing).

In some embodiments, the therapy(ies) used in combination with the sNAG nanofibers described herein or compositions thereof is a plastic surgery procedure. For example, the sNAG nanofibers described herein or compositions thereof may be used as a tissue filler in a plastic surgery procedure that requires a tissue filler treatment. In another example, the sNAG nanofibers described herein or compositions thereof may be applied topically after a plastic surgery procedure to prevent scarring, adhesions or fibrosis.

In some embodiments, the therapy(ies) used in combination with the sNAG nanofibers described herein or compositions thereof is an anti-pain medication (e.g., an analgesic). In some embodiments, the therapy(ies) used in combination with the sNAG nanofibers described herein or compositions thereof is an anti-fever medication.

### 5.8 Kits

Also disclosed herein is a pharmaceutical pack or kit comprising one or more of the sNAG nanofiber compositions described herein. The pack or kit may comprise one or more containers filled with one or more ingredients comprising the compositions described herein. The composition is preferably contained within a sealed, water proof, sterile package which facilitates removal of the composition without contamination. Materials from which containers may be made include aluminum foil, plastic, or another conventional material that is easily sterilized. The kit can contain material for a single administration or for multiple administrations of the composition, preferably wherein the material for each administration is provided in a separate, waterproof, sterile package.

In another instance, a container having dual compartments is provided. A first compartment contains any of the above-described sNAG nanofiber compositions described herein, while the second compartment contains another active agent such as another agent to be used in combination with the sNAG nanofiber composition (see, for example, Section 5.7, above, for description of additional agents that can be used in combination with the sNAG nanofibers). In the field or the clinic, the composition in the first compartment can be readily combined with the agent in the second compartment for subsequent administration to a patient.

The kit can also contain an applicator for administration of one or more of the sNAG nanofiber compositions described herein, and/or for administration of another active agent such as another agent to be used in combination with the sNAG nanofiber composition. In one instance, the kit comprises an applicator for topical administration of a sNAG nanofiber composition. Examples of applicators for topical administration of a sNAG nanofiber composition include, without limitation, a syringe, a spatula, a tube (a squeeze tube), and a cotton swab. In another instance, the kit comprises an applicator for administration of the sNAG nanofiber composition by local injection (e.g., a syringe).

Additionally, a kit designed for emergency or military use can also contain disposable pre-sterilized instruments, such as scissors, scalpel, clamp, tourniquet, elastic or inelastic bandages, or the like.

Optionally associated with such kit or pack can be a notice in the form prescribed by a governmental agency regulating the manufacture, use or sale of pharmaceuticals or biological products, which notice reflects approval by the agency of manufacture, use or sale for human administration. For example, a kit can comprise a notice regarding FDA approval and/or instructions for use.

The kits encompassed herein can be used in the above applications and methods.

### 6. EXAMPLES

The examples below show that treatment of cutaneous wounds with poly-N-acetyl-glucosamine nanofibers (sNAG) results in decreased scar sizes as compared to untreated wounds, and increased tensile strengths and elasticity. Visualization of collagen content using Masson trichrome staining by the inventors suggested that sNAG treated wounds display a reduction in collagen content and an organized collagen phenotype where collagen fibrils are aligned similarly to unwounded tissue. Further, the examples below show that sNAG treatment reduced smooth muscle actin expression within the wound, suggesting a reduction in myofibroblast content. Taken together, the data shown in Examples 1-5 below suggest that treatment of tissue with sNAG nanofibers reduces scarring by a mechanism that results in decreased collagen content, appropriate collagen fibril alignment and increased collagen III expression, and increases tissue elasticity and elastin content.

Further, the inventors found that treatment *in vitro* and *in vivo* with sNAG nanofibers results in an Aktl dependent increased expression of Epithelial Stromal Interaction Protein 1 (EPSTI1), a novel protein involved in epithelial/stromal interactions. Co-staining of OPSTI1, Hsp47 (a collagen chaperone) and vimentin showed that this protein is up regulated in properly aligned collagen producing cells. The inventors developed a fibrin gel to assess the regulation of fibroblast alignment *in vitro.* The gel-like matrix is formed within a well containing two "pins" that provide focal points upon which the gel can exert force as the cells align form pole to pole. The inventors found that sNAG stimulation of embedded fibroblasts resulted in better alignment as compared to untreated controls, by a process that is Aktl dependent. Further, the inventors found that in Aktl null animals sNAG treatment does not increase tensile strength or elasticity. Taken together, these data suggest that sNAG nanofibers stimulate an Aktl dependent pathway that results in the proper alignment of fibroblasts, decreased scarring, and increased tensile strength during cutaneous wound healing.

sNAG nanofibers (also known as Taliderm) used in the examples provided below are diatom-derived short biodegradable p-GlcNAc fibers obtained from a longer form of the fibers (known as NAG), they have an average length of 4-7µm and a polymer molecular weight of approximately 60,000Da. sNAG nanofibers used in the examples provided below were produced by Marine Polymer Technologies and formed into suitable patches for treatment.

### 6.1 Reference Example 1: sNAG Nanofibers Increase Tensile Strength And Elasticity Of Tissue

This example shows that sNAG nanofibers increase tensile strength and elasticity of tissue. In particular, this example demonstrates that cutaneous wounds treated with sNAG nanofibers exhibited tensile strength similar to that of unwounded tissue. This example further demonstrates that treatment of cutaneous wounds with sNAG nanofibers increased elasticity of tissue as compared to both untreated cutaneous wounds and unwounded control skin.

### Materials and Methods:

Eight adult male wild type C57B1/6 mice between 8-12 weeks were used in the experiment. Four mice were left unwounded during the 21 days as the control group (a representative sample of normal unwounded skin from wild type mice) and four animals were the experimental group. In the experimental group (four mice) hair was removed by depilation and the area was washed and sterilized using 70% ethanol. Mice in the experimental group were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 21 days. On day 21, wounds (treated and untreated) were harvested and skin was trimmed (15mm x 7mm) to insure even tension. Flank tissue from the control animals that were not wounded were harvested in the same manner (unwounded control).

Wounds, both treated and untreated and unwounded control skin were subjected to tensile strength and elasticity testing using an Instron 5942 strain gauge extensometer and Bluehill 3 Testing Software. Tensile strength of the skin was determined by measuring the relative stress the skin could bear before breaking 20% and elasticity was measured in the mm extension.

### Results and Conclusions:

Figures 1A and 1B show the result for Tensile Strength (Relative Stress) and Elasticity. Analysis of mechanical testing shows that sNAG treated cutaneous wounds of WT animals display an approximate 40% increase in tensile strength compared to untreated wounds (Fig. 1A). Additionally, sNAG treated wounds exhibited tensile strength recovery at levels similar to unwounded control skin (Fig. 1A). During tensile strength measurements, it was noted that sNAG treated cutaneous wounds from WT animals were more elastic than control or untreated counterparts. Figure 1B illustrates that sNAG treatment results in significantly increased elasticity of the healed tissue as compared to both untreated cutaneous wounds and unwounded control skin.

### 6.2 Reference Example 2: sNAG Nanofibers Increase Elastin Production In Tissue

This example shows that sNAG nanofibers increase elastin production. In particular, this example demonstrates that cutaneous wounds treated with sNAG nanofibers exhibited elastin production whereas untreated wounds did not.

### Materials and Methods:

Four adult male wild type C57B1/6 mice between 8-12 weeks were used in the experiment. The hair was removed by depilation and the area was washed and sterilized using 70% ethanol. Mice were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 10 days. On day 10, Wounds were fixed in 4% paraformaldehyde overnight at 4°C, embedded in paraffin, and sectioned for analysis.

Tissue sections from wounded animals, as described above, were stained for elastin fibers using Van Geison staining procedures. Briefly, sections were cleared in xylene, rehydrated through a series of graded alcohols to distilled water, stained in hematoxylin (Sigma-Aldrich), differentiated in 2% ferric chloride and washed. Tissue sections were then stained in Van Geison's counterstain prior to dehydration, clearing in xylene, and mounting with Cytoseal-XYL (Richard-Allan Scientific). Tissue sections were sections were visualized using an Olympus BX40 microscope and captured using an Olympus Camera (Model DP25) and DP2-BSW acquisition software.

### Results and Conclusions:

Figure 2 shows that wounds derived from treated animals show elastin production (as visualized by the thin black structures) in the newly healed wound whereas untreated wounds do not.

### 6.3 Reference Example 3: sNAG Nanofibers Reduce Scarring Of Tissue

This example shows that sNAG nanofibers decrease scarring of tissue. In particular, this example demonstrates that cutaneous wounds treated with sNAG nanofibers exhibited approximately 2-fold reduction in scar size as compared to untreated wounds.

### Materials and Methods:

Five adult male wild type C57B1/6 mice between 8-12 weeks were used in the experiment. The hair was removed by depilation and the area was washed and sterilized using 70% ethanol. Mice were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 21 days. On day 21, animals were euthanized and scars were measured using a caliper.

### Results and Conclusions:

As shown in Figure 3, sNAG treated wounds show an approximately 2-fold reduction in scar size as compared to untreated wounds.

### 6.4 Reference Example 4: sNAG Nanofibers Reduce Collagen Content And Help To Achieve Organized Collagen Alignment In Tissue

This example shows that sNAG nanofibers reduce collagen content and induce formation of organized collagen alignment in tissue. In particular, this example demonstrates that cutaneous wounds treated with sNAG nanofibers exhibited decreased collagen content and more organized collagen alignment as compared to untreated wounds.

### 6.4.1 Analysis of Collagen Content and Alignment Using Masson's Trichrome Stain

### Materials and Methods:

Four adult male wild type C57B1/6 mice between 8-12 weeks were used in the experiment. The hair was removed by depilation and the area was washed and sterilized using 70% ethanol. Mice were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 10 days. On day 10, wounds were fixed in 4% paraformaldehyde overnight at 4°C, embedded in paraffin, and sectioned for analysis.

Masson's Trichrome stain (Sigma-Aldrich) was performed according to manufacturer's instructions for tissue sections. Briefly, sections were deparrafanized to water and incubated in Bouin's solution. Slides were subjected to a series of incubations using hematoxylin, Biebrich Scarlet-Acid Fucshin, Phosphotungstic/Phosphomolybdic acid solution, Aniline Blue solution, and Acetic Acid as described by the manufacturer tissue sections were then dehydrated, cleared in xylene, and mounted using Cytoseal-XYL (Richard-Allan Scientific). Masson's trichrome sections were visualized using an Olympus BX40 microscope and images were captured using an Olympus Camera (Model DP25) and DP2-BSW acquisition software.

### Results and Conclusions:

To examine the amount and quality of collagen in treated and untreated wounds, Masson's trichrome staining was performed on tissue sections from 10 days post wounding. As seen in Figure 4A, sNAG treatment of cutaneous wounds results in decreased collagen content as indicated by less blue staining and more organized collagen alignment, especially as visualized at the wound borders, where new collagen in appropriately aligned with existing collagen.

### 6.4.2 Analysis of Collagen Content Using Hydroxyproline Assay

Hydroxyproline assays were performed to quantitatively analyze the amount of collagen deposition in treated and untreated wounds.

### Materials and Methods:

Four adult male wild type C57B1/6 mice between 8-12 weeks were used in the experiment. The hair was removed by depilation and the area was washed and sterilized using 70% ethanol. Mice were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 10 days. On day 10, the following steps were performed:
1. Tissue was lyophilize. Tissue was weighed to ascertain dry weight of tissue.
2. Lyophilized tissue was pulverized (the finer the ground the better, such as minced in the tube with a small weighing spatula) and placed in a hydrolysis tube (such as pyrex tubes - Fisher cat. # 14-932A). 5 ml of 6N HCl was added in a fume hood. Tubes were held under nitrogen gas to expel air (blowing N₂ for ∼20 seconds right down next to surface). Tubes were capped tightly. Tubes were gently agitated without vortexing. Tubes were placed in oven and hydrolyzed for 3 hours (or overnight) at 120 degrees.
3. After incubation, sample was transferred to a 50 ml conical tube containing 10 ml H₂O and 2 ml working buffer.
4. pH was adjusted to 7-8 using 4N NaOH, and 6N HCl was used to correct. The volume after adjusting pH was noted as it might differ significantly between samples. The differences were corrected to get accurate quantification when analyzing results.
5. ∼50 mg activated charcoal was added to each sample, vortexed to suspend the charcoal and centrifuged at ∼3,000 rpm for 10 minutes.
6. During this period, standards were made. Standards for tissue should be 0 (blank), 1, 2, 3, 4, 5, 7, and 10 ug/ml hydroxyproline. The assay is sensitive to 0.125 ug/ml and can also quantify collagen levels in tissue culture (e.g.). The standards were made fresh for each set of samples to be run from a frozen 1000 ug/ml stock aliquot. Standards were made in 15 ml tube.
7. 2 mls of sample supernatant or 2 mls of standard was added to fresh 15 ml tube (one tube needed for each sample and one for each standard). Blank: 2mls of working solution.
8. 1ml of Chloramine T was added to tube, vortexed and allowed to stand at room temp for 20 minutes.
9. 1 ml of Ehrlich's reagent was added to the reaction tube, vortexed, and incubates in water bath at 60 degrees C for 15 minutes.
10. Reaction tubes were cooled in tap water, and read in spectrophotometer at 558 nm.
11. Solution was discarded into proper hazard container.
12. Readings from the standard curve are in ug/ml. Multiply by final volume after pH for total hydroxyproline. Divide by dry weight for ug hypro/mg tissue.

### Hydroxyproline stock used:

10 mg of L-hydroxyproline (Sigma) was weighed, placed in dH₂O to create a 1 mg/ml concentration, and frozen in 1 ml aliquots.

### Dilutions for standards:

1 ml of 1000ug/ml (stock): 9 ml dH₂O = 100ug/ml
1.2 ml of 100ug/ml: 10.8 ml dH₂O = 10 ug/ml (need 11 ml)
1 ml of 10ug/ml: 9 ml dH₂O = 1 ug/ml

| | |
|---|---|
| 7 ug/ml | 3.5 ml of 10ug/ml : 1.5 ml dH₂O |
| 5 ug/ml | 2 ml of 10 ug/ml : 2 ml dH₂O |
| 4 ug/ml | 2 ml of 10 ug/ml : 3 ml dH₂O |
| 3 ug/ml | 1.5 ml of 10 ug/ml : 3.5 ml dH₂O |
| 2 ug/ml | 1 ml of 10 ug/ml : 4 ml dH₂O |
| 1 ug/ml | 1 ml of 10 ug/ml : 9 ml dH₂O |
| 0.75 ug/ml | 3 ml of 1ug/ml: 1 ml dH₂O |
| 0.50 ug/ml | 2 ml of 1 ug/ml: 2 ml dH₂O |
| 0.25 ug/ml | 1 ml of 1 ug/ml: 3 ml dH₂O |
| 0.125 ug/ml | 0.5 ml of 1 ug/ml : 3.5 ml dH₂O |

### Blank

### Acetic Acid (0.5 M):

14.4 ml Acetic Acid
485.6 dH₂O

### Stock Buffer Preparation (500 ml):

25g Anhydrous Citric Acid
6 ml 0.5M Acetic acid
60g Sodium Acetate
17g Sodium Hydroxide
Reagents were dissolved in dH₂O for a total volume of 500 ml.

### Working Buffer:

500 ml Stock Buffer
100 ml dH₂O
150 ml 1-Propanol
Adjust pH to 7-8 with Acetic Acid if necessary.
Stable for several months at 4° C.

### Chloramine T:

1.41g Chloramine T
10 ml dH₂O
10 ml 1-Propanol
80 ml Working buffer
pH read between 6-7. Stored in a dark bottle at 4° C.

### Ehrlich's Reagent:

7.5g p-dimethyl-amino-benzaldehyde
30 ml 1-propanol
13 ml perchloric acid (60%)
Makes 50 ml.
Stable for several hours only. Made prior to each set of determination.

All reagents were purchased from Sigma.

### Results and Conclusions

Hydroxyproline assays were performed to quantitatively analyze the amount of collagen deposition in treated and untreated wounds. As shown in Figure 4B, sNAG treated wounds have an approximately 3-fold decrease in overall collagen content.

### 6.5 Reference Example 5: sNAG Nanofibers Decrease Collagen I Expression And Increase Collagen III Expression

This example shows that sNAG nanofibers decrease collagen I expression and increase collagen III expression. In particular, this example demonstrates that the expression of collagen I is decreased and the expression of collagen III is increased in wounds treated with sNAG nanofibers as compared to untreated wounds.

### Materials and Methods

Four adult male wild type C57B1/6 mice between 8-12 weeks were used in the experiment. The hair was removed by depilation and the area was washed and sterilized using 70% ethanol. Mice were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 5 days. On day 5, RNA isolated from wounds (treated and untreated) were tested for expression of collagen type I and collagen type III by PCR.

For semi-quantitative RT-PCR cDNA is synthesized from total RNA (2-5µg), isolated using RNA-STAT 60 (Tel-Test, Inc.) in procedures described by the manufacturer, with a Superscript First Strand Synthesis Kit purchased from Gibco BRL using Oligo(dT) following the manufacturer's instructions. PCR reactions contained equal amounts of cDNA and 1.25µM of the appropriate primer pair (Proligo, Inc.). The primer sequences are as follows: Collagen I: forward 5' ACGGCTGCACGAGTCACAC 3', reverse 5' GGCAGGCGGGAGGTCTT 3', Collagen III: forward 5' GTTCTAGAGGATGGCTGTACTAAACACA 3', reverse 5' TTGCCTTGCGTGTTTGATATTC 3' and HPRT: forward 5' AAGGACCTCTCGAAGTGTTGGATA 3' reverse 5' CATTTAAAAGGAACTGTTGACAACG 3'. Cycling conditions were: 94°C for 5 min; 20-35 cycles of 94°C for 1min, 50-65°C (based on primer Tₘ) for 1min, 72°C for 1min 45sec + 2sec/cycle; 72°C for 7min and cooled to 4°C. Cycle number was empirically determined to be within the linear range of the assay for each primer pair used. All semi-quantitative RT-PCR was performed in tandem with HPRT primers as an internal control.

### Results and Conclusions

To test if sNAG induced a change in the type of collagen expressed, RNA isolated from wounds (treated and untreated) at day 5 post wounding were tested for expression of collagen type I and collagen type III by PCR.

As shown in Figure 5 , sNAG treated wounds have a decrease in collagen I expression and an increase in collagen III expression.

### 6.6 Reference Example 6: Reduction In Smooth Muscle Actin

This example demonstrates that sNAG nanofibers reduce myofibroblast content as assessed by measuring alpha smooth muscle actin. In particular, sNAG nanofiber-treated cutaneous wounds have at least a 2 fold reduction in alpha smooth muscle actin as compared to untreated cutaneous wounds.

### Materials and Methods

Four adult male wild type C57B1/6 mice between 8-12 weeks were used in the experiment. The hair was removed by depilation and the area was washed and sterilized using 70% ethanol. Mice were anesthetized using an O2/Isoflurane vaporizing anesthesia machine (VetEquip, Inc.). Isoflurane was used at 4% for induction; 2% for surgery. Two full thickness cutaneous wounds were created using a 4mm biopsy punch (Miltex), to create two identical wounds on each flank. One flank was treated with a thin sNAG membrane (Marine Polymer Technologies, Inc.) moistened with distilled water or the other flank was left untreated. The wound sites were covered with a polyurethane transparent dressing (Tegaderm, 3M) and left to heal for 10 days. On day 10, wounds were fixed in 4% paraformaldehyde overnight at 4°C, embedded in paraffin, and sectioned for analysis.

Paraffin embedded tissue sections were re-hydrated through xylene and a series of graded alcohols. Sections were treated with 0.01% Triton-XlOO and subjected to antigen retrieval using antigen unmasking solution (Vector Laboratories) in a pressure cooker for 5 min and allowed to cool. Samples were incubated in Background Buster (Innovex Biosciences) for 30 minutes prior to antibody labeling. Skin sections were labeled with mouse monoclonal anti-Actin α-Smooth Muscle antibody (Sigma). Sections were incubated in primary antibody overnight at 4°C, washed, and incubated with the appropriate secondary immunofluorescent antibodies (Invitrogen) for 1 hour at room temperature. Control sections for each antibody were stained without primary antibody. Tissue sections were visualized using an Olympus FluroView laser scanning confocal microscope (Model IX70) and captured at ambient temperature using an Olympus camera (Model FV5-ZM) and Fluoview 5.0 acquisition software.

### Results and Conclusions

Myofibroblasts are an important cell type in tissue repair and have been implicated in the generation of scarring via collagen production. Myofibroblast populations are reduced during fetal wound healing where scarring is absent. To visualize the distribution of myofibrobalsts populations, wound sections were labeled with an antibody directed against α-smooth muscle actin. As shown in Figure 6A and quantified in Figure 6B, sNAG-treated wounds show at least a 2-fold reduction in the expression of α- smooth muscle actin as compared to untreated wounds. In Figure 6B, the pixels contained in the red fluorescence are expressed as "arbitrary units" on the Y axis; such units are per field of tissue, providing a quantitative assessment of alpha smooth muscle actin expression and myofibroblast content.

### 6.7 Reference Example 6.7: sNAG nanofibers decrease scar size and increase tensile strength and elasticity of tissue

This example shows that sNAG nanofiber treatment of cutaneous wounds results in a smaller scar that has increased tensile strength and elasticity, decreased collagen content, increased collagen organization and decreased myofibroblast content. A fibrin gel assay was used to assess the regulation of fibroblast alignment *in vitro.* In this assay, fibrin lattice is formed with two pins that provide focal points upon which the gel can exert force as the cells align from pole to pole. Using a fibrin gel assay to assess fibroblast alignment within a lattice that provides tension points, this example shows that sNAG nanofiber stimulation results in increased fibroblast alignment as compared to untreated controls by a process also requiring Aktl. This example further shows that Aktl is required *in vivo* for the sNAG-induced increased tensile strength and elasticity. Taken together, these findings indicate that sNAG nanofibers stimulate an Aktl dependent pathway that results in the proper alignment of fibroblasts, decreased scarring, and increased tensile strength during cutaneous wound healing. Thus, pGlcNAc nanofiber treatment may result in a better tissue architecture of the repaired wound.

### Introduction

The inevitable result of adult wound healing is scar formation, which follows both surgical and trauma wounds. Scar formation following cutaneous wounding is due to an unorganized deposition of excess collagen and fibrous tissue that replaces normal dermal tissue. Scarring is a major medical complication that often results in restricted tissue movement, loss of function, poor aesthetics, and potential psychological distress [1]. The ability of mammals to heal without a scarring response (tissue regeneration), is restricted to a certain stage of development, after which wound healing proceeds with scarring. One major difference between tissue regeneration and scar formation is the organization and deposition of collagen. Comparisons of fetal regenerated tissue and adult scars show that each contains strikingly similar components, suggesting differences in regulation of tissue architecture and not in an abnormal molecular composition of the scar. Therefore tissue regeneration and scar-forming tissue repair share common mechanisms that may be controlled differently [2]. Given the impact of scar formation and the limited effectiveness of current treatments such as silicone dressings and hydrocortisone injections, the development and characterization of new treatments are needed [3].

### Materials and Methods

### Excisional Wound Healing Model, Scar Quantification, Tensile Strength Measurements:

Wild Type C57B1/6 and Aktl null mouse models [4] were used in all experiments. The Aktl null animals were created using an insertional mutagenesis strategy at the translational start site that blocks expression of the entire protein [14]. Aktl null animals on a mixed 129/C57Bl6 background were backcrossed onto C57B1/6 for eight generations. Wounding was performed on anesthetized adult male mice between 8-12 weeks old. Two full thickness cutaneous wounds were created using a 4mm biopsy punch (Miltex), to create two identical wounds on each flank. Mice were anesthetized using an O2/Isoflurane vaporizing anesthesia machine (VetEquip, Inc.). Isoflurane was used at 4% for induction; 2% for surgery. Prior to surgery hair was removed by depilation and the area was washed and sterilized using 70% ethanol. Wounds were either treated with a thin dried form of pGlcNAc (sNAG membrane) obtained from Marine Polymer Technologies, Inc. which was moistened with distilled water or left untreated. On days of interest, animals were euthanized and entire wounds were harvested including the surrounding skin using an 8 mm biopsy punch (Miltex). Wounds were fixed in 4% paraformaldehyde overnight at 4°C, embedded in paraffin, and sectioned for analysis.

For scar measurements, wild type mice were wounded as described above, or with a 1 cm² wound, which will not allow for contraction, and allowed to heal for 21 days. On day 21, animals were euthanized and scars were measured using a caliper.

For tensile strength, wounded animals were sacrificed after 21 days, wounds were harvested. Wounds, both treated and untreated and unwounded control skin was subjected to tensile strength and elasticity testing using an Instron 5942 strain gauge extensometer and Bluehill 3 Testing Software. Tensile strength of the skin was determined by measuring the relative stress the skin could bear before breaking 20% and elasticity was measured as the mm of extension.

### Hematoxylin and Eosin Staining (H&E), Masson Trichrome Staining, Picro Sirius Red Staining:

For H&E staining, sections were cleared in xylene, rehydrated through a series of graded alcohols, placed in hematoxylin followed by acid alcohol. Samples were then placed in ammonia water, rinsed in ethanol and exposed to Eosin before dehydrating through graded alcohols and clearing in xylene. Sections were mounted using Cytoseal-XYL (Richard-Allan Scientific). H&E sections were visualized using an Olympus BX40 microscope and captured using an Olympus Camera (Model DP25) and DP2-BSW acquisition software.

Masson's Trichrome stain (Sigma-Aldrich) was performed according to manufacturer's instructions for tissue sections. Briefly, sections were deparrafanized to water and incubated in Bouin's solution. Slides were subjected to a series of incubations using hematoxylin, Biebrich Scarlet-Acid Fucshin, Phosphotungstic/Phosphomolybdic acid solution, Aniline Blue solution, and Acetic Acid as described by the manufacturer tissue sections were then dehydrated, cleared in xylene, and mounted using Cytoseal-XYL (Richard-Allan Scientific). Masson's trichrome sections were visualized using an Olympus BX40 microscope and images were captured using an Olympus Camera (Model DP25) and DP2-BSW acquisition software.

### Extraction and Biochemical Quantitation of Collagen

Hydroxyproline assays were performed as previously described [15]. Briefly, equal amounts of wound tissue (8mm punch biopsy) was harvested from wild type mice treated or untreated at 21 days post wounding (n=4 for each). Tissue was lyophilized and weighed to ascertain dry weights and finely ground. Tissue collagen underwent complete acid hydrolysis with 6 N HCl for 18 h at 120°C, and neutralized to pH 7 with 4 N NaOH. One milliliter of chloramine T was added to 2-ml volumes of the collagen sample and incubated at room temperature for 20 min. One milliliter of Ehrlich's reagent (60% perchloric acid, 15 ml 1-propanol, and 3.75 g *p*-dimethyl-amino-benzaldehyde in 25 ml) was added, and samples were incubated at 60°C for 20 min. Absorbance at a wavelength of 558 was read on a spectrophotometer. Collagen was quantified as milligrams of hydroxyproline per gram dry weight of the wound tissue.

### Elastin Staining

Tissue sections from wounded animals, as described above, were stained for elastin fibers using Van Gieson staining procedures. Briefly, sections were cleared in xylene, rehydrated through a series of graded alcohols to distilled water, stained in hematoxylin (Sigma-Aldrich), differentiated in 2% ferric chloride and washed. Tissue sections were then stained in Van Gieson's counterstain prior to dehydration, clearing in xylene, and mounting with Cytoseal-XYL (Richard-Allan Scientific). Tissue sections were sections were visualized using an Olympus BX40 microscope and captured using an Olympus Camera (Model DP25) and DP2-BSW acquisition software.

### Immunofluoresence, microscopy

Paraffin embedded tissue sections were re-hydrated through xylene and a series of graded alcohols. Sections were treated with 0.01% Triton-XlOO and subjected to antigen retrieval using antigen unmasking solution (Vector Laboratories) in a pressure cooker for 5 min and allowed to cool. Samples were incubated in Background Buster (Innovex Biosciences) for 30 minutes prior to antibody labeling. Skin sections were labeled with mouse monoclonal anti-Actin α-Smooth Muscle antibody (Sigma), Vimentin mouse monoclonal antibody (Dako), Phalloidin (Molecular Probes) and DAPI (Molecular Probes). Sections were incubated in primary antibody overnight at 4°C, washed, and incubated with the appropriate secondary immunofluorescent antibodies (Invitrogen) for 1 hour at room temperature. Control sections for each antibody were stained without primary antibody. Tissue sections were visualized using an Olympus FluroView laser scanning confocal microscope (Model IX70) and captured at ambient temperature using an Olympus camera (Model FV5-ZM) and Fluoview 5.0 acquisition software.

### Tissue Culture, Fibrin Gel Assay

C3H10 fibroblasts were maintained at 37°C with 5% CO2 in minimal essential medium with Earle's balanced salt solution (MEM/EBSS, Thermo) supplemented with 1% penicillin/streptomycin (Invitrogen). Normal human fibroblasts (ATCC) were maintained in MEM supplemented with 20% FCS. Serum starvation was performed at 80-90% confluency in MEM/EBSS supplemented with 0.1% fetal calf serum (Valley Biomedical) for 24 hours followed by stimulation with highly purified pGlcNAc (50 µg/ml) nanofiber slurry (sNAG) in sterile water (Marine Polymer Technologies, Inc.).

Fibrin Gel Assays were performed in 24 well tissue culture plates (Becton Dickinson). Plates were prepared for procedure by addition of 300 ml Sylgard 184 elastomer (Dow Corning) to the bottom of the well, which was allowed to set overnight at 37°C with 5% CO2. Two 0.1 mm minutien pins (Fine Science Tools) were inserted into the Sylgard along the (16 mm) diameter of the well bottom, 3 mm away from each edge. Wells were blocked with 3% BSA in PBS for 30 minutes. Cells were counted and resuspended in a fibrinogen solution composed of 5% human fibrinogen (Sigma) dissolved in cold MEM/EBSS media with the addition of 10 µl/ml ascorbate and plated at a concentration of 250,000 cells per well. Thrombin (100 units/mL) from human plasma (Sigma) was added to 5 mg/mL fibrinogen and cell solution. The gels were incubated for 30 minutes at 37° with 5% CO2 prior to the addition of 1 ml of MEM/EBSS supplemented with 5% fetal calf serum. A small pipette tip was used to gently detach gels from the well walls. Gels were then monitored for contraction over time. At 6 to 8 hours after incubation, the media was removed, and the gels were washed 3 times with PBS, 5 minutes each and fixed with 4% paraformaldehyde for 30 minutes prior to either embedding in paraffin for tissue analysis or for phalloidin staining. For phalloidin staining: fibrin gels were treated with 0.3% Triton-XlOO and incubated in phalloidin for 1 hour at room temperature, and counter-stained with DAPI for 10 minutes. Sections were mounted using fluorogel. For tissue analysis, fixed fibrin gel sections were subjected to H&E staining as described above.

### Lentiviral Infection

Mission shRNA lentiviral constructs directed against Aktl were purchased from Sigma/Aldrich. Lentiviruses were propagated in 293T cells, maintained in DMEM supplemented as above. Lentiviral production was performed using psPAX2 and pMD2.G packaging vectors purchased from Addgene using the protocol for producing lentiviral particles from Addgene. For infection of target cells, 7.5 X 10⁵ cells were plated on 100 mm² plates and allowed to incubate overnight. The next day, cells were transduced using a final concentration of 1 mg/ml polybrene and Aktl shRNA lentiviruses. After transduction, fibroblasts were serum starved overnight and stimulated with sNAG (50 mg/ml). Any off target effects were controlled by verifying phenotypes using RNAi (Dharmacon) directed against a nonhomologous Aktl sequence (data not shown).

### Results

### sNAG treatment results in decreased scar size and increased collagen alignment

In this study, the effect of sNAG on scar formation was analyzed. Excisional wounds were created in wild type animals which were either treated with a membrane form of sNAG or left untreated. At 21 days post-wounding, animals were sacrificed and scar sizes were measured. As shown by the quantitation in Figure 7A, sNAG treated wounds show an approximately 2-fold reduction in scar size as compared to untreated wounds. To examine the amount and quality of collagen in treated and untreated wounds, Masson's trichrome staining was performed on tissue sections from 10 days post wounding. As seen in Figure 7B, sNAG treatment of cutaneous wounds results in decreased collagen content as indicated by less blue staining and more organized collagen alignment, especially as visualized at the wound borders, where new collagen in appropriately aligned with existing collagen. Hydroxyproline assays were performed to quantitatively analyze the amount of collagen deposition in treated and untreated wounds. As shown in Figure 7C, sNAG treated wounds have an approximately 3-fold decrease in overall collagen content.

Myofibroblasts are an important cell type in tissue repair and have been implicated in the generation of scarring via collagen production [16, 17]. Myofibroblast populations are reduced during fetal wound healing where scarring is absent. To visualize the distribution of myofibroblast populations, wound sections were labeled with an antibody directed against alpha smooth muscle actin (αSMA). As shown in Figure 8A and quantitated in Figure 8B, treated wounds show at least a 2-fold reduction in the expression of αSMA, suggesting that the reduction in collagen content are due to reduced numbers of myofibroblast cell number.

### sNAG treatment causes increases in tensile strength and elasticity of wounded skin

Given that collagen imparts strength and mechanical properties [18] and that collagen deposition and scar formation differs between treated and untreated cutaneous wounds, it was evaluated whether sNAG treatment results in increased tensile strength and elasticity. To measure tensile strength and elasticity, cutaneous wounds generated in wild type animals, both untreated and treated, were harvested after 21 days and subjected to mechanical testing using a strain gauge extensometer. Normal unwounded skin from wild type mice was used as a control. Analysis of mechanical testing shows that sNAG treated cutaneous wounds of WT animals display an approximate 40% increase in tensile strength compared to untreated wounds (Figure 9A). Additionally, sNAG treated wounds exhibited tensile strength recovery at levels similar to unwounded control skin (Figure 9A). During tensile strength measurements, it was noted that sNAG treated cutaneous wounds from WT animals were more elastic than control or untreated counterparts. Figure 9B illustrates that sNAG treatment results in significantly increased elasticity of the healed tissue as compared to both untreated cutaneous wounds and unwounded control skin. To assess if sNAG treatment of cutaneous wounds results in increased elastin production, Van Gieson staining was analyzed. In Figure 9C, wounds derived from treated animals show elastin production (as visualized by the thin black structures) in the newly healed wound whereas untreated wounds do not. These findings indicate that sNAG treatment results in increased tensile strength as well as increased elasticity and support the hypothesis that nanofiber treatment of cutaneous wounds decreases scarring while concurrently increasing mechanical properties of healing skin.

### sNAG treatment results in increased fibroblast alignment and contraction of fibrin gels

Since alignment and organization of collagen fibers is likely related to appropriate alignment of fibroblasts, a fibrin gel assay was utilized to assess if sNAG treatment results in alignment of fibroblasts *in vitro* [19]. To test this hypothesis, fibroblasts were treated with sNAG or left untreated prior to being embedded in fibrin gels. Fibrin gels were allowed to contract overnight and were then analyzed for contraction and sectioned for analysis of cell alignment. To visualize cell alignment, the gels were sectioned and stained with either phalloidin or H&E. Representative images of H&E stained gel sections show that sNAG treatment results in aligned fibroblasts (Figure 10A). Black circles indicate where pins were placed to allow tension points along which the gels could contract. sNAG-treated fibroblasts radiate from the pins in a linear, organized manner as compared to the scattered and unorganized arrangement of untreated cells. Phalloidin staining of gel sections were also performed to visualize the organized alignment of sNAG treated cells (Figure 4B). sNAG-dependent fibroblast alignment was confirmed in human dermal fibroblasts (data not shown). In addition, sNAG-treated fibroblasts correlated with increased gel contraction as compared to untreated controls (see below). Taken together, these data indicate that sNAG treatment of fibroblasts results in organized cell alignment. This alignment of fibroblasts likely results in the more aligned collagen fibers and increased tensile strength shown in sNAG treated cutaneous wounds, as seen in Figures 7-9.

### sNAG dependent fibroblasts alignment requires Aktl

To determine if Aktl is also required for the sNAG-dependent increase in fibroblast alignment, fibroblasts were transduced with a shRNA expressing lentivirus directed against Aktl prior to embedding in the fibrin gels. As shown in Figure 11A, sNAG-treated fibroblasts contracted the gels approximately 40% compared to gels containing untreated fibroblasts. Gels containing sNAG-treated shAkt1 expressing fibroblasts contracted significantly less than sNAG-treated controls. Analysis of H&E stained sections from the fibrin gels shows that alignment is inhibited when Aktl expression is blocked (Figure 11B). As shown in Figure 11C, sNAG-treated fibroblasts have an increased phosphorylation of Akt as compared to untreated controls, suggesting activation of the Akt pathway. Since the knockdown of Aktl resulted in decreased fibroblasts alignment *in vitro,* it was assessed whether tensile strength of cutaneous wounds derived from Aktl null animals would be affected by treatment with sNAG. Cutaneous wounds that had been treated with sNAG or left untreated were subjected to tensile strength testing using a strain gauge extensometer as described above. As shown in Figure 11D, there was no significant difference between sNAG treated and untreated wounds. Taken together, these findings indicate that Aktl is required for the sNAG-dependent alignment of fibroblasts as well as sNAG-dependent increase in tensile strength *in vivo.*

### Discussion

The above-presented findings show that highly purified pGlcNAc nanofiber (sNAG) treatment of cutaneous wounds results in reductions in scar formation. This decrease in scar formation is correlated with increased collagen organization, tensile strength and elasticity. Aktl ablation blocks the increased tensile strength induced by sNAG treatment. Tensile strength is determined by collagen remodeling during the maturation phase of wound healing, where collagen becomes increasingly organized due to a balance between synthesis and turnover [20]. In general, scar formation is characterized by increased collagen synthesis and reductions in collagen cross linking and alignment which causes an overall reduction in tensile strengths. Indeed, only 70% of the original tensile strength is regained after wound repair and tissue remodeling [21].

As shown by Masson trichrome staining and hydroxyproline quantitation of treated wounds, sNAG treatment results in a reduction in overall collagen content. Masson trichrome staining of treated wounds shows that sNAG treatment leads to more resolved collagen, increased kinetics, and collagen alignment at the wound borders. Picro Sirius Red staining indicates that sNAG treated wounds have a more seamless collagen fiber organization, suggesting that sNAG treatment increases the kinetics of wound resolution and remodeling. In support of the reductions in collagen content, a decrease in α-smooth muscle actin (αSMA) staining is demonstrated in this example, suggesting a decreased number of myofibroblasts in the wound. Myofibroblasts are atypical fibroblasts responsible for the fibrotic response during tissue repair and can be distinguished from un-activated fibroblasts by virtue of their expression of αSMA. Myofibroblasts are the major producers of collagen in cutaneous wound healing, are active during contraction, and are cleared from the wound by apoptosis [17]. Myofibroblast persistence can result in increased scarring or keloid formation [22, 23]. At 10 days post wounding, sNAG treatment resulted in an almost 3-fold reduction in the expression of aSMA. Since there are numerous ways by which myofibroblasts can differentiate, e.g., loss of smooth muscle contractile phenotype, epithelial trans-differentiation, recruitment of fibrocytes from the stroma or from the circulation, the findings presented in this example suggest that sNAG stimulation during wound repair could affect their differentiation or could increase their clearance.

To understand the role of sNAG in collagen alignment, a fibrin gel assay was used. A fibrin gel assay provides tension lines on which cells can align. An assumption was made that in order for collagen to be properly deposited, the fibroblasts themselves must be aligned. Cellular alignment is crucial for the formation of proper tissue architecture and provides appropriate mechanical function. Use of a three dimensional environment that more closely mimics an *in vivo* situation, provides an *in vitro* system in which to study these cellular responses. This system provides fibroblasts with a fibrin clot, a lattice similar to that which occurs during normal wound healing. Using this three dimensional system, the results presented above show that pretreatment of fibroblasts with sNAG under serum starved conditions resulted in a marked alignment of cells toward the tension poles as visualized by both H&E and phalloidin staining (Figure 10). As a more quantitative measurement of alignment, the results presented above show that aligned cells physically contract the fibrin clot (Figure 11) and that both the contraction and alignment is ablated under conditions of Aktl knockdown. Interestingly, a slight but reproducible increased Akt phosphorylation in aligned cells was observed. These findings show that Aktl is required for sNAG-induced cellular alignment *in vitro.* Aktl null animals validate these findings since the tensile strength of wounded skin was not affected by sNAG treatment in this model.

It is of note that Aktl null and wild type animals have similar tensile strengths of unwounded skin (data not shown). This suggests that the cellular programs controlling skin development are intact in the absence of Aktl. The double Akt1/Akt2 null animal, in addition to dwarfism and early neonatal lethality, presents with a thin skin phenotype, characterized by a marked reduction in the number of cells within each skin layer. This phenotype is due to a lack of proliferation that cannot be compensated by Akt3 alone [14]. The presence of either Akt2 or Akt3 does not affect sNAG stimulated wound repair and tensile strength as shown above, or the clearance of infection and wound closure as previously reported [4]. This demonstrates that these two kinases are not functionally redundant for sNAG induced tissue repair or antimicrobial effects.

Akt signals to the cytoskeleton via the Rho-family of GTPases and PAC1, among others, controlling cytoskeletal remodeling [24]. Akt can directly phosphorylate vimentin, an intermediate filament protein involved in cell shape and motility changes [25]. In motile cells, phospho-Akt is localized to the leading edge whereas in apical/basal polarized cells, phospho-Akt tends to be localized to the cell periphery. These findings suggest that Akt plays a role in cell polarity, cytoskeletal organization and directed cell movement. The general consensus in fibroblasts, as determined by both knockdown and overexpression analyses, is that Aktl positively directs migration while Akt2 blocks migration [26]. The inverse is true in epithelial cells. In the fibrin gel assay presented above, Aktl is required for the sNAG-induced alignment of cells between two tension points. Aktl must then be required for cytoskeletal rearrangement and cellular positioning, possibly affecting front to back cell polarity.

This example shows that sNAG stimulation results in an increased elasticity as compared to both unwounded and wounded skin. Elasticity is conferred by elastin fibers formed during development. Elastin synthesis is absent in the adult but can be induced following injury through the upregulation of certain cytokines and growth factors, e.g., insulin-like growth factor-1 (IGF1) and interleukin 1 (IL-1), but is aberrantly deposited and is not detected until months after healing. Aberrant deposition of elastin leads to decreased elasticity and flexibility of the scar [27, 28]. The results presented above show that sNAG treatment, by 21 day post wounding, leads to an increase in elastin fiber staining as compared to untreated wounds. This increased staining correlates with a marked increase in skin elasticity (Figure 9) and an increase in trophoelastin protein expression. At least *in vitro,* it has been shown that sNAG stimulation results in an increased expression of IL-1, suggesting a connection between this cytokine and elastin fiber staining [12]. It is well established that Aktl is a major kinase functioning downstream of both IL-1 and IGF-1.

In endothelial cells, the inventors found an increased expression of elastin in response to sNAG nanofiber treatment. Aktl has been linked to extracellular matrix assembly and fibronectin organization via the activation of β1 integrins [29]. It has been demonstrated that sNAG specifically activates integrin-dependent signaling and that this leads to Aktl activation [30]. Therefore, it is possible that elastin deposition in the sNAG stimulated wounds is due to an integrin/Aktl dependent pathway. Elastin fiber deposition does not generally occur in the adult. Thus, it is important to understand the mechanisms by which sNAG stimulates elastogenesis for skin repair and tissue bioengineering applications.

The findings presented in this example suggest that sNAG stimulates a cellular program that results in recovery of tissue architecture. While the exact mechanistic differences between fetal and adult wound healing remain unknown, it has been shown that significant differences exist among the inflammatory responses, extracellular matrix, cellular mediators, and gene expression profiles of fetal and postnatal wounds [31]. Due to a developing immune system, embryonic wounds have far fewer inflammatory cells and the inflammatory cells present are less differentiated [32] and have notable differences in the growth factor profiles of embryonic wounds.

Previously published results have shown that sNAG nanofiber stimulation results in increased metabolism [13], increased endothelial cell motility and angiogenesis [12], and importantly, enhanced innate immune responses leading to antibacterial activity [4], activities that are at least in part, dependent on Aktl activation. This example shows that sNAG stimulation results in an increased collagen and fibroblast alignment, increased tensile strength and elasticity during cutaneous wound healing. These findings suggest a model by which sNAG nanofibers stimulate outside-in integrin signaling that results in an activation of Aktl which controls cellular alignment under tension, and reductions in collagen I deposition and scar formation. Scarring is a major medical complication that causes decreased tissue function and aesthetics and the limitation of current treatment options, and this example shows that treatment of cutaneous wounds with sNAG nanofibers can be used to decrease scarring.

### References Cited in Example 7 and Other References:

1. Ferguson, M.W. and S. O'Kane, Scar-free healing: from embryonic mechanisms to adult therapeutic intervention. Philos Trans R Soc Lond B Biol Sci, 2004. 359(1445): p. 839-50.
2. Occleston, N.L., et al., Therapeutic improvement of scarring: mechanisms of scarless and scar-forming healing and approaches to the discovery of new treatments. Dermatol Res Pract, 2010.2010.
3. Bayat, A., D.A. McGrouther, and M.W. Ferguson, Skin scarring. BMJ, 2003. 326(7380): p. 88-92.
4. Lindner, H.B., et al., Anti-bacterial effects of poly-N-acetyl-glucosamine nanofibers in cutaneous wound healing: requirement for Akt1. PLoS One, 2011. 6(4): p. e18996.
5. Pietramaggiori, G., et al., Effects of poly-N-acetyl glucosamine (pGlcNAc) patch on wound healing in db/db mouse. J Trauma, 2008. 64(3): p. 803-8.
6. Kelechi, T.J., et al., A randomized, investigator-blinded, controlled pilot study to evaluate the safety and efficacy of a poly-N-acetyl glucosamine-derived membrane material in patients with venous leg ulcers. J Am Acad Dermatol, 2012. 66(6): p. e209-15.
7. Maus, E.A., Successful treatment of two refractory venous stasis ulcers treated with a novel poly-N-acetyl glucosamine-derived membrane. BMJ Case Rep, 2012. 2012.
8. Hankin, C.S., et al., Clinical and cost efficacy of advanced wound care matrices for venous ulcers. J Manag Care Pharm, 2012. 18(5): p. 375-84.
9. Hirsch, J.A., et al., Non-invasive hemostatic closure devices: "patches and pads". Tech Vasc Interv Radiol, 2003. 6(2): p. 92-5.
10. Palmer, B.L., et al., Effectiveness and safety of manual hemostasis facilitated by the SyvekPatch with one hour of bedrest after coronary angiography using six-French catheters. Am J Cardiol, 2004. 93(1): p. 96-7.
11. Scherer, S.S., et al., Poly-N-acetyl glucosamine nanofibers: a new bioactive material to enhance diabetic wound healing by cell migration and angiogenesis. Ann Surg, 2009. 250(2): p. 322-30.
12. Vournakis, J.N., et al., Poly-N-acetyl glucosamine nanofibers regulate endothelial cell movement and angiogenesis: dependency on integrin activation of Ets1. J Vasc Res, 2008. 45(3): p. 222-32.
13. Beeson, C.C., et al., Integrin-dependent Aktl activation regulates PGC-1 expression and fatty acid oxidation. J Vasc Res. 49(2): p. 89-100.
14. Mao, C., et al., Unequal contribution of Akt isoforms in the double-negative to double-positive thymocyte transition. J Immunol, 2007. 178(9): p. 5443-53.
15. Baicu, C.F., et al., Effects of the absence of procollagen C-endopeptidase enhancer-2 on myocardial collagen accumulation in chronic pressure overload. Am J Physiol Heart Circ Physiol, 2012. 303(2): p. H234-40.
16. van Amerongen, M.J., et al., Bone marrow-derived myofibroblasts contribute functionally to scar formation after myocardial infarction. J Pathol, 2008. 214(3): p. 377-86.
17. Sarrazy, V., et al., Mechanisms of pathological scarring: role of myofibroblasts and current developments. Wound Repair Regen, 2011. 19 Suppl 1: p. s10-5.
18. Buehler, M.J., Nature designs tough collagen: explaining the nanostructure of collagen fibrils. Proc Natl Acad Sci U S A, 2006. 103(33): p. 12285-90.
19. Kalson, N.S., et al., An experimental model for studying the biomechanics of embryonic tendon: Evidence that the development of mechanical properties depends on the actinomyosin machinery. Matrix Biol, 2010. 29(8): p. 678-89.
20. Oxlund, H., et al., Collagen deposition and mechanical strength of colon anastomoses and skin incisional wounds of rats. J Surg Res, 1996. 66(1): p. 25-30.
21. Clark, R.A., Wound repair. Curr Opin Cell Biol, 1989. 1(5): p. 1000-8.
22. Chipev, C.C., et al., Myofibroblast phenotype and apoptosis in keloid and palmar fibroblasts in vitro. Cell Death Differ, 2000. 7(2): p. 166-76.
23. Lee, Y.S. and S. Vijayasingam, Mast cells and myofibroblasts in keloid: a light microscopic, immunohistochemical and ultrastructural study. Ann Acad Med Singapore, 1995. 24(6): p. 902-5.
24. Zhou, G.L., et al., Akt phosphorylation of serine 21 on Pak1 modulates Nck binding and cell migration. Mol Cell Biol, 2003. 23(22): p. 8058-69.
25. Zhu, Q.S., et al., Vimentin is a novel AKT1 target mediating motility and invasion. Oncogene, 2011. 30(4): p. 457-70.
26. Fayard, E., et al., Protein kinase B (PKB/Akt), a key mediator of the PI3K signaling pathway. Curr Top Microbiol Immunol, 2010. 346: p. 31-56.
27. Pierce, R.A., C.H. Moore, and M.C. Arikan, Positive transcriptional regulatory element located within exon 1 of elastin gene. Am J Physiol Lung Cell Mol Physiol, 2006. 291(3): p. L391-9.
28. Vrhovski, B. and A.S. Weiss, Biochemistry of tropoelastin. Eur J Biochem, 1998. 258(1): p. 1-18.
29. Somanath, P.R., et al., Aktl signaling regulates integrin activation, matrix recognition, and fibronectin assembly. J Biol Chem, 2007. 282(31): p. 22964-76.
30. Beeson, C.C., et al., Integrin-dependent Aktl activation regulates PGC-1 expression and fatty acid oxidation. J Vasc Res, 2012. 49(2): p. 89-100.
31. Larson, B.J., M.T. Longaker, and H.P. Lorenz, Scarless fetal wound healing: a basic science review. Plast Reconstr Surg, 2010. 126(4): p. 1172-80.
32. Cowin, A.J., et al., Endogenous inflammatory response to dermal wound healing in the fetal and adult mouse. Dev Dyn, 1998. 212(3): p. 385-93.

## Claims

1. A method for treating wrinkles or depressions in the skin's surface in a human subject, comprising administering a composition comprising shortened fibers of poly-N-acetylglucosamine ("sNAG nanofibers") to the human subject, wherein the composition is prepared for topical administration directly or in proximity to, or local injection directly to, the wrinkles or depressions, wherein more than 70% of the monosaccharides of the sNAG nanofibers are N-acetylglucosamine monosaccharides, and wherein the sNAG nanofibers are: (i) from about 1 µm to less than 10 µm in length, or (ii) less than 10 µm in average length, and have the microstructure of non-irradiated poly-N-acetylglucosamine fibers.

2. A composition comprising shortened fibers of poly-N-acetylglucosamine ("sNAG nanofibers") for use in a method for treating wrinkles or depressions in the skin's surface in a human subject, comprising administering the composition to the human subject, wherein the composition is prepared for topical administration directly or in proximity to, or local injection directly to, the wrinkles or depressions, wherein more than 70% of the monosaccharides of the sNAG nanofibers are N-acetylglucosamine monosaccharides, and wherein the sNAG nanofibers are: (i) from about 1 µm to less than 10 µm in length, or (ii) less than 10 µm in average length, and have the microstructure of non-irradiated poly-N-acetylglucosamine fibers.

3. The method of claim 1, or the composition for use of claim 2, wherein more than 80%, more than 90%, more than 95%, or 100% of the monosaccharides of the sNAG nanofibers are N-acetylglucosamine monosaccharides.

4. The method or composition for use of any of claims 1 to 3, wherein the subject has an increased total collagen content, increased expression of collagen type I, decreased expression of collagen type III, decreased expression of elastin, increased expression of smooth muscle actin, decreased tensile strength of the skin and/or decreased elasticity of the skin.

5. The method or composition for use of any of claims 1 to 4, wherein the sNAG nanofibers are nonreactive when tested in an intramuscular implantation test; and/or wherein the sNAG nanofibers increase the metabolic rate of serum-starved human umbilical cord vein endothelial cells in a MTT assay and/or do not rescue apoptosis of serum-starved human umbilical cord endothelial cells in a trypan blue exclusion test.

6. The method or composition for use of any of claims 1 to 5, wherein more than 50% of the sNAG nanofibers are between about 2 to 10 µm in length; wherein more than 50% of the sNAG nanofibers are between about 4 to 7 µm in length; or wherein the sNAG nanofibers have an average length of 4 to 7 µm.

7. The method or composition for use of any of claims 1 to 6, wherein the sNAG nanofibers are produced by gamma irradiation of poly-N-acetylglucosamine and/or a derivative thereof, and wherein the poly-N-acetylglucosamine and/or a derivative thereof is irradiated in the form of dry fibers or a dry fiber membrane at 500-2,000 kgy, optionally 750-1,250 kgy; or the poly-N-acetylglucosamine and/or a derivative thereof was irradiated in the form of a suspension, a slurry or a wet cake at 100-500 kgy, optionally 150-250 kgy.

8. The method or composition for use of any of claims 1 to 7, wherein the sNAG nanofibers are produced by irradiation of poly-N-acetylglucosamine or a derivative thereof with a dose of irradiation that reduces the average length of the sNAG nanofibers to less than 10 µm in length.

9. The method or composition for use of any of claims 1 to 8, wherein the sNAG nanofibers are administered in an amount effective to achieve at least one or more of the following effects: (a) the increase in the tensile strength of a tissue in a subject, (b) the increase in elasticity in a tissue of a subject, (c) the increase in elastin content or production in a tissue of a subject, (d) the decrease in total collagen content in a tissue of a subject, (e) the decrease of collagen I expression or content in a tissue of a subject, (f) the increase in collagen III expression or content in a tissue of a subject, (g) the inducement of more organized collagen alignment in a tissue of a subject, (h) the reduction in smooth muscle actin content or expression in a tissue of a subject, and (i) the reduction in myofibroblast content in a tissue of a subject.

10. The method or composition for use of any claims 1 to 9, wherein the infrared spectrum of the sNAG nanofibers is equivalent or substantially equivalent to that of non-irradiated microalgal poly-β-1→4-N-acetylglucosamine fibers.

11. The method or composition for use of any of claims 1 to 10, wherein the composition is formulated as a cream, a membrane, a film, a liquid solution, a suspension, a powder, a paste, an ointment, an aerosol, a gel, or a spray.

12. The method or composition for use of any of claims 1 to 11, wherein the composition is formulated for administration to the subject once or twice a day for a duration equal to or greater than 1 week.

13. The method or composition for use of any of claims 1 to 12, wherein the composition comprises (i) 0.2 to 20 mg/cm² of sNAG nanofibers per dose/application of the composition in a form suitable for topical delivery to the subject, or (ii) 5 to 50 mg/ml of sNAG nanofibers per dose/application of the composition in a form suitable for topical delivery to the subject.

14. The method or composition for use of any of claims 1 to 10, wherein the composition is formulated for local injection and the wrinkles are from a few micrometers to several millimeters into the skin.

15. The method or composition for use of any of claims 1 to 14, wherein the sNAG nanofibers are not produced from crustacean poly-N-acetylglucosamine; and/or wherein the sNAG nanofibers are produced from a microalgal poly-N-acetylglucosamine.

16. The method of claim 1, or the composition for use of claim 2, wherein the composition is prepared for topical administration directly to the wrinkles or depressions in the skin's surface of the human subject, wherein greater than 50% of the sNAG nanofibers have an average length of 4 to 7 µm, and wherein the infrared spectrum of the sNAG nanofibers is equivalent or substantially equivalent to that of non-irradiated poly-β-1→4-N-acetylglucosamine fibers.

## Patentansprüche

1. Verfahren zur Behandlung von Falten oder Vertiefungen in der Hautoberfläche bei einem menschlichen Subjekt, umfassend Verabreichen einer Zusammensetzung, die verkürzte Fasern von Poly-N-acetylglucosamin ("sNAG-Nanofasern") umfasst, an ein menschliches Subjekt, wobei die Zusammensetzung zubereitet ist zur topischen Verabreichung direkt auf oder in der Nähe von oder als lokale Injektion direkt in die Falten oder Vertiefungen, wobei mehr als 70 % der Monosaccharide der sNAG-Nanofasern N-Acetylglucosamin-Monosaccharide sind und wobei die sNAG-Nanfasern (i) von ungefähr 1 µm bis weniger als 10 µm lang, oder (ii) weniger als durchschnittlich 10 µm lang sind und die Mikrostruktur von unbestrahlten Poly-N-acetylglucosamin-Fasern aufweisen.

2. Zusammensetzung umfassend verkürzte Fasern von Poly-N-acetylglucosamin ("sNAG-Nanofasern) zur Verwendung in einem Verfahren zur Behandlung von Falten oder Vertiefungen in der Hautoberfläche bei einem menschlichen Subjekt, umfassend Verabreichen der Zusammensetzung an das menschliche Subjekt, wobei die Zusammensetzung zubereitet ist zur topischen Verabreichung direkt auf oder in der Nähe von oder als lokale Injektion direkt in die Falten oder Vertiefungen, wobei mehr als 70 % der Monosaccharide der sNAG-Nanofasern N-Acetylglucosamin-Monosaccharide sind und wobei die sNAG-Nanfasern (i) von ungefähr 1 µm bis weniger als 10 µm lang, oder (ii) weniger als durchschnittlich 10 µm lang sind, und die Mikrostruktur von unbestrahlten Poly-N-acetylglucosamin-Fasern aufweisen.

3. Verfahren nach Anspruch 1 oder Zusammensetzung zur Verwendung nach Anspruch 2, wobei mehr als 80 %, mehr als 90 %, mehr als 95 %, oder 100 % der Monosaccharide der sNAG-Nanofasern N-Acetylglucosamin-Monosaccharide sind.

4. Verfahren oder Zusammensetzung zur Verwendung nach einem der Ansprüche 1 bis 3, wobei das Subjekt einen erhöhten Gesamtkollagengehalt, einer erhöhte Expression von Kollagen Typ 1, eine verringerte Expression von Kollagen Typ III, eine verringerte Expression von Elastin, einer erhöhte Expression von glattem Muskelaktin, eine verringerte Zugfestigkeit der Haut und/oder eine verringerte Elastizität der Haut aufweist.

5. Verfahren oder Zusammensetzung zur Verwendung nach einem der Ansprüche 1 bis 4, wobei die sNAG-Nanofasern unreaktiv sind, wenn sie in einem intramuskulären Implantationstest getestet werden; und/oder wobei die sNAG-Nanofasern die Stoffwechselrate von serumgehungerten humanen Endothelzellen der Nabelschnurvene in einem MTT-Assay erhöhen und/oder die Apoptose von serumgehungerten humanen Endothelzellen der Nabelschnurvene in einem Trypan-Blau-Ausschlusstest nicht retten.

6. Verfahren oder Zusammensetzung zur Verwendung nach einem der Ansprüche 1 bis 5, wobei mehr als 50 % der sNAG-Nanofasern zwischen ungefähr 2 bis 10 µm lang sind; wobei mehr als 50 % der sNAG-Nanofasern zwischen ungefähr 4 bis 7 µm lang sind; oder wobei die sNAG-Nanofasern eine durchschnittliche Länge von 4 bis 7 µm aufweisen.

7. Verfahren oder Zusammensetzung zur Verwendung nach einem der Ansprüche 1 bis 6, wobei die sNAG-Nanofasern durch Gamma-Bestrahlung von Poly-N-acetylglucosamin und/oder einem Derivat davon hergestellt werden, und wobei das Poly-N-acetylglucosamin und/oder ein Derivat davon in Form von trockenen Fasern oder einer trockenen Fasermembran bei 500 - 2.000 kgy, gegebenenfalls 750 - 1.250 kgy, bestrahlt werden; oder das Poly-N-acetylglucosamin und/oder ein Derivat davon in Form einer Suspension, einer Aufschlämmung oder eines Nasskuchens bei 100 - 500 kgy, gegebenenfalls 150 - 250 kgy, bestrahlt wurden.

8. Verfahren oder Zusammensetzung zur Verwendung nach einem der Ansprüche 1 bis 7, wobei die sNAG-Nanofasern durch Bestrahlung von Poly-N-acetylglucosamin und/oder einem Derivat davon mit einer Bestrahlungsdosis hergestellt werden, die die durchschnittliche Länge der sNAG-Nanofasern auf weniger als 10 µm Länge verringern.

9. Verfahren oder Zusammensetzung zur Verwendung nach einem der Ansprüche 1 bis 8, wobei die sNAG-Nanofasern in einer Menge verabreicht werden, die wirksam ist, um wenigsten eine oder mehrere der folgenden Wirkungen zu erzielen: (a) die Erhöhung der Zugfestigkeit eines Gewebes in einem Subjekt, (b) die Erhöhung der Elastizität in einem Gewebe eines Subjekts, (c) die Erhöhung des Elastingehalts oder der Elastinproduktion in einem Gewebe eines Subjekts, (d) die Verringerung des Gesamtkollagengehalts in einem Gewebe eines Subjekts, (e) die Verringerung der Kollagen-I-Expression oder des Kollagen-I-Gehalts in einem Gewebe eines Subjekts, (f) die Erhöhung der Kollagen-III-Expression oder des Kollagen-III-Gehalts in einem Gewebe eines Subjekts, (g) die Induktion eines organisierteren Kollagenausrichtung in einem Gewebe eines Subjekts, (h) die Verringerung des glatten Muskelatkin-Gehalts oder der glatten Muskelaktin-Expression in einem Gewebe eines Subjekts, und (i) die Verringerung des Myofibroblasten-Gehalts in einem Gewebe eines Subjekt.

10. Verfahren oder Zusammensetzung zur Verwendung nach einem der Ansprüche 1 bis 9, wobei das Infrarotspektrum der sNAG-Nanofasern äquivalent oder im wesentlichen äquivalent zu dem von unbestrahlten Mikroalgen-Poly-β-1→4-N-acetylglucosamin-Fasern ist.

11. Verfahren oder Zusammensetzung zur Verwendung nach einem der Ansprüche 1 bis 10, wobei die Zusammensetzung als eine Creme, eine Membran, ein Film, eine flüssige Lösung, eine Suspension, ein Pulver, eine Paste, eine Salbe, ein Aerosol, ein Gel oder ein Spray formuliert ist.

12. Verfahren oder Zusammensetzung zur Verwendung nach einem der Ansprüche 1 bis 11, wobei die Zusammensetzung zur Verabreichung einmal oder zweimal am Tag für die Dauer von gleich oder mehr als 1 Woche an das Subjekt formuliert ist.

13. Verfahren oder Zusammensetzung zur Verwendung nach einem der Ansprüche 1 bis 12, wobei die Zusammensetzung (i) 0,2 bis 20 mg/cm² sNAG-Fasern pro Dosis/Anwendung der Zusammensetzung in einer zur topischen Verabreichung an das Subjekt geeigneten, oder (ii) 5 bis 50 mg/ml der sNAG-Fasern pro Dosis/Anwendung der Zusammensetzung in einer zur topischen Verabreichung an das Subjekt geeigneten Form umfasst.

14. Verfahren oder Zusammensetzung zur Verwendung nach einem der Ansprüche 1 bis 10, wobei die Zusammensetzung für eine lokale Injektion formuliert ist und die Falten von wenigen Mikrometern bis einige Millimeter in die Haut reichen.

15. Verfahren oder Zusammensetzung zur Verwendung nach einem der Ansprüche 1 bis 14, wobei die sNAG-Nanofasern nicht aus Schalentier-Poly-N-acetylglucosamin hergestellt werden; und/oder wobei die sNAG-Nanofasern aus Mikroalgen-Poly-N-acetylglucosamin hergestellt werden.

16. Verfahren nach Anspruch 1 oder Zusammensetzung zur Verwendung nach Anspruch 2, wobei die Zusammensetzung zubereitet ist zur direkten topischen Verabreichung auf die Falten oder Vertiefungen in der Hautoberfläche des humanen Subjekts, wobei mehr als 50 % der sNAG-Nanofasern eine durchschnittliche Länge von 4 bis 7 µm aufweisen und wobei das Infrarotspektrum der sNAG-Nanofasern äquivalent oder im wesentlichen äquivalent zur dem von unbestrahlten Poly-β-1→4-N-acetylglucosamin-Fasern ist.

## Revendications

1. Procédé pour le traitement des rides ou des dépressions dans la surface de la peau chez un sujet humain, comprenant l'administration d'une composition comprenant des fibres raccourcies de poly-N-acétylglucosamine (« nanofibres de sNAG ») au sujet humain, dans lequel la composition est préparée pour une administration topique directement sur ou à proximité des ou par injection locale directement dans les rides ou dépressions, dans lequel plus de 70 % des monosaccharides des nanofibres de sNAG sont des monosaccharides de N-acétylglucosamine, et dans lequel les nanofibres de sNAG sont : (i) d'une longueur d'environ 1 µm à moins de 10 µm, ou (ii) d'une longueur inférieure à 10 µm en moyenne, et ont la microstructure de fibres de poly-N-acétylglucosamine non irradiées.

2. Composition comprenant des fibres raccourcies de poly-N-acétylglucosamine (« nanofibres de sNAG ») pour une utilisation dans un procédé de traitement des rides ou des dépressions dans la surface de la peau chez un sujet humain, comprenant l'administration de la composition au sujet humain, dans laquelle la composition est préparée pour une administration topique directement sur ou à proximité des ou par injection locale directement dans les rides ou dépressions, dans lequel plus de 70 % des monosaccharides des nanofibres de sNAG sont des monosaccharides de N-acétyl-glucosamine, et dans laquelle les nanofibres de sNAG sont : (i) d'une longueur d'environ 1 µm à moins de 10 µm, ou (ii) d'une longueur inférieure à 10 µm en moyenne, et ont la microstructure de fibres de poly-N-acétylglucosamine non irradiées.

3. Procédé selon la revendication 1, ou composition pour une utilisation selon la revendication 2, dans lequel plus de 80 %, plus de 90 %, plus de 95 % ou 100 % des monosaccharides des nanofibres de sNAG sont des monosaccharides de N-acétyl-glucosamine.

4. Procédé ou composition pour une utilisation selon l'une quelconque des revendications 1 à 3, dans lequel le sujet présente une teneur en collagène total augmentée, une expression du collagène de type I augmentée, une expression du collagène de type III diminuée, une expression de l'élastine diminuée, une expression de l'actine des muscles lisses augmentée, une résistance à la traction de la peau diminuée et/ou une élasticité de la peau diminuée.

5. Procédé ou composition pour une utilisation selon l'une quelconque des revendications 1 à 4, dans lequel les nanofibres de sNAG sont non réactives lorsqu'elles sont testées dans un test d'implantation intramusculaire ; et/ou dans lequel les nanofibres de sNAG augmentent le taux métabolique des cellules endothéliales de la veine du cordon ombilical humain privées de sérum dans un test MTT et/ou n'empêchent pas l'apoptose des cellules endothéliales du cordon ombilical humain privées de sérum dans un test d'exclusion au bleu de trypan.

6. Procédé ou composition pour une utilisation selon l'une quelconque des revendications 1 à 5, dans lequel plus de 50 % des nanofibres de sNAG ont une longueur située entre environ 2 et 10 µm ; dans lequel plus de 50 % des nanofibres de sNAG ont une longueur située entre environ 4 et 7 µm ; ou dans lequel les nanofibres de sNAG ont une longueur moyenne de 4 à 7 µm.

7. Procédé ou composition pour une utilisation selon l'une quelconque des revendications 1 à 6, dans lequel les nanofibres de sNAG sont produites par irradiation gamma de poly-N-acétylglucosamine et/ou de l'un de ses dérivés, et dans lequel la poly-N-acétylglucosamine et/ou l'un de ses dérivés est irradiée sous la forme de fibres sèches ou d'une membrane de fibres sèches à 500 à 2000 kgy, éventuellement 750 à 1250 kgy ; ou la poly-N-acétylglucosamine et/ou l'un de ses dérivés a été irradiée sous la forme d'une suspension, d'une suspension épaisse ou d'un gâteau humide à 100 à 500 kgy, éventuellement 150 à 250 kgy.

8. Procédé ou composition pour une utilisation selon l'une quelconque des revendications 1 à 7, dans lequel les nanofibres de sNAG sont produites par irradiation de poly-N-acétylglucosamine ou de l'un de ses dérivés avec une dose d'irradiation qui réduit la longueur moyenne des nanofibres de sNAG à une longueur inférieure à 10 µm.

9. Procédé ou composition pour une utilisation selon l'une quelconque des revendications 1 à 8, dans lequel les nanofibres de sNAG sont administrées dans une quantité efficace pour obtenir au moins un ou plusieurs des effets suivants : (a) l'augmentation de la résistance à la traction d'un tissu chez un sujet, (b) l'augmentation de l'élasticité dans un tissu d'un sujet, (c) l'augmentation de la teneur ou de la production d'élastine dans un tissu d'un sujet, (d) la diminution de la teneur en collagène total dans un tissu d'un sujet, (e) la diminution de l'expression ou de la teneur en collagène I dans un tissu d'un sujet, (f) l'augmentation de l'expression ou de la teneur en collagène III dans un tissu d'un sujet, (g) l'incitation à un alignement du collagène plus organisé dans un tissu d'un sujet, (h) la réduction de la teneur ou de l'expression d'actine des muscles lisses dans un tissu d'un sujet, et (i) la réduction de la teneur en myofibroblastes dans un tissu d'un sujet.

10. Procédé ou composition pour une utilisation selon l'une quelconque des revendications 1 à 9, dans lequel le spectre infrarouge des nanofibres de sNAG est équivalent ou sensiblement équivalent à celui des fibres de poly-β-1→4-N-acétylglucosamine des microalgues non irradiées.

11. Procédé ou composition pour une utilisation selon l'une quelconque des revendications 1 à 10, dans lequel la composition est formulée sous la forme d'une crème, d'une membrane, d'un film, d'une solution liquide, d'une suspension, d'une poudre, d'une pâte, d'une pommade, d'un aérosol, d'un gel, ou d'une pulvérisation.

12. Procédé ou composition pour une utilisation selon l'une quelconque des revendications 1 à 11, dans lequel la composition est formulée pour une administration au sujet une ou deux fois par jour pendant une durée égale ou supérieure à 1 semaine.

13. Procédé ou composition pour une utilisation selon l'une quelconque des revendications 1 à 12, dans lequel la composition comprend (i) 0,2 à 20 mg/cm² de nanofibres de sNAG par dose/application de la composition sous une forme appropriée pour une administration topique au sujet, ou (ii) 5 à 50 mg/ml de nanofibres de sNAG par dose/application de la composition sous une forme appropriée pour une administration topique au sujet.

14. Procédé ou composition pour une utilisation selon l'une quelconque des revendications 1 à 10, dans lequel la composition est formulée pour une injection locale et les rides sont de quelques micromètres à plusieurs millimètres dans la peau.

15. Procédé ou composition pour une utilisation selon l'une quelconque des revendications 1 à 14, dans lequel les nanofibres de sNAG ne sont pas produites à partir de poly-N-acétylglucosamine de crustacés ; et/ou dans lequel les nanofibres de sNAG sont produites à partir d'une poly-N-acétylglucosamine de microalgues.

16. Procédé selon la revendication 1, ou composition pour une utilisation selon la revendication 2, dans lequel la composition est préparée pour une administration topique directement aux rides ou dépressions dans la surface de la peau du sujet humain, dans lequel plus de 50 % des nanofibres de sNAG ont une longueur moyenne de 4 à 7 µm, et dans lequel le spectre infrarouge des nanofibres de sNAG est équivalent ou sensiblement équivalent à celui des fibres de poly-β-1→4-N-acétylglucosamine non irradiées.
